(19) Europäisches Patentamt
European Patent Office
Office européen des brevets

(11) EP 3 689 866 A1

(12) **EUROPEAN PATENT APPLICATION**
published in accordance with Art. 153(4) EPC

(43) Date of publication:
**05.08.2020 Bulletin 2020/32**

(21) Application number: **18861867.2**

(22) Date of filing: **21.09.2018**

(51) Int Cl.:
*C07D 403/14* (2006.01) *A01N 43/42* (2006.01)
*A01N 43/60* (2006.01) *A01N 43/653* (2006.01)
*A01N 43/713* (2006.01) *A01N 43/76* (2006.01)
*A01N 43/78* (2006.01) *A01N 43/80* (2006.01)
*A01N 43/824* (2006.01) *A01N 43/836* (2006.01)
*A01N 43/90* (2006.01) *A01N 47/02* (2006.01)
*A01N 47/40* (2006.01) *A01N 55/10* (2006.01)
*A01P 3/00* (2006.01) *C07D 405/12* (2006.01)
*C07D 413/12* (2006.01) *C07D 413/14* (2006.01)
*C07D 417/14* (2006.01) *C07D 487/04* (2006.01)

(86) International application number:
**PCT/JP2018/035113**

(87) International publication number:
**WO 2019/065516 (04.04.2019 Gazette 2019/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.09.2017 JP 2017184719**

(71) Applicant: **Nippon Soda Co., Ltd.**
**Tokyo 100-8165 (JP)**

(72) Inventors:
- **TAMAI, Tetsuo**
  **Joetsu-shi**
  **Niigata 949-2392 (JP)**
- **ITO, Syuichi**
  **Odawara-shi**
  **Kanagawa 250-0280 (JP)**
- **KOUBORI, Shinya**
  **Odawara-shi**
  **Kanagawa 250-0280 (JP)**
- **FUJII, Takayuki**
  **Odawara-shi**
  **Kanagawa 250-0280 (JP)**
- **NISHINO, Shigeki**
  **Yama-gun**
  **Fukushima 969-3302 (JP)**
- **WATANABE, Shinya**
  **Odawara-shi**
  **Kanagawa 250-0280 (JP)**
- **KATO, Hideki**
  **Odawara-shi**
  **Kanagawa 250-0280 (JP)**
- **MIYASHITA, Yasuhiro**
  **Odawara-shi**
  **Kanagawa 250-0280 (JP)**
- **MAKINO, Satoshi**
  **Odawara-shi**
  **Kanagawa 250-0280 (JP)**
- **NOMURA, Juri**
  **Odawara-shi**
  **Kanagawa 250-0280 (JP)**
- **KUNISHIMA, Mikiko**
  **Odawara-shi**
  **Kanagawa 250-0280 (JP)**
- **KAWASAKI, Tatsuhiro**
  **Odawara-shi**
  **Kanagawa 250-0280 (JP)**
- **SANO, Hiroshi**
  **Odawara-shi**
  **Kanagawa 250-0280 (JP)**

(74) Representative: **Wibbelmann, Jobst**
**Wuesthoff & Wuesthoff**
**Patentanwälte PartG mbB**
**Schweigerstrasse 2**
**81541 München (DE)**

(54) **QUINOLINE COMPOUND, AND BACTERICIDAL AGENT FOR AGRICULTURAL AND HORTICULTURAL USE**

(57) A compound represented by a formula (II), an N-oxide compound, or a salt thereof:



Printed by Jouve, 75001 PARIS (FR)

(Cont. next page)

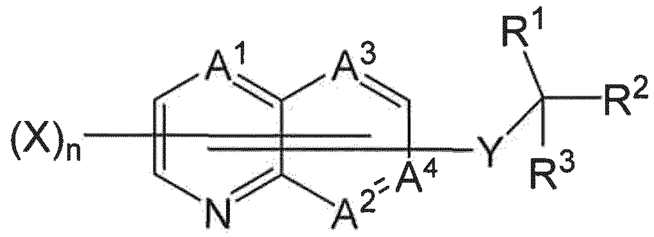

(II)

wherein $R^1$ is a hydrogen atom, a substituted or unsubstituted $C_{1-6}$ alkyl group, or a halogeno group; $R^2$ is a substituted or unsubstituted 5- to 6-membered heteroaryl group; $R^3$ is a substituted or unsubstituted $C_{6-10}$ aryl group or a substituted or unsubstituted 5- to 10-membered heteroaryl group; Y is an oxygen atom, a nitrogen atom, or a sulfur atom; $A^1$, $A^2$, $A^3$ and $A^4$ are each independently a carbon atom or a nitrogen atom, provided that two or more of $A^1$ to $A^4$ do not represent nitrogen atoms at the same time; X is a substituent; n represents the number of chemically acceptable substituents represented by X, and is any one of integers of 0 to 5; and when n is 2 or more, the substituents X may be the same or different from each other.

## Description

[Technical Field]

The present invention relates to a quinoline compound and an agricultural and horticultural fungicide. More specifically, the present invention relates to a quinoline compound having excellent fungicidal activity, excellent in safety, and can be synthesized in an industrially favorable manner; and an agricultural and horticultural fungicide containing this compound as an active ingredient.

Priority is claimed on Japanese Patent Application No. 2017-184719, filed September 26, 2017, the content of which is incorporated herein by reference.

[Background Art]

In the cultivation of agricultural and horticultural crops, a large number of control agents against crop diseases have been proposed. Most of the proposed control agents are not sufficiently satisfactory because of insufficient control efficacy, restrictions on their use due to the emergence of drug-resistant pathogens, occurrence of phytotoxicity or contamination in plant bodies, high toxicity to humans, animals and fish, or impact on the environment, and the like. For this reason, there is a strong demand for the advent of a control agent having few of these disadvantages that can also be used safely.

Patent Document 1 discloses 2-[(4-chloro-2-ethoxyphenoxy)(phenyl)methyl]morpholine and the like. It has been shown that the aforementioned compounds are used in agents for the treatment of urinary incontinence.

[Citation List]

[Patent Document]

[Patent Document 1] WO2005 / 105100A

[Summary of Invention]

[Technical Problem]

An object of the present invention is to provide a quinoline compound which is excellent in fungicidal activity, excellent in safety, and can be synthesized in an industrially favorable manner; and an agricultural and horticultural fungicide containing this compound as an active ingredient.

[Solution to Problem]

As a result of intensive studies in order to solve the above problems, the present invention including the following embodiments has been completed.

[1] A compound represented by a formula (II), an N-oxide compound, or a salt thereof:

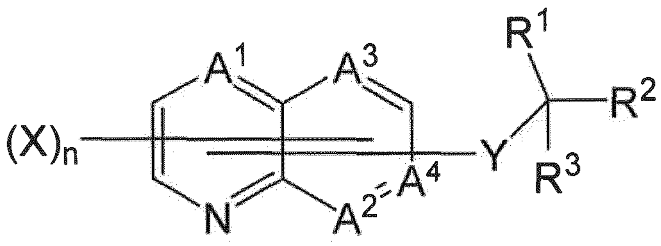

(II)

In the formula (II),

$R^1$ is a hydrogen atom, a substituted or unsubstituted $C_{1-6}$ alkyl group, or a halogeno group;
$R^2$ is a substituted or unsubstituted 5- to 6-membered heteroaryl group;
$R^3$ is a substituted or unsubstituted $C_{6-10}$ aryl group or a substituted or unsubstituted 5- to 10-membered heteroaryl group;

Y is an oxygen atom, a nitrogen atom, or a sulfur atom;
$A^1$, $A^2$, $A^3$ and $A^4$ are each independently a carbon atom or a nitrogen atom,
provided that two or more of $A^1$ to $A^4$ do not represent nitrogen atoms at the same time;
X is a substituent;
n represents the number of chemically acceptable substituents represented by X and is any one of integers of 0 to 5; and when n is 2 or more, the substituents X may be the same or different from each other.

[2] The compound according to [1], wherein the formula (II) is a formula (I), an N-oxide compound, or a salt thereof:

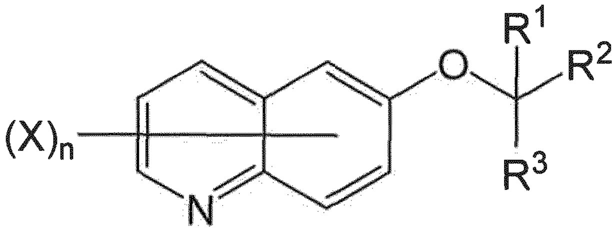

(I)

In the formula (I), $R^1$, $R^2$, $R^3$, X and n are the same as those defined in the formula (II).
[3] An agricultural and horticultural fungicide containing, as an active ingredient, at least one selected from the group consisting of the compound according to the above [1] or [2] and a salt thereof.

[Advantageous Effects of Invention]

The quinoline compound of the present invention and the salts thereof have excellent fungicidal activity and have reliable effects, are excellent in safety, and can be synthesized in an industrially favorable manner. The agricultural and horticultural fungicide of the present invention has an excellent control effect, does not cause phytotoxicity to plant bodies, and has little toxicity to humans, animals and fish as well as little impact on the environment.

[Description of Embodiments]

[Compound represented by formula (II)]

The quinoline compound of the present invention is a compound represented by a formula (II) (hereinafter, sometimes referred to as compound (II)) or a salt of the compound (II).

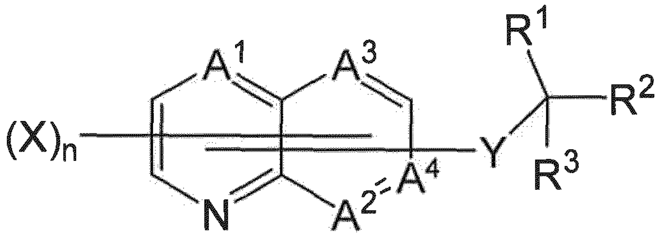

(II)

In the present invention, the term "unsubstituted" means that it is composed only of a group which becomes a mother nucleus. When it is described only by the name of the group which becomes the mother nucleus without being described as "substituted", it means "unsubstituted" unless otherwise stated.
On the other hand, the term "substituted" means that any hydrogen atom of the group which is to become the mother nucleus is substituted with a group (substituent) having the same or different structure as that of the mother nucleus. Therefore, a "substituent" is another group bonded to the group which becomes the mother nucleus. The number of substituents may be one, or two or more. The two or more substituents may be the same or different.
The terms "$C_{1-6}$" and the like mean that the number of carbon atoms in the group which becomes the mother nucleus is 1 to 6, and so on. The number of carbon atoms does not include the number of carbon atoms present in the substituent. For example, a butyl group having an ethoxy group as a substituent is classified as a C2 alkoxy C4 alkyl group.
A "substituent" is not particularly limited as long as it is chemically acceptable and has the effects of the present invention. Hereinafter, groups which can be a "substituent" are exemplified.
A $C_{1-6}$ alkyl group such as a methyl group, an ethyl group, an n-propyl group, an i-propyl group, an n-butyl

group, an s-butyl group, an i-butyl group, a t-butyl group, an n-pentyl group, and an n-hexyl group;
a $C_{2-6}$ alkenyl group such as a vinyl group, a 1-methylethenyl group, a 1-propenyl group, a 2-propenyl group (allyl group), a 1-butenyl group, a 2-butenyl group, a 3-butenyl group, a 1-methyl-2-propenyl group, a 2-methyl-1-propenyl group, and a 2-methyl-2-propenyl group;
a $C_{2-6}$ alkynyl group such as an ethynyl group, a 1-propynyl group, a 2-propynyl group, a 1-butynyl group, a 2-butynyl group, a 3-butynyl group, and a 1-methyl-2-propynyl group;
a $C_{3-8}$ cycloalkyl group such as a cyclopropyl group, a cyclobutyl group, a cyclopentyl group, a cyclohexyl group, and a cubanyl group;
a $C_{3-8}$ alkyl $C_{2-6}$ alkynyl group such as a cyclopropylethynyl group;
a $C_{6-10}$ aryl group such as a phenyl group and a naphthyl group;
a $C_{6-10}$ aryl $C_{1-6}$ alkyl group such as a benzyl group and a phenethyl group;
a 3- to 6-membered heterocyclyl group;
a 3- to 6-membered heterocyclyl $C_{1-6}$ alkyl group;
a hydroxyl group;
a $C_{1-6}$ alkoxy group such as a methoxy group, an ethoxy group, an n-propoxy group, an i-propoxy group, an n-butoxy group, an s-butoxy group, an i-butoxy group, and a t-butoxy group;
a $C_{2-6}$ alkenyloxy group such as a vinyloxy group, an allyloxy group, a propenyloxy group, and a butenyloxy group;
a $C_{2-6}$ alkynyloxy group such as an ethynyloxy group and a propargyloxy group;
a $C_{6-10}$ aryloxy group such as a phenoxy group and a naphthoxy group;
a $C_{6-10}$ aryl $C_{1-6}$ alkoxy group such as a benzyloxy group and a phenethyloxy group;
a 5- to 6-membered heteroaryloxy group such as a thiazolyloxy group and a pyridyloxy group;
a 5- to 6-membered heteroaryl $C_{1-6}$ alkyloxy group such as a thiazolylmethyloxy group and a pyridylmethyloxy group;
a $C_{1-6}$ alkoxy $C_{2-6}$ alkynyl group such as a 2-methoxy-1-propynyl group and a 2-ethoxy-1-propynyl group;
a formyl group;
a $C_{1-6}$ alkylcarbonyl group such as an acetyl group and a propionyl group;
a formyloxy group;
a $C_{1-6}$ alkylcarbonyloxy group such as an acetyloxy group and a propionyloxy group;
a $C_{6-10}$ arylcarbonyl group such as a benzoyl group;
a $C_{1-6}$ alkoxycarbonyl group such as a methoxycarbonyl group, an ethoxycarbonyl group, an n-propoxycarbonyl group, an i-propoxycarbonyl group, an n-butoxycarbonyl group and a t-butoxycarbonyl group;
a $C_{1-6}$ alkoxycarbonyloxy group such as a methoxycarbonyloxy group, an ethoxycarbonyloxy group, an n-propoxycarbonyloxy group, an i-propoxycarbonyloxy group, an n-butoxycarbonyloxy group and a t-butoxycarbonyloxy group;
a carboxyl group;
a halogeno group such as a fluoro group, a chloro group, a bromo group, and an iodo group;
a $C_{1-6}$ haloalkyl group such as a fluoromethyl group, a difluoromethyl group, a trifluoromethyl group, a 2,2,2-trifluoroethyl group, a pentafluoroethyl group, a 3,3,3-trifluoropropyl group, a 2,2,3,3,3-pentafluoropropyl group, a perfluoropropyl group, a 2,2,2-trifluoro-1-trifluoromethylethyl group, a perfluoroisopropyl group, a 4-fluorobutyl group, a 2,2,3,3,4,4,4-heptafluorobutyl group, a perfluorobutyl group, a perfluoropentyl group, a perfluorohexyl group, a chloromethyl group, a bromomethyl group, a dichloromethyl group, a dibromomethyl group, a trichloromethyl group, a tribromomethyl group, a 1-chloroethyl group, a 2,2,2-trichloroethyl group, a 4-chlorobutyl group, a perchlorohexyl group and a 2,4,6-trichlorohexyl group;
a $C_{2-6}$ haloalkenyl group such as a 2,2-difluorovinyl group, a 2,2-dichlorovinyl group, a 2,2-dibromovinyl group, a 2-chloro-1-propenyl group, a 3,3,3-trifluoro-1-propenyl group and a 2-fluoro-1-butenyl group;
a $C_{2-6}$ haloalkynyl group such as a 4,4-dichloro-1-butynyl group, a 4-fluoro-1-pentynyl group, and a 5-bromo-2-pentynyl group;
a $C_{1-6}$ haloalkoxy group such as a difluoromethoxy group, a trifluoromethoxy group, a 2-chloro-n-propoxy group, and a 2,3-dichlorobutoxy group;
a $C_{2-6}$ haloalkenyloxy group such as a 2-chloropropenyloxy group and a 3-bromobutenyloxy group;
a $C_{1-6}$ haloalkylcarbonyl group such as a chloroacetyl group, a trifluoroacetyl group and a trichloroacetyl group;
an amino group;
a $C_{1-6}$ alkylamino group such as a methylamino group, a dimethylamino group, a diethylamino group and a t-butylamino group;
a $C_{6-10}$ arylamino group such as an anilino group and a naphthylamino group;
a $C_{6-10}$ aryl $C_{1-6}$ alkylamino group such as a benzylamino group and a phenethylamino group;
a formylamino group;
a $C_{1-6}$ alkylcarbonylamino group such as an acetylamino group, a diacetylamino group, a propanoylamino group, a butyrylamino group and an i-propylcarbonylamino group;
a $C_{1-6}$ alkoxycarbonylamino group such as a methoxycarbonylamino group, an ethoxycarbonylamino group, an n-propoxycarbonylamino group and an i-propoxycarbonylamino group;
an unsubstituted or substituted aminocarbonyl group such as an aminocarbonyl group, a dimethylaminocarbonyl group, a phenylaminocarbonyl group and an N-phenyl-N-methylaminocarbonyl group;
an imino $C_{1-6}$ alkyl group such as an iminomethyl group, a (1-imino)ethyl group and a (1-imino)-n-propyl group;
a hydroxyimino $C_{1-6}$ alkyl group such as a hydroxyiminomethyl group, a 1-(hydroxyimino) ethyl group and a 2-(hydroxyimino) ethyl group;
a $C_{1-6}$ alkoxyimino $C_{1-6}$ alkyl group such as a methoxyiminomethyl group, a 1-(methoxyimino) ethyl group, a 2-(methoxyimino) ethyl group, an ethoxyiminomethyl group, a 1-(ethoxyimino) ethyl group, a 2-(ethoxyimino) ethyl group, an n-propoxyiminomethyl group, a 1-(n-propoxyimino) ethyl group, a 2-(n-propoxyimino) ethyl group, an i-propoxyiminomethyl group, a 1-(i-propoxyimino) ethyl group and a 2-(i-propoxyimino) ethyl group;
a $C_{1-6}$ haloalkoxyimino $C_{1-6}$ alkyl group such as a trifluoromethoxyiminomethyl group, a 1-(trifluoromethoxyimino) ethyl group and a 2-(trifluoromethoxyimino) ethyl group;
an aminocarbonyloxy group;
a $C_{1-6}$ alkyl-substituted aminocarbonyloxy group such as an ethylaminocarbonyloxy group, and a dimethylaminocarbonyloxy group;
a mercapto group;
a $C_{1-6}$ alkylthio group such as a methylthio group, an ethylthio group, an n-propylthio group, an i-propylthio group, an n-butylthio group, an i-butylthio group, an s-butylthio group and a t-butylthio group;
a $C_{1-6}$ haloalkylthio group such as a trifluoromethylthio group and a 2,2,2-trifluoroethylthio group;
a $C_{6-10}$ arylthio group such as a phenylthio group and a naphthylthio group;
a $C_{6-10}$ aryl $C_{1-6}$ alkylthio group such as a benzylthio group;
a 5- to 6-membered heteroarylthio group such as a thiazolylthio group and a pyridylthio group;
a $C_{1-6}$ alkylsulfinyl group such as a methylsulfinyl group, an ethylsulfinyl group and a t-butylsulfinyl group;
a $C_{1-6}$ haloalkylsulfinyl group such as a trifluoromethylsulfinyl group and a 2,2,2-trifluoroethylsulfinyl group;
a $C_{6-10}$ arylsulfinyl group such as a phenylsulfinyl group;
a 5- to 6-membered heteroarylsulfinyl group such as a thiazolylsulfinyl group and a pyridylsulfinyl group;
a $C_{1-6}$ alkylsulfonyl group such as a methylsulfonyl group, an ethylsulfonyl group and a t-butylsulfonyl group;
a $C_{1-6}$ haloalkylsulfonyl group such as a trifluoromethylsulfonyl group and a 2,2,2-trifluoroethylsulfonyl group;
a $C_{6-10}$ arylsulfonyl group such as a phenylsulfonyl group;
a 5- to 6-membered heteroarylsulfonyl group such as a thiazolylsulfonyl group and a pyridylsulfonyl group;
a $C_{1-6}$ alkylsulfonyloxy group such as a methylsulfonyloxy group, an ethylsulfonyloxy group and a t-butylsulfonyloxy group;
a $C_{1-6}$ haloalkylsulfonyloxy group such as a trifluoromethylsulfonyloxy group and a 2,2,2-trifluoroethylsulfonyloxy group;
an N-cyano-S-$C_{1-6}$ alkylsulfinimidoyl group such as an N-cyano-S-methylsulfinimidoyl group; an N-cyano-S-$C_{1-6}$ alkylsulfonimidoyl group such as an N-cyano-S-methylsulfonimidoyl group;
a tri $C_{1-6}$ alkyl-substituted silyl group such as a trimethylsilyl group, a triethylsilyl group and a t-butyldimethylsilyl group;
a tri $C_{6-10}$ aryl-substituted silyl group such as a triphenylsilyl group;
a cyano group; a nitro group.

Further, in these "substituents", any hydrogen atom in the substituent may be substituted with a group having a different structure. Examples of the "substituent" in this case include a $C_{1-6}$ alkyl group, a $C_{1-6}$ haloalkyl group, a hydroxyl group, a $C_{1-6}$ alkoxy group, a $C_{1-6}$ haloalkoxy group, a hydroxyl group, a $C_{1-6}$ alkoxy group, a $C_{1-6}$ alkylcarbonyloxy group, a $C_{1-6}$ alkylthio group, a $C_{1-6}$ alkylsulfinyl group, a $C_{1-6}$ alkylsulfonyl group, a $C_{6-10}$ aryl group, a tri$C_{1-6}$ alkyl-substituted silyl group, a halogeno group, a cyano group and a nitro group.

Further, the above-described "3- to 6-membered heterocyclyl group" includes 1 to 4 hetero atoms selected from the group consisting of a nitrogen atom, an oxygen atom and a sulfur atom as constituent atoms of the ring. The heterocyclyl group may be either monocyclic or polycyclic. As long as the polycyclic heterocyclyl group includes at least one heterocyclic ring, the remaining ring may be any of a saturated alicyclic ring, an unsaturated alicyclic ring or an aromatic ring. Examples of the "3- to 6-membered heterocyclyl group" include a 3- to 6-membered saturated heterocyclyl group, a 5- to 6-membered heteroaryl group, and a 5- to 6-membered partially unsaturated heterocyclyl group.

Examples of the 3- to 6-membered saturated heterocyclyl group include an aziridinyl group, an epoxy group, a pyrrolidinyl group, a tetrahydrofuranyl group, a thiazolidinyl group, a piperidyl group, a piperazinyl group, a morpholinyl group, a dioxolanyl group, and a dioxanyl group.

Examples of the 5-membered heteroaryl group include a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group and a tetrazolyl group.

Examples of the 6-membered heteroaryl group include a pyridyl group, a pyrazinyl group, a pyrimidinyl group, a pyridazinyl group and a triazinyl group.

Examples of the 5- to 6-membered partially unsaturated heterocyclyl group include a 4,5-dihydroisoxazolyl group.

[$A^1$, $A^2$, $A^3$, $A^4$]

In the formula (II), $A^1$, $A^2$, $A^3$ and $A^4$ are each independently a carbon atom or a nitrogen atom. However, two or more of $A^1$ to $A^4$ do not represent nitrogen atoms at the same time.

That is, the compound represented by the formula (II) is a compound represented by formulas (II-1) to (II-5).

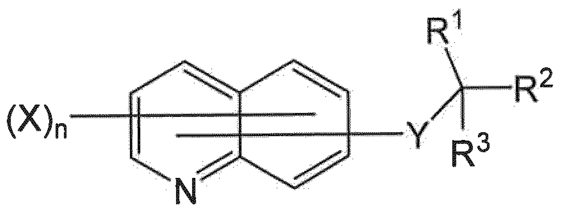

(II-1)

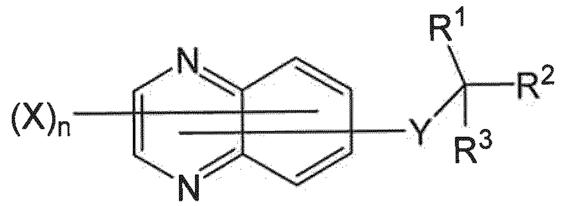

(II-2)

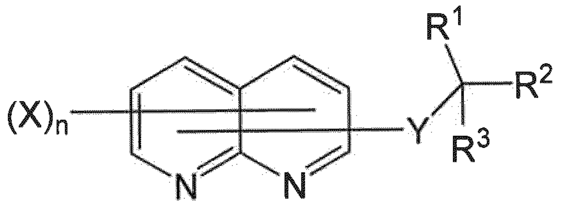

(II-3)

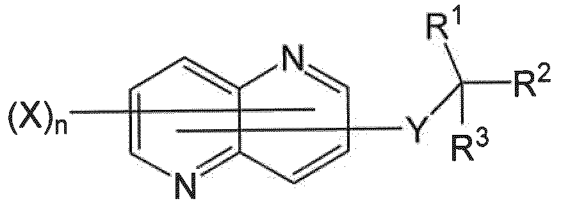

(II-4)

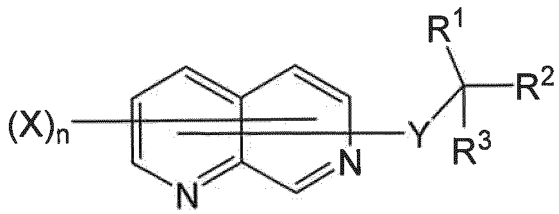

(II-5)

In the formulas (II-1) to (II-5), $R^1$, $R^2$, $R^3$, Y, X and n are the same as those defined in the formula (II).The compound represented by the formula (II) is preferably a compound represented by the formula (II-1).

[$R^1$]

In the formula (II), $R^1$ represents a hydrogen atom, a substituted or unsubstituted $C_{1-6}$ alkyl group or a halogeno group.

The "$C_{1-6}$ alkyl group" represented by $R^1$ may be linear or branched. Examples of the alkyl group include a methyl group, an ethyl group, an n-propyl group, an n-butyl group, an n-pentyl group, an n-hexyl group, an i-propyl group, an i-butyl group, an s-butyl group, a t-butyl group, an i-pentyl group, a neopentyl group, a 2-methylbutyl group, a 2,2-dimethylpropyl group and an i-hexyl group.

Specific examples of "$C_{1-6}$ alkyl group having a substituent" include:

a $C_{1-6}$ haloalkyl group such as a fluoromethyl group, a chloromethyl group, a bromomethyl group, a difluoromethyl group, a dichloromethyl group, a dibromomethyl group, a trifluoromethyl group, a trichloromethyl group, a tribromomethyl group, a 1-chloroethyl group, a 2,2,2-trifluoroethyl group, a 2,2,2-trichloroethyl group, a pentafluoroethyl group, a 4-fluorobutyl group, a 2,2,3,3,4,4,4-heptafluorobutyl group, a 4-chlorobutyl group, a 3,3,3-trifluoropropyl group, a 2,2,3,3,3-pentafluoropropyl group, a 1,1,1,3,3,3-hexafluoropropan-2-yl group, a perfluoropropan-2-yl group, a perfluorohexyl group, a perchlorohexyl group and a 2,4,6-trichlorohexyl group;

a hydroxy $C_{1-6}$ alkyl group such as a hydroxymethyl group and a hydroxyethyl group;

a $C_{1-6}$ alkoxy $C_{1-6}$ alkyl group such as a methoxymethyl group, an ethoxymethyl group, a methoxyethyl group, an ethoxyethyl group, a methoxy-n-propyl group, an n-propoxymethyl group, an i-propoxyethyl group, an s-butoxymethyl

group and a t-butoxyethyl group;

a $C_{6-10}$ aryl $C_{1-6}$ alkyl group such as a benzyl group and a phenethyl group;

a $C_{3-8}$ cycloalkyl $C_{1-6}$ alkyl group such as a cyclopropylmethyl group, a 2-cyclopropylethyl group, a cyclopentylmethyl group, a 2-cyclohexylethyl group and a 2-cyclooctylethyl group; and the like.

Preferred examples of the substituent on the "$C_{1-6}$ alkyl group" represented by $R^1$ include a halogeno group such as a fluoro group, a chloro group, a bromo group and an iodo group; a $C_{1-6}$ alkoxy group such as a methoxy group, an ethoxy group, an n-propoxy group, an i-propoxy group, an n-butoxy group, an s-butoxy group, an i-butoxy group and a t-butoxy group; a $C_{1-6}$ haloalkoxy group such as a 2-chloro-n-propoxy group, a 2,3-dichlorobutoxy group and a trifluoromethoxy group; and a cyano group.

As the "halogeno group" represented by $R^1$, a fluoro group, a chloro group, a bromo group, an iodo group and the like can be mentioned.

$R^1$ is preferably a hydrogen atom.

[$R^2$]

In the formula (II), $R^2$ represents a substituted or unsubstituted 5- to 6-membered heteroaryl group.

Examples of the "5- to 6-membered heteroaryl group" represented by $R^2$ include 5-membered heteroaryl groups such as a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group and a tetrazolyl group; and 6-membered heteroaryl groups such as a pyridyl group, a pyrazinyl group, a pyrimidinyl group, a pyridazinyl group and a triazinyl group.

Preferred examples of the substituent on the "5- to 6-membered heteroaryl group" represented by $R^2$ include a halogeno group such as a fluoro group, a chloro group, a bromo group and an iodo group; a $C_{1-6}$ alkyl group such as a methyl group, an ethyl group, an n-propyl group, an i-propyl group, an n-butyl group, an s-butyl group, an i-butyl group, a t-butyl group, an n-pentyl group and an n-hexyl group; a hydroxyl-substituted $C_{1-6}$ alkyl group such as a hydroxymethyl group and a 2-hydroxypropan-2-yl group; a $C_{1-6}$ alkoxycarbonyl group such as a methoxycarbonyl group, an ethoxycarbonyl group, an n-propoxycarbonyl group, an i-propoxycarbonyl group, an n-butoxycarbonyl group and a t-butoxycarbonyl group; and a $C_{1-6}$ alkylcarbonylamino group such as a diacetylamino group.

$R^2$ is preferably a 5- to 6-membered heteroaryl group, more preferably a furyl group, an oxazolyl group, an isoxazolyl group, a thiazolyl group, an oxadiazolyl group, a thiadiazolyl group, or a pyrazinyl group, and particularly preferably an oxadiazolyl group.

[$R^3$]

In the formula (II), $R^3$ represents a substituted or unsubstituted $C_{6-10}$ aryl group or a substituted or unsubstituted 5- to 10-membered heteroaryl group.

The "$C_{6-10}$ aryl group" represented by $R^3$ is a group formed by eliminating one hydrogen on the ring of a monocyclic or polycyclic aromatic hydrocarbon. Examples of the "$C_{6-10}$ aryl group" include a phenyl group and a naphthyl group. Of these, a phenyl group is preferred.

Examples of the "5- to 10-membered heteroaryl group" represented by $R^3$ include 5-membered heteroaryl groups such as a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group and a tetrazolyl group; 6-membered heteroaryl groups such as a pyridyl group, a pyrazinyl group, a pyrimidinyl group, a pyridazinyl group and a triazinyl group; 9-membered heteroaryl groups such as an indolyl group, an isoindolyl group, a benzofuranyl group, a dihydrobenzofuranyl group, an indazolyl group, a benzoxazolyl group, a benzoisoxazolyl group, a benzothiazolyl group, a benzoisothiazolyl group, a pyridylpyrazole group and a triazolopyridyl group; and 10-membered heteroaryl groups such as a quinolinyl group, an isoquinolinyl group, a cinnolinyl group, a phthalazinyl group, a quinazolinyl group, and a quinoxalinyl group. Among these, a thienyl group, a pyrazolyl group, a thiazolyl group, an oxadiazolyl group, a pyridyl group, a pyrazinyl group, a pyrimidinyl group, a pyridazinyl group and an isoquinolinyl group are preferable, and a pyridyl group is particularly preferable.

Preferred examples of the substituent on the "$C_{6-10}$ aryl group" and the "5- to 10-membered heteroaryl group" represented by $R^3$ include a halogeno group such as a fluoro group, a chloro group, a bromo group and an iodo group; a $C_{1-6}$ alkyl group such as a methyl group, an ethyl group, an n-propyl group, an i-propyl group, an n-butyl group, an s-butyl group, an i-butyl group, a t-butyl group, an n-pentyl group and an n-hexyl group; a $C_{1-6}$ haloalkyl group such as a chloromethyl group, a chloroethyl group, a trifluoromethyl group, a 1,2-dichloro-n-propyl group, a 1-fluoro-n-butyl group and a perfluoro-n-pentyl group; a $C_{1-6}$ alkoxy $C_{1-6}$ alkyl group such as a methoxymethyl group, a dimethoxymethyl group, an ethoxymethyl group, a diethoxymethyl group, an i-propoxymethyl group and a 2-methoxyethyl group; a $C_{1-6}$ alkylthio

$C_{1-6}$ alkyl group such as a methylthiomethyl group, an ethylthiomethyl group and a 2-methylthioethyl group; a $C_{2-6}$ alkenyl group such as a vinyl group, a 1-propenyl group, a 2-propenyl group, a 1-butenyl group, a 2-butenyl group, a 3-butenyl group, a 1-methyl-2-propenyl group, a 2-methyl-2-propenyl group, a 1-pentenyl group, a 2-pentenyl group, a 3-pentenyl group, a 4-pentenyl group, a 1-methyl-2-butenyl group, a 2-methyl-2-butenyl group, a 1-hexenyl group, a 2-hexenyl group, a 3-hexenyl group, a 4-hexenyl group and a 5-hexenyl group; a $C_{2-6}$ alkynyl group such as an ethynyl group, a 1-propynyl group, a 2-propynyl group, a 1-butynyl group, a 2-butynyl group, a 3-butynyl group, a 1-methyl-2-propynyl group, a 2-methyl-3-butynyl group, a 1-pentynyl group, a 2-pentynyl group, a 3-pentynyl group, a 4-pentynyl group, a 1-methyl-2-butynyl group, a 2-methyl-3-pentynyl group, a 1-hexynyl group and a 1,1-dimethyl-2-butynyl group; a $C_{1-6}$ alkoxy $C_{2-6}$ alkynyl group such as a 2-methoxy-1-propynyl group and 2-ethoxy-1-propynyl group; a $C_{1-6}$ alkoxy group such as a methoxy group, an ethoxy group, an n-propoxy group, an i-propoxy group, an n-butoxy group, an s-butoxy group, an i-butoxy group and a t-butoxy group; a $C_{1-6}$ haloalkoxy group such as a 2-chloro-n-propoxy group, a 2,3-dichlorobutoxy group, a difluoromethoxy group and a trifluoromethoxy group; a $C_{1-6}$ alkoxycarbonyl group such as a methoxycarbonyl group, an ethoxycarbonyl group, an n-propoxycarbonyl group, an i-propoxycarbonyl group, an n-butoxycarbonyl group and a t-butoxycarbonyl group; a $C_{1-6}$ alkylthio group such as a methylthio group, an ethylthio group, an n-propylthio group, an i-propylthio group, an n-butylthio group, an i-butylthio group, an s-butylthio group, a t-butylthio group, an n-pentylthio group and an n-hexylthio group; a benzylthio group; a 4-methoxy-benzylthio group; a $C_{1-6}$ haloalkylthio group such as a trifluoromethylthio group and a 2,2,2-trifluoroethylthio group; a $C_{1-6}$ alkylsulfinyl group such as a methylsulfinyl group, an ethylsulfinyl group and a t-butylsulfinyl group; a $C_{1-6}$ alkylsulfonyl group such as a methylsulfonyl group, an ethylsulfonyl group and a t-butylsulfonyl group; a $C_{1-6}$ alkoxyimino $C_{1-6}$ alkyl group such as a methoxyiminomethyl group, an ethoxyiminomethyl group, an n-propoxyiminomethyl group, an i-propoxyiminomethyl group and a (1-methoxyimino) ethyl group; a 2-propynyloxyiminomethyl group; a phenyl group; a 5-membered heteroaryl group such as a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group and a tetrazolyl group; a cyclic ether group such as an oxiranyl group, a tetrahydrofuryl group, a dioxolanyl group and a dioxanyl group; a cyano group; and a nitro group.

[Y]

In the formula (II), Y is an oxygen atom, a nitrogen atom or a sulfur atom.
Y is preferably an oxygen atom.

[X]

In the formula (II), X represents a substituent. The substituent represented by X is as described above.
Preferred examples of the substituent represented by X (hereinafter sometimes described as G) include a substituted or unsubstituted $C_{1-6}$ alkyl group, a substituted or unsubstituted $C_{2-6}$ alkenyl group, a substituted or unsubstituted $C_{2-6}$ alkynyl group, a hydroxyl group, a substituted or unsubstituted $C_{1-6}$ alkoxy group, a formyl group, a substituted or unsubstituted $C_{1-6}$ alkylcarbonyl group, a substituted or unsubstituted $C_{1-6}$ alkoxycarbonyl group, a substituted or unsubstituted $C_{1-6}$ alkylthio group, a substituted or unsubstituted $C_{1-6}$ alkylsulfinyl group, a substituted or unsubstituted $C_{1-6}$ alkylsulfonyl group, an N-cyano-S-$C_{1-6}$ alkylsulfinimidoyl group, an N-cyano-S-$C_{1-6}$ alkylsulfonimidoyl group, a substituted or unsubstituted $C_{3-8}$ cycloalkyl group, a substituted or unsubstituted $C_{6-10}$ aryl group, a substituted or unsubstituted 3- to 6-membered heterocyclyl group, an amino group, a substituted or unsubstituted $C_{1-6}$ alkylamino group, a halogeno group and a cyano group.
As the "$C_{1-6}$ alkyl group" and "halogeno group" represented by G, the same as those specifically exemplified for $R^1$ can be mentioned.
Examples of the "substituted $C_{1-6}$ alkyl group" represented by G include a $C_{1-6}$ haloalkyl group such as a fluoromethyl group, a difluoromethyl group, a trifluoromethyl group, a 2,2,2-trifluoroethyl group, a pentafluoroethyl group, a 3,3,3-trifluoropropyl group, a 2,2,3,3,3-pentafluoropropyl group, a perfluoropropyl group, a 2,2,2-trifluoro-1-trifluoromethylethyl group, a perfluoroisopropyl group, a 4-fluorobutyl group, a 2,2,3,3,4,4,4-heptafluorobutyl group, a perfluorobutyl group, a perfluoropentyl group, a perfluorohexyl group, a chloromethyl group, a bromomethyl group, a dichloromethyl group, a dibromomethyl group, a trichloromethyl group, a tribromomethyl group, a 1-chloroethyl group, a 2,2,2-trichloroethyl group, a 4-chlorobutyl group, a perchlorohexyl group and a 2,4,6-trichlorohexyl group; a $C_{6-10}$ aryl $C_{1-6}$ alkyl group such as a benzyl group and a phenethyl group; a hydroxy $C_{1-6}$ alkyl group such as a hydroxymethyl group, a 1-hydroxyethyl group and a 2-hydroxyethyl group; a $C_{1-6}$ alkoxy $C_{1-6}$ alkyl group such as a methoxymethyl group, a dimethoxymethyl group, an ethoxymethyl group, a diethoxymethyl group and an i-propoxymethyl group; a $C_{1-6}$ alkylcarbonyloxy $C_{1-6}$ alkyl group such as an acetyloxymethyl group and a propionyloxymethyl group; a $C_{1-6}$ alkylthio $C_{1-6}$ alkyl group such as a methylthiomethyl group and an ethylthiomethyl group; a $C_{1-6}$ alkylsulfinyl $C_{1-6}$ alkyl group such as a methylsulfinylmethyl group; a $C_{1-6}$ alkylsulfonyl $C_{1-6}$ alkyl group such as methylsulfonylmethyl group; a 2-(trimethylsilyl)

ethyl group; a $C_{1-6}$ alkoxyimino $C_{1-6}$ alkyl group such as a methoxyiminomethyl group, a 1-(methoxyimino) ethyl group, a 2-(methoxyimino) ethyl group, an ethoxyiminomethyl group, a 1-(ethoxyimino) ethyl group, a 2-(ethoxyimino) ethyl group, an n-propoxyiminomethyl group, a 1-(n-propoxyimino) ethyl group, a 2- (n-propoxyimino) ethyl group, an i-propoxyiminomethyl group, a 1-(i-propoxyimino) ethyl group and a 2-(i-propoxyimino) ethyl group; and a $C_{1-6}$ haloalkoxyimino $C_{1-6}$ alkyl group such as a trifluoromethoxyiminomethyl group, 1-(trifluoromethoxyimino) ethyl group and a 2-(trifluoromethoxyimino) ethyl group.

Preferred examples of the substituent on the "$C_{1-6}$ alkyl group" represented by G include a halogeno group such as a fluoro group, a chloro group, a bromo group and an iodo group; a phenyl group; a hydroxyl group; a $C_{1-6}$ alkoxy group such as a methoxy group, an ethoxy group, an n-propoxy group, an i-propoxy group, an n-butoxy group, an s-butoxy group, an i-butoxy group and a t-butoxy group; a $C_{1-6}$ alkylcarbonyloxy group such as an acetyloxy group and a propionyloxy group; a $C_{1-6}$ alkylthio group such as a methylthio group, an ethylthio group, an n-propylthio group, an i-propylthio group, an n-butylthio group, an i-butylthio group, an s-butylthio group and a t-butylthio group; a $C_{1-6}$ alkylsulfinyl group such as a methylsulfinyl group, an ethylsulfinyl group and a t-butylsulfinyl group; a $C_{1-6}$ alkylsulfonyl group such as a methylsulfonyl group, an ethylsulfonyl group and a t-butylsulfonyl group; a $C_{1-6}$ alkoxyimino group such as a methoxyimino group, an ethoxyimino group, an n-propoxyimino group, an i-propoxyimino group and an n-butoxyimino group; and a $C_{1-6}$ haloalkoxyimino group such as a trifluoromethoxyimino group.

Examples of the "$C_{2-6}$ alkenyl group" represented by G include a vinyl group, a 1-methylethenyl group, a 1-propenyl group, a 2-propenyl group (allyl group), a 1-butenyl group, a 2-butenyl group, a 3-butenyl group, a 1-methyl-2-propenyl group, a 2-methyl-1-propenyl group and a 2-methyl-2-propenyl group.

Examples of the "substituted $C_{2-6}$ alkenyl group" represented by G include a $C_{2-6}$ haloalkenyl group such as a 2,2-difluorovinyl group, a 2,2-dichlorovinyl group, a 2,2-dibromovinyl group, a 2-chloro-1-propenyl group, a 3,3,3-trifluoro-1-propenyl group and a 2-fluoro-1-butenyl group.

Examples of the "$C_{2-6}$ alkynyl group" represented by G include an ethynyl group, a 1-propynyl group, a 2-propynyl group, a 1-butynyl group, a 2-butynyl group, a 3-butynyl group, a 1-methyl-2-propynyl group, a 2-methyl-3-butynyl group, a 1-pentynyl group, a 2-pentynyl group, a 3-pentynyl group, a 4-pentynyl group, a 1-methyl-2-butynyl group, a 2-methyl-3-pentynyl group, a 1-hexynyl group and a 1,1-dimethyl-2-butynyl group.

Examples of the "substituted $C_{2-6}$ alkynyl group" represented by G include a $C_{3-8}$ alkyl $C_{2-6}$ alkynyl group such as a cyclopropylethynyl group; and a tri $C_{1-6}$ alkyl-substituted silyl $C_{2-6}$ alkynyl group such as a trimethylsilylethynyl group.

Examples of the "$C_{1-6}$ alkoxy group" represented by G include a methoxy group, an ethoxy group, an n-propoxy group, an i-propoxy group, an n-butoxy group, an s-butoxy group, an i-butoxy group and a t-butoxy group.

Examples of the "$C_{1-6}$ alkylcarbonyl group" represented by G include an acetyl group and a propionyl group.

Examples of the "$C_{1-6}$ alkoxycarbonyl group" represented by G include a methoxycarbonyl group, an ethoxycarbonyl group, an n-propoxycarbonyl group, an i-propoxycarbonyl group, an n-butoxycarbonyl group and a t-butoxycarbonyl group.

Examples of the "$C_{1-6}$ alkylthio group" represented by G include a methylthio group, an ethylthio group, an n-propylthio group, an n-butylthio group, an n-pentylthio group, an n-hexylthio group and an i-propylthio group.

Examples of the "$C_{1-6}$ alkylsulfinyl group" represented by G include a methylsulfinyl group, an ethylsulfinyl group and a t-butylsulfinyl group.

Examples of the "$C_{1-6}$ alkylsulfonyl group" represented by G include a methylsulfonyl group, an ethylsulfonyl group and a t-butylsulfonyl group.

Examples of the "N-cyano-S-$C_{1-6}$ alkylsulfinimidoyl group" represented by G include an N-cyano-S-methylsulfinimidoyl group.

Examples of the "N-cyano-S-$C_{1-6}$ alkylsulfonimidoyl group" represented by G include an N-cyano-S-methylsulfonimidoyl group.

Examples of the "alkylamino group" represented by G include a methylamino group, an ethylamino group, a t-butylamino group, a dimethylamino group and a diethylamino group.

Preferred examples of the substituent on the "$C_{2-6}$ alkynyl group", "$C_{1-6}$ alkoxy group", "$C_{1-6}$ alkoxycarbonyl group", "$C_{1-6}$ alkylthio group", "$C_{1-6}$ alkylsulfinyl group", "$C_{1-6}$ alkylsulfonyl group" and "alkylamino group" represented by G include a halogeno group such as a fluoro group, a chloro group, a bromo group and an iodo group; and a trimethylsilyl group.

Examples of the "$C_{3-8}$ cycloalkyl group" represented by G include a cyclopropyl group, a cyclobutyl group, a cyclopentyl group, a cyclohexyl group, a cycloheptyl group and a cubanyl group.

Preferred examples of the substituent on the "$C_{3-8}$ cycloalkyl group" represented by G include a halogeno group such as a fluoro group, a chloro group, a bromo group and an iodo group; and a $C_{1-6}$ alkyl group such as a methyl group, an ethyl group, an n-propyl group, an i-propyl group, an n-butyl group, an s-butyl group, an i-butyl group, a t-butyl group, an n-pentyl group and an n-hexyl group.

As the "$C_{6-10}$ aryl group" represented by G, the same as those specifically exemplified for $R^3$ can be mentioned.

Preferred examples of the substituent on the "$C_{6-10}$ aryl group" represented by G include a halogeno group

such as a fluoro group, a chloro group, a bromo group and an iodo group; a $C_{1-6}$ alkyl group such as a methyl group, an ethyl group, an n-propyl group, an i-propyl group, an n-butyl group, an s-butyl group, an i-butyl group, a t-butyl group, an n-pentyl group and an n-hexyl group; a $C_{1-6}$ haloalkyl group such as a fluoromethyl group, a difluoromethyl group, a trifluoromethyl group, a 2,2,2-trifluoroethyl group, a pentafluoroethyl group, a 3,3,3-trifluoropropyl group, a 2,2,3,3,3-pentafluoropropyl group, a perfluoropropyl group, a 2,2,2-trifluoro-1-trifluoromethylethyl group, a perfluoroisopropyl group, a 4-fluorobutyl group, a 2,2,3,3,4,4,4-heptafluorobutyl group, a perfluorobutyl group, a perfluoropentyl group, a perfluorohexyl group, a chloromethyl group, a bromomethyl group, a dichloromethyl group, a dibromomethyl group, a trichloromethyl group, a tribromomethyl group, a 1-chloroethyl group, a 2,2,2-trichloroethyl group, a 4-chlorobutyl group, a perchlorohexyl group and a 2,4,6-trichlorohexyl group; a $C_{1-6}$ alkoxy group such as a methoxy group, an ethoxy group, an n-propoxy group, an i-propoxy group, an n-butoxy group, an s-butoxy group, an i-butoxy group and a t-butoxy group; a $C_{1-6}$ alkylthio group such as a methylthio group, an ethylthio group, an n-propylthio group, an n-butylthio group, an n-pentylthio group, an n-hexylthio group and an i-propylthio group; a $C_{1-6}$ alkylsulfinyl group such as a methylsulfinyl group, an ethylsulfinyl group and a t-butylsulfinyl group; a $C_{1-6}$ alkylsulfonyl group such as a methylsulfonyl group, an ethylsulfonyl group and a t-butylsulfonyl group; and a cyano group.

Examples of the "3- to 6-membered heterocyclyl group" represented by G include a 3- to 6-membered saturated heterocyclyl group, a 5- to 6-membered heteroaryl group, and a 5- to 6-membered partially unsaturated heterocyclyl group.

Examples of the "3- to 6-membered saturated heterocyclyl group" include an aziridinyl group, an epoxy group, a pyrrolidinyl group, a tetrahydrofuranyl group, a thiazolidinyl group, a piperidyl group, a piperazinyl group, a morpholinyl group, a dioxolanyl group and a dioxanyl group.

Examples of the "5-membered heteroaryl group" include a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group and a tetrazolyl group.

Examples of the "6-membered heteroaryl group" include a pyridyl group, a pyrazinyl group, a pyrimidinyl group, a pyridazinyl group and a triazinyl group.

Examples of the "5- to 6-membered partially unsaturated heterocyclyl group" include a 4,5-dihydroisoxazolyl group.

Preferred examples of the substituent on the "3- to 6-membered heterocyclyl group" represented by G include a halogeno group such as a fluoro group, a chloro group, a bromo group and an iodo group; a $C_{1-6}$ alkyl group such as a methyl group, an ethyl group, an n-propyl group, an i-propyl group, an n-butyl group, an s-butyl group, an i-butyl group, a t-butyl group, an n-pentyl group and an n-hexyl group; and a $C_{1-6}$ haloalkyl group such as a fluoromethyl group, a difluoromethyl group, a trifluoromethyl group, a 2,2,2-trifluoroethyl group, a pentafluoroethyl group, a 3,3,3-trifluoropropyl group, a 2,2,3,3,3-pentafluoropropyl group, a perfluoropropyl group, a 2,2,2-trifluoro-1-trifluoromethylethyl group, a perfluoroisopropyl group, a 4-fluorobutyl group, a 2,2,3,3,4,4,4-heptafluorobutyl group, a perfluorobutyl group, a perfluoropentyl group, a perfluorohexyl group, a chloromethyl group, a bromomethyl group, a dichloromethyl group, a dibromomethyl group, a trichloromethyl group, a tribromomethyl group, a 1-chloroethyl group, a 2,2,2-trichloroethyl group, a 4-chlorobutyl group, a perchlorohexyl group and a 2,4,6-trichlorohexyl group.

[n]

In the formula (II), n represents the number of chemically acceptable substituents represented by X and is an integer of 0 to 5. When n is 2 or more, the substituents X may be the same or different from each other.

n is preferably from 0 to 2, and more preferably 0 or 1.

The salt of compound (II) is not particularly limited as long as it is an agriculturally and horticulturally acceptable salt. Examples thereof include salts of inorganic acids such as hydrochloric acid and sulfuric acid; salts of organic acids such as acetic acid and lactic acid; salts of alkali metals such as lithium, sodium and potassium; salts of alkaline earth metals such as calcium and magnesium; salts of transition metals such as iron and copper; salts of organic bases such as ammonia, triethylamine, tributylamine, pyridine and hydrazine, and the like.

The compound (II) or the salt of the compound (II) is not particularly limited by the production method thereof. Further, the salt of the compound (II) can be obtained from the compound (II) by a known method. For example, the compound (II) or the salt of the compound (II) of the present invention can be obtained by a known production method described in Examples and the like.

[Compound represented by formula (I)]

The quinoline compound of the present invention is preferably a compound represented by the formula (I).

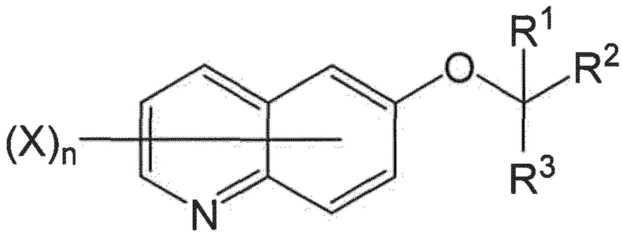

(I)

In the formula (I), $R^1$, $R^2$, $R^3$, X and n are the same as those defined in the formula (II).

[Agricultural and horticultural fungicide]

The agricultural and horticultural fungicide of the present invention contains at least one selected from the compound (II) or a salt thereof as an active ingredient. The amount of the compound (II) or a salt thereof contained in the agricultural and horticultural fungicide of the present invention is not particularly limited as long as the fungicidal effects are exhibited.

The agricultural and horticultural fungicide of the present invention can be used for controlling plant diseases caused by a wide variety of filamentous fungi, such as fungi belonging to Oomycetes, Ascomycetes, Deuteromycetes, Basidiomycetes and Zygomycetes.

Examples of plant diseases (pathogens) to be controlled are shown below.

Sugar beet: cercospora leaf spot (Cercospora beticola), aphanomyces root rot (Aphanomyces cochlioides), root rot (Thanatephorus cucumeris), leaf blight (Thanatephorus cucumeris), rust (Uromyces betae), powdery mildew (Oidium sp.), ramularia leaf spot (Ramularia beticola), damping-off (Aphanomyces cochlioides, Pythium ultimum), and the like;
Peanut: brown leaf spot (Mycosphaerella arachidis), leaf mold (Ascochyta sp.), rust (Puccinia arachidis), damping-off (Pythium debaryanum), alternaria leaf spot (Alternaria alternata), southern blight (Sclerotium rolfsii), late leaf spot (Mycosphaerella berkeleyi), and the like;
Cucumber: powdery mildew (Sphaerotheca fuliginea), downy mildew (Pseudoperonospora cubensis), gummy stem blight (Mycosphaerella melonis), fusarium wilt (Fusarium oxysporum), sclerotinia rot (Sclerotinia sclerotiorum), gray mold (Botrytis cinerea), anthracnose (Colletotrichum orbiculare), scab (Cladosporium cucumerinum), corynespora leaf spot (Corynespora cassiicola), damping-off (Pythium debaryanum, Rhizoctonia solani Kuhn), phomopsis root rot (Phomopsis sp.), bacterial spot (Pseudomonas syringae pv. lechrymans), and the like;
Tomato: gray mold (Botrytis cinerea), leaf mold (Cladosporium fulvum), late blight (Phytophthora infestans), verticillium wilt (Verticillium albo-atrum, Verticillium dahliae), powdery mildew (Oidium neolycopersici), early blight (Alternaria solani), black leaf mold (Pseudocercospora fuligena), and the like;
Eggplant: gray mold (Botrytis cinerea), black rot (Corynespora melongenae), powdery mildew (Erysiphe cichoracearum), leaf mold (Mycovellosiella nattrassii), stem rot (Sclerotinia sclerotiorum), verticillium wilt (Verticillium dahliae), brown spot (Phomopsis vexans), and the like;
Strawberry: gray mold (Botrytis cinerea), powdery mildew (Sphaerotheca humuli), anthracnose (Colletotrichum acutatum, Colletotrichum fragariae), phytophthora rot (Phytophthora cactorum), soft rot (Rhizopus stolonifer), fusarium wilt (Fusarium oxysporum), verticillium wilt (Verticillium dahliae), and the like;
Onion: gray-mold neck rot (Botrytis allii), gray mold (Botrytis cinerea), leaf blight (Botrytis squamosa), downy mildew (Peronospora destructor), leaf blight (Phytophthora porri), leaf blight (Ciborinia allii), and the like;
Welsh onion: bacterial soft rot (Pectobacterium carotovorum), downy mildew (Peronospora destructor), leaf blight (Pleospora allii), white rot (Sclerotium cepivorum), rust (Puccinia allii), leaf blight (Botrytis squamosa), and the like;
Cabbage: clubroot (Plasmodiophora brassicae), bacterial soft rot (Erwinia carotovora), black rot (Xanthomonas campesrtis pv. campestris), bacterial leaf spot (Pseudomonas syringae pv. maculicola, P. s. pv. alisalensis), downy mildew (Peronospora parasitica), sclerotinia rot (Sclerotinia sclerotiorum), alternaria sooty spot (Alternaria brassicicola), gray mold (Botrytis cinerea), and the like;
Kidney bean: stem rot (Sclerotinia sclerotiorum), gray mold (Botrytis cinerea), anthracnose (Colletotrichum lindemuthianum), angular leaf spot (Phaeoisariopsis griseola), and the like;
Apple: powdery mildew (Podosphaera leucotricha), scab (Venturia inaequalis), blossom blight (Monilinia mali), fruit spot (Mycosphaerella pomi), valsa canker (Valsa mali), alternaria blotch (Alternaria mali), rust (Gymnosporangium yamadae), ring rot (Botryosphaeria berengeriana), bitter rot (Glomerella cingulata, Colletotrichum acutatum), blotch (Diplocarpon mali), flyspeck (Zygophiala jamaicensis), sooty blotch (Gloeodes pomigena), violet root rot (Helicobasidium mompa), gray mold (Botrytis cinerea), fire blight (Erwinia amylovora), and the like;
Japanese apricot: scab (Cladosporium carpophilum), gray mold (Botrytis cinerea), brown rot (Monilinia mumecola),

sooty blotch (Peltaster sp.), and the like;

Persimmon: powdery mildew (Phyllactinia kakicola), anthracnose (Gloeosporium kaki), angular leaf spot (Cercospora kaki), circular leaf spot (Mycosphaerella nawae), and the like;

Peach: brown rot (Monilinia fructicola), scab (Cladosporium carpophilum), phomopsis rot (Phomopsis sp.), bacterial shot hole (Xanthomonas campestris pv. pruni), leaf curl (Taphrina deformans), anthracnose (Colletotrichum gloeosporioides), and the like;

Almond: brown rot (Monilinia laxa), leaf spot (Stigmina carpophila), scab (Cladosporium carpophilum), leaf blister (Polystigma rubrum), alternaria leaf spot (Alternaria alternata), anthracnose (Colletotrichum gloeospoides), and the like;

Sweet cherry: brown rot (Monilinia fructicola), anthracnose (Colletotrichum acutatum), black spot (Alternaria sp.), young-fruit rot (Monilinia kusanoi), cylindrosporium leaf spot (Mycosphaerella cerasella), and the like;

Grape: gray mold (Botrytis cinerea), powdery mildew (Uncinula necator), ripe rot (Glomerella cingulata, Colletotrichum acutatum), downy mildew (Plasmopara viticola), anthracnose (Elsinoe ampelina), leaf blight (Pseudocercospora vitis), black rot (Guignardia bidwellii), white rot (Coniella castaneicola), rust (Phakopsora ampelopsidis), cottony bunch (unidentified pathogen), and the like;

Pear: scab (Venturia nashicola), rust (Gymnosporangium asiaticum), black spot (Alternaria kikuchiana), ring rot (Botryosphaeria berengeriana), powdery mildew (Phyllactinia mali), phomopsis canker (Phomopsis fukushii), brown spot (Stemphylium vesicarium), anthracnose (Glomerella cingulata), and the like;

Tea: gray blight (Pestalotiopsis longiseta, P. theae), anthracnose (Colletotrichum theae-sinensis), net blister blight (Exobasidium reticulatum), and the like;

Citrus: scab (Elsinoe fawcettii), blue mold (Penicillium italicum), green mold (Penicillium digitatum), gray mold (Botrytis cinerea), melanose (Diaporthe citri), canker (Xanthomonas campestris pv. Citri), powdery mildew (Oidium sp.), phytophthora rot (Phytophthora citrophthora), anthracnose (Colletotrichum fioriniae), and the like;

Wheat: powdery mildew (Blumeria graminis f.sp. tritici), fusarium head blight (Gibberella zeae), leaf rust (Puccinia recondita), yellow rust (Puccinia striiformis), browning root rot (Pythium iwayamai), snow mold (Monographella nivalis), eye spot (Pseudocercosporella herpotrichoides), speckled leaf blotch (Septoria tritici), glume blotch (Leptosphaeria nodorum), typhula snow blight (Typhula incarnata), sclerotinia snow blight (Myriosclerotinia borealis), take-all (Gaeumannomyces graminis), ergot (Claviceps purpurea), stinking smut (Tilletia caries), loose smut (Ustilago nuda), blast (Pyricularia grisea), and the like;

Barley: stripe (Pyrenophora graminea), net blotch (Pyrenophora teres), leaf blotch (Rhynchosporium secalis), loose smut (Ustilago tritici, U. nuda), and the like;

Rice: blast (Pyricularia oryzae), sheath blight (Rhizoctonia solani), bakanae disease (Gibberella fujikuroi), brown spot (Cochliobolus miyabeanus), seedling blight (Pythium graminicola), bacterial leaf blight (Xanthomonas oryzae), bacterial seedling blight (Burkholderia plantarii), bacterial brown stripe (Acidovorax avenae), bacterial grain rot (Burkholderia glumae), cercospora leaf spot (Cercospora oryzae), false smut (Ustilaginoidea virens), discolored rice grains (Alternaria alternata, Curvularia intermedia), kernel discoloration of rice (Alternaria padwickii), pink coloring of rice grains (Epicoccum purpurascens), and the like;

Tobacco: sclerotinia rot (Sclerotinia sclerotiorum), powdery mildew (Erysiphe cichoracearum), black shank (Phytophthora nicotianae), and the like;

Tulip: gray mold (Botrytis cinerea), and the like;

Sunflower: downy mildew (Plasmopara halstedii), sclerotinia rot (Sclerotinia sclerotiorum), gray mold (Botrytis cinerea), and the like;

Bentgrass: snow mold (Sclerotinia borealis), large patch (Rhizoctonia solani), brown patch (Rhizoctonia solani), dollar spot (Sclerotinia homoeocarpa), blast (Pyricularia sp.), pythium red blight (Pythium aphanidermatum), anthracnose (Colletotrichum graminicola), and the like;

Orchardgrass: powdery mildew (Erysiphe graminis), and the like;

Soybean: purple stain (Cercospora kikuchii), downy mildew (Peronospora manshurica), phytophthora rot (Phytophthora sojae), rust (Phakopsora pachyrhizi), sclerotinia rot (Sclerotinia sclerotiorum), anthracnose (Colletotrichum truncatum), gray mold (Botrytis cinerea), sphaceloma scab (Elsinoe glycines), pod and stem blight (Diaporthe phaseolorum var. sojae), and the like;

Potato: late blight (Phytophthora infestans), early blight (Alternaria solani), black scurf (Thanatephorus cucumeris), verticillium wilt (Verticillium albo-atrum, V. dahliae, V. nigrescens), blackleg (Pectobacterium atrosepticum), soft rot (Pectobacterium carotovorum), and the like;

Banana: Panama disease (Fusarium oxysporum), Sigatoka disease (Mycosphaerella fijiensis, M. musicola), and the like;

Mango: anthracnose (Colletotrichum aenigma);

Rapeseed: sclerotinia stem rot (Sclerotinia sclerotiorum), root rot (Phoma lingam), alternaria black spot (Alternaria brassicae), and the like;

Coffee: rust (Hemileia vastatrix), anthracnose (Colletotrichum coffeanum), brown eye spot (Cercospora coffeicola), and the like;
Sugarcane: brown rust (Puccinia melanocephala), and the like;
Maize: zonate leaf spot (Gloeocercospora sorghi), rust (Puccinia sorghi), southern rust (Puccinia polysora), smut (Ustilago maydis), southern leaf blight (Cochliobolus heterostrophus), northern leaf blight (Setosphaeria turcica), and the like;
Cotton: damping-off (Pythium sp.), rust (Phakopsora gossypii), areolate mildew (Mycosphaerella areola), anthracnose (Glomerella gossypii), and the like

Examples of plants to which the agricultural and horticultural fungicide of the present invention can be applied include grains, vegetables, root vegetables, potatoes, trees, pasture grasses and turf grasses. The agricultural and horticultural fungicide of the present invention is preferably used for grains; vegetables; root vegetables; potatoes; trees such as fruit trees, tea, coffee and cacao; pasture grasses; turf grasses; and plants such as cotton.

The agricultural and horticultural fungicide of the present invention can be applied to each portion of these plants, for example, leaves, stems, stalks, flowers, buds, fruits, seeds, sprouts, roots, tubers, tuberous roots, shoots, cuttings and the like. In addition, improved varieties and variant species, cultivars, as well as mutants, hybrids and genetically modified organisms (GMOs) of these plants can also be targeted.

The agricultural and horticultural fungicide of the present invention can be used for seed treatment, foliage application, soil application, water surface application and the like, which are performed to control various diseases occurring in agricultural and horticultural crops including flowers and ornamental plants, turf grasses and pasture grasses.

The agricultural and horticultural fungicide of the present invention may contain components other than the quinoline compound of the present invention. As other components, known carriers used for formulation and the like can be mentioned. In addition, as other components, conventionally known fungicides, insecticidal / acaricidal agents, nematicides, soil pesticides, plant regulators, synergists, fertilizers, soil conditioners, animal feeds and the like can be mentioned. By including such other components, synergistic effects may be obtained.

Specific examples of the fungicides which can be mixed with or used in combination with the agricultural and horticultural fungicide of the present invention are shown below.

(1) Nucleic acid biosynthesis inhibitors:

(a) RNA polymerase I inhibitors: benalaxyl, benalaxyl-M, furalaxyl, metalaxyl, metalaxyl-M, oxadixyl, clozylacon, ofurace;
(b) adenosine deaminase inhibitors: bupirimate, dimethirimol, ethirimol;
(c) DNA/RNA synthesis inhibitors: hymexazol, octhilinone;
(d) DNA topoisomerase II inhibitors: oxolinic acid.

(2) Mitotic inhibitors and cell division inhibitors:

(a) β-tubulin polymerization inhibitors: benomyl, carbendazim, chlorfenazole, fuberidazole, thiabendazole, thiophanate, thiophanate-methyl, diethofencarb, zoxamide, ethaboxam;
(b) cell division inhibitors: pencycuron;
(c) delocalization inhibitors of spectrin-like protein: fluopicolide.

(3) Respiratory inhibitors:

(a) complex I NADH oxidoreductase inhibitors: diflumetorim, tolfenpyrad;
(b) complex II succinate dehydrogenase inhibitors: benodanil, flutolanil, mepronil, isofetamid, fluopyram, fenfuram, furmecyclox, carboxin, oxycarboxin, thifluzamide, benzovindiflupyr, bixafen, fluxapyroxad, furametpyr, isopyrazam, penflufen, penthiopyrad, sedaxane, boscalid, pyraziflumid, pydiflumetofen, isoflucypram, inpyrfluxam;
(c) complex III ubiquinol oxidase Qo inhibitors: azoxystrobin, coumoxystrobin, coumethoxystrobin, enoxastrobin, flufenoxystrobin, picoxystrobin, pyraoxystrobin, pyraclostrobin, pyrametostrobin, triclopyricarb, kresoxim-methyl, trifloxystrobin, dimoxystrobin, fenaminstrobin, metominostrobin, orysastrobin, famoxadone, fluoxastrobin, fenamidone, pyribencarb, mandestrobin, metyltetraprole;
(d) complex III ubiquinol reductase Qi inhibitors: cyazofamid, amisulbrom, fenpicoxamid;
(e) oxidative phosphorylation uncouplers: binapacryl, meptyldinocap, dinocap, fluazinam, ferimzone;
(f) oxidative phosphorylation inhibitors (ATP synthase inhibitors): fentin acetate, fentin chloride, fentin hydroxide;
(g) ATP production inhibitor: silthiofam;

(h) complex III: Qx (unknown) inhibitors of cytochrome bc1 (ubiquinone reductase): ametoctradin.

(4) Amino acid and protein synthesis inhibitors:

(a) methionine biosynthesis inhibitors: andoprim, cyprodinil, mepanipyrim, pyrimethanil;
(b) protein synthesis inhibitors: blasticidin S, kasugamycin, kasugamycin hydrochloride, streptomycin, oxytetracycline.

(5) Signal transduction inhibitors:

(a) signal transduction inhibitors: quinoxyfen, proquinazid;
(b) MAP/histidine kinase inhibitors in osmotic signal transduction: fenpiclonil, fludioxonil, chlozolinate, iprodione, procymidone, vinclozolin.

(6) Lipid and cell membrane synthesis inhibitors:

(a) phospholipid biosynthesis and methyltransferase inhibitors: edifenphos, iprobenfos, pyrazophos, isoprothiolane;
(b) lipid peroxidants: biphenyl, chloroneb, dicloran, quintozene, tecnazene, tolclofos-methyl, etridiazole;
(c) agents acting on cell membranes: iodocarb, propamocarb, propamocarb hydrochloride, propamocarb-fosetylate, prothiocarb;
(d) microorganisms disturbing pathogenic cell membranes: Bacillus subtilis, Bacillus subtilis strain QST713, Bacillus subtilis strain FZB24, Bacillus subtilis strain MBI600, Bacillus subtilis strain D747, Bacillus amyloliquefaciens;
(e) agents disturbing cell membranes: extracts of Melaleuca alternifolia (tea tree).

(7) Sterol biosynthesis inhibitors of cell membranes:

(a) C14 demethylation inhibitors in sterol biosynthesis: triforine, pyrifenox, pyrisoxazole, fenarimol, flurprimidol, nuarimol, imazalil, imazalil sulfate, oxpoconazole fumarate, pefurazoate, prochloraz, triflumizole, viniconazole, azaconazole, bitertanol, bromuconazole, cyproconazole, diclobutrazol, difenoconazole, diniconazole, diniconazole-M, epoxiconazole, etaconazole, fenbuconazole, fluquinconazole, flusilazole, flutriafol, fluconazole, furconazole-cis, hexaconazole, imibenconazole, ipconazole, metconazole, myclobutanil, penconazole, propiconazole, fluquinconazole, simeconazole, tebuconazole, tetraconazole, triadimefon, triadimenol, triticonazole, prothioconazole, voriconazole, mefentrifluconazole;
(b) inhibitors of $\Delta 14$ reductase and $\Delta 8 \rightarrow \Delta 7$-isomerase in sterol biosynthesis: aldimorph, dodemorph, dodemorph acetate, fenpropimorph, tridemorph, fenpropidin, piperalin, spiroxamine;
(c) 3-keto reductase inhibitors in C4 demethylation in sterol biosynthesis system: fenhexamid, fenpyrazamine;
(d) squalene epoxidase inhibitors in sterol biosynthesis system: pyributicarb, naftifine, terbinafine.

(8) Cell wall synthesis inhibitors:

(a) trehalase inhibitor: validamycin;
(b) chitin synthase inhibitors: polyoxins, polyoxorim;
(c) cellulose synthase inhibitors: dimethomorph, flumorph, pyrimorph; benchthiavalicarb-isopropyl, iprovalicarb, valifenalate, mandipropamid.

(9) Melanin biosynthesis inhibitors:

(a) reductase inhibitors in melanin biosynthesis: fthalide, pyroquilon, tricyclazole;
(b) anhydrase inhibitors in melanin biosynthesis: carpropamid, diclocymet, fenoxanil;
(c) polyketide synthesis inhibitor in melanin biosynthesis: tolprocarb.

(10) Resistance inducers of host plants:

(a) agents acting on salicylic acid synthetic pathway: acibenzolar-S-methyl;
(b) other agents: probenazole, tiadinil, isotianil, dichlobentiazox, laminarin, Reynoutria sachalinensis extract.

(11) Agents with unknown actions: cymoxanil, fosetyl-aluminium, phosphoric acid (phosphates), tecloftalam, triazoxide, flusulfamide, diclomezine, methasulfocarb, cyflufenamid, metrafenone, pyriofenone, dodine, dodine free base, flutianil.
(12) Agents having multiple points of action: copper (copper salts), Bordeaux mixture, copper hydroxide, copper naphthalate, copper oxide, copper oxychloride, copper sulfate, sulfur, sulfur products, calcium polysulfide, ferbam, mancozeb, maneb, mancopper, metiram, polycarbamate, propineb, thiram, zineb, ziram, captan, captafol, folpet, chlorothalonil, dichlofluanid, tolylfluanid, guazatine, iminoctadine acetates (iminoctadine triacetate), iminoctadine albesilates (iminoctadine trialbesilate), anilazine, dithianon, quinomethionate, fluoroimide.
(13) Other agents: DBEDC, fluorfolpet, guazatine acetate, bis(8-quinolinolato) copper (II), propamidine, chloropicrin, cyprofuram, agrobacterium, bethoxazin, diphenylamine, methyl isothiocyanate (MITC), mildiomycin, capsaicin, cufraneb, cyprosulfamide, dazomet, debacarb, dichlorophen, flumetover, fosetyl-calcium, fosetyl-sodium, irumamycin, natamycin, nitrothal-isopropyl, oxamocarb, pyrrolnitrin, tebufloquin, tolnifanide, zarilamide, algophase, amicarthiazol, oxathiapiprolin, metiram zinc, benthiazole, trichlamide, uniconazole, oxyfenthiin, picarbutrazox, dichlobentiazox, quinofumelin, thiuram, ambam, Agrobacterium radiobacter, Coniothyrium minitans, Pseudomonas fluorescens, Pseudomonas rhodesiae, Talaromyces flavus, Trichoderma atroviride, Erwinia carotovora subsp. carotovora, Bacillus simplex, Variovorax paradoxus, Lactobacillus plantarum, florylpicoxamid, pyrapropoyne, fluindapyr, aminopyrifen, pyridachlometyl, ipflufenoquin.

Specific examples of insecticidal / acaricidal agents, nematicides, soil pesticides, anthelmintics and the like which can be mixed with or used in combination with the agricultural and horticultural fungicide of the present invention are shown below.

(1) Acetylcholinesterase inhibitors:

(a) carbamate-based inhibitors: alanycarb, aldicarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, oxamyl, pirimicarb, propoxur, thiodicarb, thiofanox, triazamate, trimethacarb, XMC, xylylcarb, fenothiocarb, MIPC, MPMC, MTMC, aldoxycarb, allyxycarb, aminocarb, bufencarb, cloethocarb, metam-sodium, promecarb;
(b) organophosphorus-based inhibitors: acephate, azamethiphos, azinphos-ethyl, azinphos-methyl, cadusafos, chlorethoxyfos, chlorfenvinphos, chlormephos, chlorpyrifos, chlorpyrifos-methyl, coumaphos, cyanophos, demeton-S-methyl, diazinon, dichlorvos/DDVP, dicrotophos, dimethoate, dimethylvinphos, disulfoton, EPN, ethion, ethoprophos, famphur, fenamiphos, fenitrothion, fenthion, fosthiazate, heptenophos, imicyafos, isofenphos, isocarbophos, isoxathion, malathion, mecarbam, methamidophos, methidathion, mevinphos, monocrotophos, naled, omethoate, oxydemeton-methyl, parathion, parathion-methyl, phenthoate, phorate, phosalone, phosmet, phosphamidon, phoxim, pirimiphos-methyl, profenofos, propetamphos, prothiofos, pyraclofos, pyridaphenthion, quinalphos, sulfotep, tebupirimphos, temephos, terbufos, tetrachlorvinphos, thiometon, triazophos, trichlorfon, vamidothion, bromophos-ethyl, BRP, carbophenothion, cyanofenphos, CYAP, demeton-S-methyl sulfone, dialifos, dichlofenthion, dioxabenzofos, etrimfos, fensulfothion, flupyrazofos, fonofos, formothion, fosmethilan, isazofos, iodofenphos, methacrifos, pirimiphos-ethyl, phosphocarb, propaphos, prothoate, sulprofos.

(2) GABA-gated chloride channel antagonists: acetoprole, chlordane, endosulfan, ethiprole, fipronil, pyrafluprole, pyriprole; camphechlor, heptachlor, dienochlor.
(3) Sodium channel modulators: acrinathrin, d-cis/trans allethrin, d-trans allethrin, bifenthrin, bioallethrin, bioallethrin S-cyclopentyl isomers, bioresmethrin, cycloprothrin, cyfluthrin, $\beta$-cyfluthrin, cyhalothrin, $\lambda$-cyhalothrin, $\gamma$-cyhalothrin, cypermethrin, $\alpha$-cypermethrin, $\beta$-cypermethrin, $\theta$-cypermethrin, $\zeta$-cypermethrin, cyphenothrin [(1R)-trans isomers], deltamethrin, empenthrin [(EZ)-(1R)-isomers], esfenvalerate, etofenprox, fenpropathrin, fenvalerate, flucythrinate, flumethrin, $\tau$-fluvalinate, halfenprox, imiprothrin, kadethrin, permethrin, phenothrin [(1R)-trans isomers], prallethrin, pyrethrum, resmethrin, silafluofen, tefluthrin, tetramethrin [(1R)-isomers], tralomethrin, transfluthrin, allethrin, pyrethrin, pyrethrin I, pyrethrin II, profluthrin, dimefluthrin, bioethanomethrin, biopermethrin, transpermethrin, fenfluthrin, fenpirithrin, flubrocythrinate, flufenprox, metofluthrin, protrifenbute, pyresmethrin, terallethrin.
(4) Nicotinic acetylcholine receptor agonists: acetamiprid, clothianidin, dinotefuran, imidacloprid, nitenpyram, nithiazine, thiacloprid, thiamethoxam, sulfoxaflor, nicotine, flupyradifurone, flupyrimine.
(5) Nicotinic acetylcholine receptor allosteric modulators: spinetoram, spinosad.
(6) Chloride channel activators: abamectin, emamectin-benzoate, lepimectin, milbemectin, ivermectin, selamectin, doramectin, eprinomectin, moxidectin, milbemycin, milbemycin oxime, nemadectin.
(7) Juvenile hormone analogues: hydroprene, kinoprene, methoprene, fenoxycarb, pyriproxyfen, diofenolan,

epofenonane, triprene.
(8) Other nonspecific inhibitors: methyl bromide, chloropicrin, sulfuryl fluoride, borax, tartar emetic.
(9) Homoptera selective antifeedants: flonicamid, pymetrozine, pyrifluquinazon.
(10) Mite growth inhibitors: clofentezine, diflovidazin, hexythiazox, etoxazole.
(11) Insect midgut inner membrane disrupting agents derived from microorganisms: Bacillus thuringiensis subsp. israelensi, Bacillus sphaericus, Bacillus thuringiensis subsp. aizawai, Bacillus thuringiensis subsp. kurstaki, Bacillus thuringiensis subsp. tenebrionis, Bt crop proteins: Cry1Ab, Cry1Ac, Cry1Fa, Cry1A.105, Cry2Ab, Vip3A, mCry3A, Cry3Ab, Cry3Bb, Cry34Abl / Cry35Ab1.
(12) Mitochondrial ATP biosynthetic enzyme inhibitors: diafenthiuron, azocyclotin, cyhexatin, fenbutatin oxide, propargite, tetradifon.
(13) Oxidative phosphorylation uncouplers: chlorfenapyr, sulfluramid, DNOC, binapacryl, dinobuton, dinocap.
(14) Nicotinic acetylcholine receptor channel blockers: bensultap, cartap hydrochloride, nereistoxin, thiosultap-sodium, thiocyclam.
(15) Chitin synthesis inhibitors: bistrifluron, chlorfluazuron, diflubenzuron, flucycloxuron, flufenoxuron, hexaflumuron, lufenuron, novaluron, noviflumuron, teflubenzuron, triflumuron, buprofezin, fluazuron.
(16) Diptera molting disrupting agents: cyromazine.
(17) Molting hormone receptor agonists: chromafenozide, halofenozide, methoxyfenozide, tebufenozide.
(18) Octopamine receptor agonists: amitraz, demiditraz, chlordimeform.
(19) Mitochondrial electron transport system complex III inhibitors: acequinocyl, fluacrypyrim, hydramethylnon.
(20) Mitochondrial electron transport system complex I inhibitors: fenazaquin, fenpyroximate, pyrimidifen, pyridaben, tebufenpyrad, tolfenpyrad, rotenone.
(21) Voltage-dependent sodium channel blockers: indoxacarb, metaflumizone.
(22) Acetyl CoA carboxylase inhibitors: spirodiclofen, spiromesifen, spirotetramat.
(23) Mitochondrial electron transport system complex IV inhibitors: aluminum phosphide, calcium phosphide, phosphine, zinc phosphide, cyanide.
(24) Mitochondrial electron transport system complex II inhibitors: cyenopyrafen, cyflumetofen, pyflubumide.
(25) Ryanodine receptor modulators: chlorantraniliprole, cyantraniliprole, flubendiamide, cyclaniliprole, tetraniliprole.
(26) Mixed function oxidase inhibitor compounds: piperonyl butoxide.
(27) Latrophilin receptor agonists: depsipeptide, cyclodepsipeptide, 24-membered cyclodepsipeptide, emodepside.
(28) Other agents (with unknown action mechanisms): azadirachtin, benzoximate, bifenazate, bromopropylate, quinomethionate, cryolite, dicofol, pyridalyl, benclothiaz, sulfur, amidoflumet, 1,3-dichloropropene, DCIP, phenisobromolate, benzomate, metaldehyde, chlorobenzilate, clothiazoben, dicyclanil, fenoxacrim, fentrifanil, flubenzimine, fluphenazine, gossyplure, japonilure, metoxadiazone, oil, potassium oleate, tetrasul, triarathene, afidopyropen, flometoquin, flufiprole, fluensulfone, meperfluthrin, tetramethylfluthrin, tralopyril, dimefluthrin, methylneodecanamide, fluralaner, afoxolaner, fluxametamide, 5-[5-(3,5-dichlorophenyl)-5-trifluoromethyl-4,5-dihydroisoxazol-3-yl]-2-(1H-1,2,4-triazol-1-yl) benzonitrile (CAS: 943137-49-3), broflanilide, other meta-diamides, Steinernema carpocapsae, Steinernema glaseri, Pasteuria penetrans, Paecilomyces tenuipes, Paecilomyces fumosoroseus, Beauveria bassiana, Beauveria brongniartii, Metarhizium anisopliae, Verticillium lecanii, acynonapyr.
(29) Anthelmintics:

(a) benzimidazole-based anthelmintics: fenbendazole, albendazole, triclabendazole, oxibendazole, mebendazole, oxfendazole, parbendazole, flubendazole, febantel, netobimin, thiophanate, thiabendazole, cambendazole;
(b) salicylanilide-based anthelmintics: closantel, oxyclozanide, rafoxanide, niclosamide;
(c) substituted phenol-based anthelmintics: nitroxinil, nitroscanate;
(d) pyrimidine-based anthelmintics: pyrantel, morantel;
(e) imidazothiazole-based anthelmintics: levamisole, tetramisole;
(f) tetrahydropyrimidine-based anthelmintics: praziquantel, epsiprantel; and
(g) other anthelmintics: cyclodiene, ryania, clorsulon, metronidazole, demiditraz, piperazine, diethylcarbamazine, dichlorophen, monepantel, tribendimidine, amidantel, thiacetarsamide, melarsomine, arsenamide.

Specific examples of plant regulators which can be mixed with or used in combination with the pest control agent of the present invention are shown below.

Abscisic acid, kinetin, benzylaminopurine, 1,3-diphenylurea, forchlorfenuron, thidiazuron, chlorfenuron, dihydrozeatin, gibberellin A, gibberellin A4, gibberellin A7, gibberellin A3, 1-methylcyclopropane, N-acetyl aminoethoxyvinyl glycine (aka: aviglycine), aminooxyacetate, silver nitrate, cobalt chloride, IAA, 4-CPA, cloprop, 2,4-D, MCPB, indole-3-butyrate, dichlorprop, phenothiol, 1-naphthylacetamide, ethychlozate, cloxyfonac, maleic acid hydrazide, 2,3,5-triiodobenzoic acid, salicylic acid, methyl salicylate, (-)-jasmonic acid, methyl jasmonate, (+)-strigol, (+)-deoxystrigol, (+)-orobanchol, (+)-sorgolactone, 4-oxo-4-(2-phenylethyl) aminobutyric acid, ethephon, chlormequat, mepiquat chloride, benzyl adenine, 5-aminolevulinic acid, daminozide.

[Examples]

[Pharmaceutical formulation]

The agricultural and horticultural fungicide, the pest control agent, and the insecticide or acaricide of the present invention are not particularly limited by the dosage form. Examples thereof include dosage forms such as wettable powders, emulsions, powders, granules, water soluble powders, suspensions, granular wettable powders, and tablets. The method for preparing a formulation is not particularly limited, and a known preparation method can be employed depending on the dosage form.

A few formulation examples are shown below. The following pharmaceutical formulations are merely examples and can be modified without departing from the gist of the present invention, and the present invention is in no way limited by the following formulation examples. The term "parts" indicates "parts by weight" unless otherwise specified.

(Formulation 1: wettable powder)

40 parts of the quinoline compound of the present invention, 53 parts of diatomaceous earth, 4 parts of a higher alcohol sulfuric acid ester and 3 parts of an alkyl naphthalene sulfonate are uniformly mixed and finely pulverized to obtain a wettable powder containing 40% of an active ingredient.

(Formulation 2: emulsion)

30 parts of the quinoline compound of the present invention, 33 parts of xylene, 30 parts of dimethylformamide and 7 parts of a polyoxyethylene alkyl allyl ether are mixed and dissolved to obtain an emulsion containing 30% of an active ingredient.

(Formulation 3: granule)

5 parts of the quinoline compound of the present invention, 40 parts of talc, 38 parts of clay, 10 parts of bentonite and 7 parts of sodium alkylsulfate are uniformly mixed and finely pulverized, and then granulated into a granular form having a diameter of 0.5 to 1.0 mm to obtain a granule containing 5% of an active ingredient.

(Formulation 4: granule)

5 parts of the quinoline compound of the present invention, 73 parts of clay, 20 parts of bentonite, 1 part of sodium dioctyl sulfosuccinate and 1 part of potassium phosphate are thoroughly ground and mixed, water is added and thoroughly kneaded, followed by granulation and drying to obtain a granule containing 5% of an active ingredient.

(Formulation 5: suspension)

10 parts of the quinoline compound of the present invention, 4 parts of a polyoxyethylene alkyl allyl ether, 2 parts of sodium polycarboxylate, 10 parts of glycerin, 0.2 parts of xanthan gum and 73.8 parts of water are mixed and subjected to wet grinding until the particle size becomes 3 microns or less to obtain a suspension containing 10% of an active ingredient.

(Formulation 6: granular wettable powder)

40 parts of the quinoline compound of the present invention, 36 parts of clay, 10 parts of potassium chloride, 1 part of sodium alkylbenzenesulfonate, 8 parts of sodium lignin sulfonate, and 5 parts of formaldehyde condensate of sodium alkylbenzenesulfonate are uniformly mixed and finely pulverized, and then an appropriate amount of water is added, followed by kneading to form a clay-like material. The clay-like material is granulated and then dried to obtain a granular wettable powder containing 40% of an active ingredient.

Next, the present invention will be described in more detail by showing compound examples. However, the present invention is in no way limited by the following compound examples.

[Example 1]

2-(((8-bromoquinolin-6-yl)oxy)(5-fluoropyridin-2-yl)methyl)-1,3,4-oxadiazole Synthesis of [2-(((8-bromoquinolin-6-yl)oxy)(5-fluoropyridin-2-yl)methyl)-1,3,4-oxadiazole] (Compound No. a-115)

(Step 1) Ethyl 2-(5-fluoropyridin-2-yl) acetate

Synthesis of [ethyl 2-(5-fluoropyridin-2-yl) acetate]

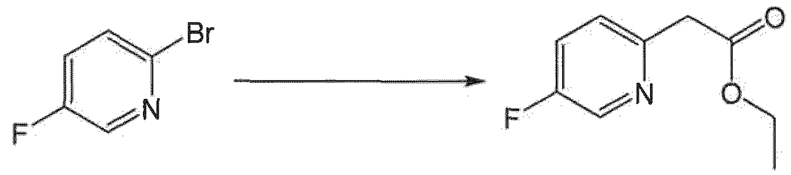

Copper iodide (10.8 g, 56.7 mmol), picolinic acid (14.0 g, 114 mmol), and cesium carbonate (185.2 g, 568 mmol) were suspended in 1,4-dioxane (850 mL). Under a nitrogen atmosphere, 2-bromo-5-fluoropyridine (50.2 g, 285 mmol) and diethyl malonate (91.2 g, 569 mmol) were added to the obtained suspension, and the resulting mixture was stirred at 100°C for 25 hours. Thereafter, a saturated aqueous ammonium chloride solution was added thereto, and the resulting mixture was extracted with ethyl acetate. The organic layer was dried over anhydrous magnesium sulfate, and then inorganic substances were removed by filtration. The resulting filtrate was concentrated under reduced pressure. Ethanol (850 mL) and concentrated hydrochloric acid (48 mL) were added to the obtained concentrate, and the resulting mixture was stirred at 80°C for 20 hours and was then concentrated under reduced pressure. The obtained concentrate was neutralized by adding a saturated aqueous sodium bicarbonate solution. Then, the resulting mixture was extracted with diethyl ether. The organic layer was dried over anhydrous magnesium sulfate, and then inorganic substances were removed by filtration. The resulting filtrate was concentrated under reduced pressure. The obtained concentrate was purified by silica gel column chromatography to obtain 31.8 g (yield: 61%) of a desired product.

$^1$H-NMR data of the obtained desired product are shown below.

$^1$H-NMR (400 MHz, $CDCl_3$): δ 1.26 (t, 3H), 3.83 (s, 2H), 4.19 (q, 2H), 7.29-7.39 (m, 2H), 8.42 (s, 1H).

(Step 2) 2-((5-fluoropyridin-2-yl)methyl)-1,3,4-oxadiazole

Synthesis of [2-((5-fluoropyridin-2-yl)methyl)-1,3,4-oxadiazole]

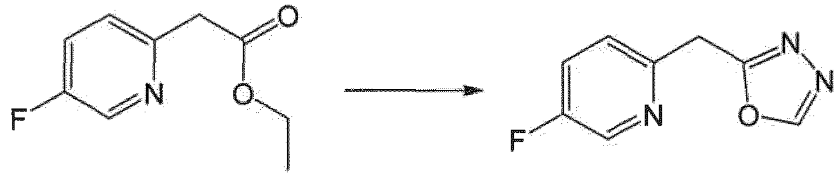

Ethyl 2-(5-fluoropyridin-2-yl) acetate (30.0 g, 164 mmol) was dissolved in tetrahydrofuran (540 mL). Hydrazine monohydrate (24.6 g, 491 mmol) and DBU (5.0 g, 32.8 mmol) were added to the obtained solution, and the resulting mixture was heated under reflux overnight. The solvent was distilled off under reduced pressure and the obtained residue was washed with diethyl ether, and then collected and dried by filtration. The resultant was dissolved in acetic acid (540 mL), methyl orthoformate (86.9 g, 819 mmol) was added to the obtained solution, and the resulting mixture was heated under reflux for 30 minutes and was concentrated under reduced pressure. The concentrate was neutralized by adding a saturated aqueous sodium bicarbonate solution. Then, the resulting mixture was extracted with ethyl acetate. The organic layer was dried over anhydrous magnesium sulfate, inorganic substances were removed by filtration, and the resultant was concentrated under reduced pressure. The obtained concentrate was purified by silica gel column chromatography to obtain 23.9 g (yield: 81%) of a desired product.

$^1$H-NMR data of the obtained desired product are shown below.

$^1$H-NMR (400 MHz, $CDCl_3$): δ 4.24 (s, 2H), 7.25-7.39 (m, 2H), 8.37 (s, 1H), 8.39 (s, 1H).

(Step 3) 2-(((8-bromoquinolin-6-yl)oxy)(5-fluoropyridin-2-yl)methyl)-1,3,4-oxadiazole

Synthesis of [2-(((8-bromoquinolin-6-yl)oxy)(5-fluoropyridin-2-yl)methyl)-1,3,4-oxadiazole]

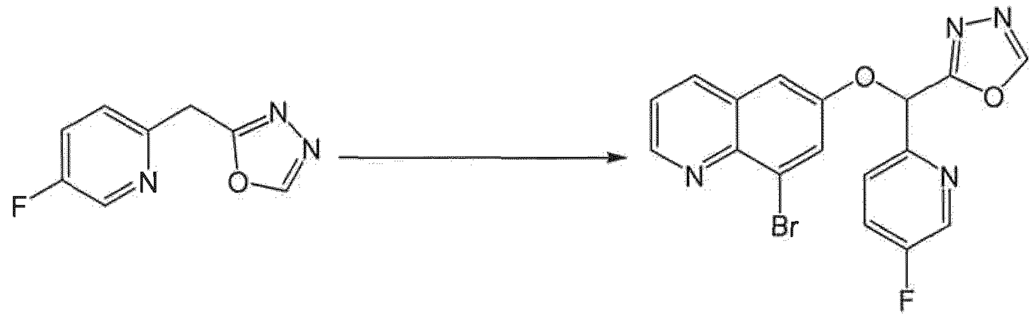

2-((5-Fluoropyridin-2-yl)methyl)-1,3,4-oxadiazole (1.1 g, 6.14 mmol) was dissolved in carbon tetrachloride (35 mL). N-bromosuccinimide (1.09 g, 6.14 mmol) and benzoyl peroxide (0.15 g, 0.6 mmol) were added to the obtained solution, and the resulting mixture was stirred with heating under reflux for 3.75 hours and was then allowed to cool. The obtained liquid was filtered through celite, and the filtrate was concentrated under reduced pressure. The obtained concentrate was dissolved in acetone (30 mL). 8-Bromo-6-quinolinol (1.14 g, 5.10 mmol) and potassium carbonate (2.07 g, 15 mmol) were added to the obtained solution, and the resulting mixture was stirred at room temperature for 2.5 hours. The obtained liquid was filtered through celite, and the filtrate was concentrated under reduced pressure. The obtained concentrate was purified by silica gel chromatography to obtain 0.83 g (yield: 40%) of a desired product.

$^1$H-NMR data of the obtained desired product are shown below.

$^1$H-NMR (400 MHz, $CDCl_3$): $\delta$ 6.83 (s, 1H), 7.28 (s, 1H), 7.40-7.58 (m, 2H), 7.80-7.90 (m, 1H), 7.91 (s, 1H), 8.04 (d, 1H), 8.43 (s, 1H), 8.47 (s, 1H), 8.92 (s, 1H).

[Example 2]

2-((5-fluoropyridin-2-yl)((8-(pyridin-3-yl)quinolin-6-yl)oxy)methyl)-1,3,4-oxadiazole

Synthesis of [2-((5-fluoropyridin-2-yl)((8-(pyridin-3-yl)quinolin-6-yl)oxy)methyl)-1,3,4-oxadiazole] (Compound No. a-134)

(Step 1) 2-((5-fluoropyridin-2-yl)((8-(pyridin-3-yl)quinolin-6-yl)oxy)methyl)-1,3,4-oxadiazole

Synthesis of [2-((5-fluoropyridin-2-yl)((8-(pyridin-3-yl)quinolin-6-yl)oxy)methyl)-1,3,4-oxadiazole]

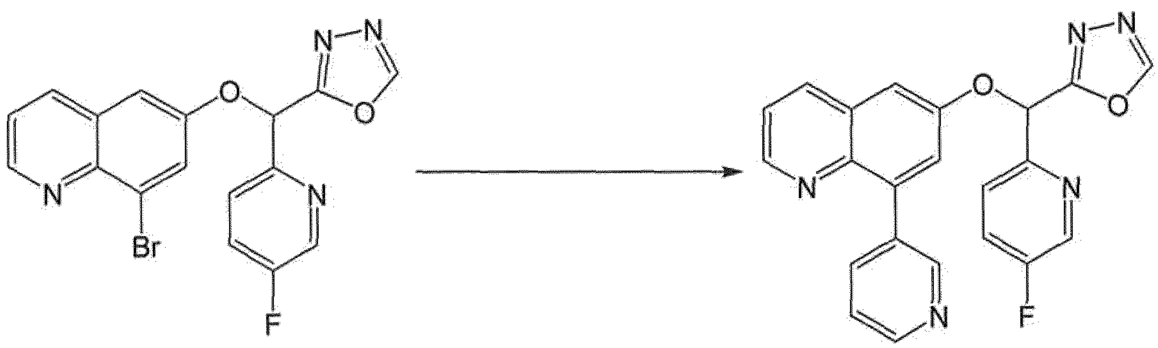

2-(((8-bromoquinolin-6-yl)oxy)(5-fluoropyridin-2-yl)methyl)-1,3,4-oxadiazole (0.15 g, 0.37 mmol) and 3-pyridylboronic acid (0.07 g, 0.56 mmol) were dissolved in a mixed solvent of toluene (5 mL) and water (1 mL). Under a nitrogen atmosphere, tetrakis(triphenylphosphine)palladium(0) (0.04 g, 0.035 mmol) and sodium carbonate (0.16 g, 1.50 mmol) were added to the obtained solution, and the resulting mixture was stirred at 80°C overnight and was then allowed to

cool. The obtained liquid was filtered through celite, and the filtrate was concentrated under reduced pressure. The obtained concentrate was purified by silica gel chromatography to obtain 0.1 g (yield: 65%) of a desired product.

$^1$H-NMR data of the obtained desired product are shown below.

$^1$H-NMR (400 MHz, $CDCl_3$): δ 6.89 (s, 1H), 7.32 (s, 1H), 7.39-7.57 (m, 4H), 7.86-7.90 (m, 1H), 8.03-8.11 (m, 2H), 8.43 (s, 1H), 8.48 (s, 1H), 8.64 (s, 1H), 8.81 (s, 1H), 8.89 (s, 1H).

[Example 3]

2-(((3-ethylquinolin-6-yl)oxy)(5-fluoropyridin-2-yl)methyl)-1,3,4-oxadiazole Synthesis of [2-(((3-ethylquinolin-6-yl)oxy)(5-fluoropyridin-2-yl)methyl)-1,3,4-oxadiazole] (Compound No. a-106)

(Step 1) Ethyl 2-((3-bromoquinolin-6-yl)oxy)-2-(5-fluoropyridin-2-yl) acetate

Synthesis of [ethyl 2-((3-bromoquinolin-6-yl)oxy)-2-(5-fluoropyridin-2-yl) acetate]

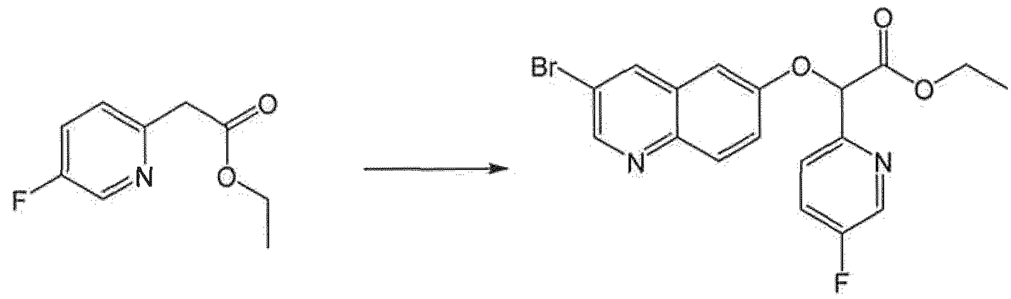

Ethyl 2-(5-fluoropyridin-2-yl) acetate (1.96 g, 10.76 mmol) was dissolved in carbon tetrachloride (36 mL). N-bromosuccinimide (1.90 g, 10.76 mmol) and benzoyl peroxide (0.17 g, 0.53 mmol) were added to the obtained solution, and the resulting mixture was stirred with heating under reflux for 4.5 hours and was then allowed to cool. The obtained liquid was filtered through celite, and the filtrate was concentrated under reduced pressure. The obtained concentrate was dissolved in N,N-dimethylformamide (27 mL). 3-bromo-6-quinolinol (1.84 g, 8.23 mmol) and potassium carbonate (2.27 g, 16.4 mmol) were added to the obtained solution, and the resulting mixture was stirred at room temperature for 2 days. The obtained liquid was filtered through celite, and the filtrate was concentrated under reduced pressure. The obtained concentrate was purified by silica gel chromatography to obtain 2.35 g (yield: 71%) of a desired product.

$^1$H-NMR data of the obtained desired product are shown below.

$^1$H-NMR (400 MHz, $CDCl_3$): δ 1.28 (t, 3H), 4.21-4.35 (m, 2H), 5.94 (s, 1H), 7.03 (d, 1H), 7.47-7.54 (m, 2H), 7.71 (dd, 1H), 8.01 (d, 1H), 8.17 (d, 1H), 8.51 (d, 1H), 8.74 (dd, 1H).

(Step 2) 2-(((3-bromoquinolin-6-yl)oxy)(5-fluoropyridin-2-yl)methyl)-1,3,4-oxadiazole

Synthesis of [2-(((3-bromoquinolin-6-yl)oxy)(5-fluoropyridin-2-yl)methyl)-1,3,4-oxadiazole]

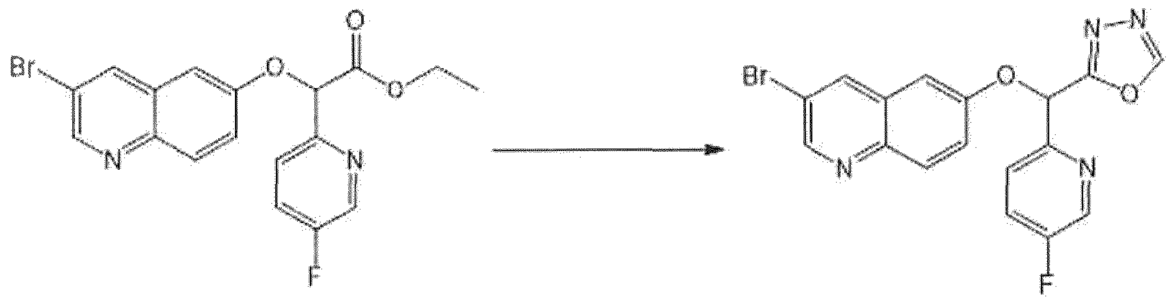

Ethyl 2-((3-bromoquinolin-6-yl)oxy)-2-(5-fluoropyridin-2-yl) acetate (2.35 g, 5.80 mmol) was dissolved in tetrahydrofuran (20 mL) and ethanol (20 mL). Hydrazine monohydrate (1.45 g, 29.6 mmol) was added to the obtained solution, and the resulting mixture was stirred at room temperature overnight. The obtained liquid was concentrated, and ethyl acetate was added to the obtained concentrate. The resulting mixture was washed with water, the organic layer was dried over anhydrous magnesium sulfate, inorganic substances were removed by filtration, and the resultant was concentrated under reduced pressure. The obtained concentrate was suspended in acetic acid (8 mL). Methyl orthoformate (0.75 g, 7.07 mmol) was added to the obtained suspension, and the resulting mixture was heated under reflux for 15 minutes. The obtained liquid was neutralized by adding a saturated aqueous sodium bicarbonate solution. Then, the resulting mixture was extracted with ethyl acetate. The organic layer was dried over anhydrous magnesium sulfate, inorganic substances were removed by filtration, and the resultant was concentrated under reduced pressure. The obtained concentrate was purified by silica gel chromatography to obtain 0.79 g (yield: 84%) of a desired product.

$^1$H-NMR data of the obtained desired product are shown below.

$^1$H-NMR (400 MHz, $CDCl_3$): δ 6.84 (s, 1H), 7.20 (d, 1H), 7.50-7.57 (m, 2H), 7.85 (dd, 1H), 8.02 (d, 1H), 8.20 (d, 1H), 8.44 (s, 1H), 8.49 (d, 1H), 8.79 (d, 1H).

(Step 3) 2-(((3-ethylquinolin-6-yl)oxy)(5-fluoropyridin-2-yl)methyl)-1,3,4-oxadiazole

Synthesis of [2-(((3-ethylquinolin-6-yl)oxy)(5-fluoropyridin-2-yl)methyl)-1,3,4-oxadiazole]

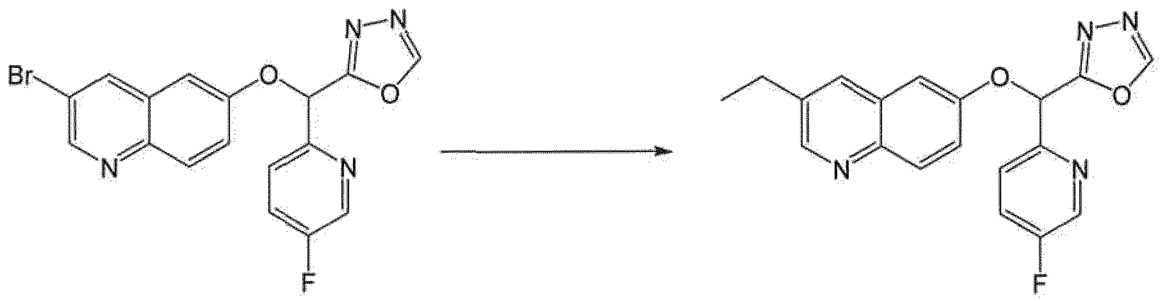

2-(((3-bromoquinolin-6-yl)oxy)(5-fluoropyridin-2-yl)methyl)-1,3,4-oxadiazole (0.13 g, 0.32 mmol) was dissolved in 1,4-dioxane (3 mL). [1,1'-bis(diphenylphosphino)ferrocene]palladium(II) dichloride dichloromethane adduct (0.013 g, 0.016 mmol) and a 1.0 M diethyl zinc tetrahydrofuran solution (0.65 mL) were added thereto, and the resulting mixture was stirred at 80°C for 30 minutes. An aqueous ammonium chloride solution was added to the obtained liquid, and then the resulting mixture was extracted with ethyl acetate. The organic layer was dried over anhydrous magnesium sulfate, inorganic substances were removed by filtration, and the resultant was concentrated under reduced pressure. The obtained concentrate was purified by silica gel chromatography to obtain 0.10 g (yield: 94%) of a desired product.

$^1$H-NMR data of the obtained desired product are shown below.

$^1$H-NMR (400 MHz, $CDCl_3$): δ1.33 (t, 3H), 2.80 (q, 2H), 6.86 (s, 1H), 7.25 (d, 1H), 7.45 (dd, 1H), 7.53 (dt, 1H), 7.80 (d, 1H), 7.87 (dd, 1H), 8.01 (d, 1H), 8.47 (s, 1H), 8.48 (d, 1H), 8.68 (d, 1H).

[Example 4]

2-(phenyl(quinolin-6-yloxy)methyl) oxazole

Synthesis of [2-(phenyl(quinolin-6-yloxy)methyl) oxazole] (Compound No. b-11)

(Step 1) Methyl 2-phenyl-2-(quinolin-6-yloxy) acetate

Synthesis of [methyl 2-phenyl-2-(quinolin-6-yloxy) acetate]

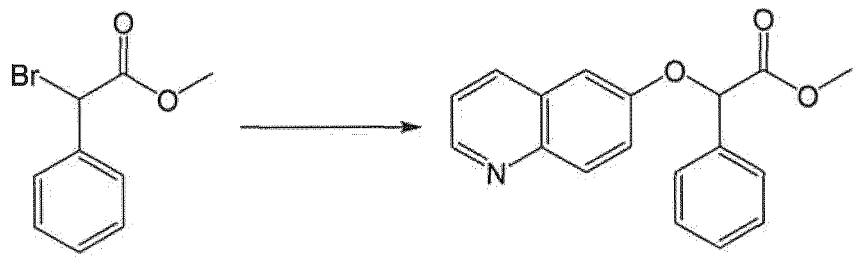

55% sodium hydride (1.80 g, 41.2 mmol) was suspended in N,N-dimethylformamide. 6-quinolinol (5.0 g, 34.4 mmol) was added to the obtained suspension under ice cooling, and the resulting mixture was stirred for 1 hour. Thereafter, methyl α-bromophenylacetate (8.68 g, 37.9 mmol) and potassium carbonate (5.58 g, 40.3 mmol) were added, and the resulting mixture was stirred at 50°C for 6 hours. Water was added to the obtained liquid, and the resulting mixture was extracted with ethyl acetate. The organic layer was dried over anhydrous magnesium sulfate, inorganic substances were removed by filtration, and the resultant was concentrated under reduced pressure. The obtained concentrate was purified by silica gel chromatography to obtain a desired product (yield: 75%).

$^1$H-NMR data of the obtained desired product are shown below.

$^1$H-NMR (400 MHz, $CDCl_3$): δ3.79 (s, 3H), 5.79 (s, 1H), 7.05 (s, 1H), 7.22-7.53 (m, 5H), 7.62 (d, 2H), 8.00 (d, 1H), 8.03 (d, 1H), 8.77 (s, 1H).

(Step 2) 2-phenyl-2-(quinolin-6-yloxy) acetic acid

Synthesis of [2-phenyl-2-(quinolin-6-yloxy) acetic acid]

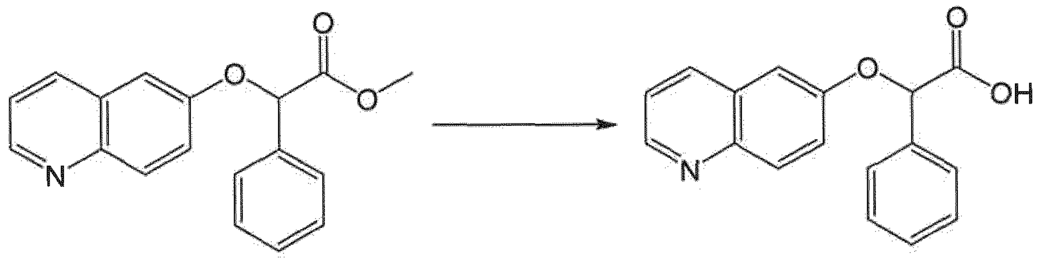

Methyl 2-phenyl-2-(quinolin-6-yloxy) acetate (6.7 g, 22.84 mmol) was dissolved in a mixed solvent of tetrahydrofuran (160 mL) and water (40 mL). Sodium hydroxide (1.83 g, 45.7 mmol) was added to the obtained solution, and the resulting mixture was stirred at 40°C for 3 hours. Dilute hydrochloric acid was added to the obtained liquid, and the resulting mixture was extracted with chloroform. The organic layer was dried over anhydrous magnesium sulfate, inorganic substances were removed by filtration, and the resultant was concentrated under reduced pressure to obtain 6.37 g (yield: >99%) of a desired product.

$^1$H-NMR data of the obtained desired product are shown below.

$^1$H-NMR (400 MHz, DMSO-$d_6$): δ5.96 (s, 1H), 7.35-7.52 (m, 6H), 7.62 (t, 2H), 7.94 (d, 1H), 8.21 (d, 1H), 8.74 (dd, 1H).

(Step 3) 2-(phenyl(quinolin-6-yloxy)methyl) oxazole

Synthesis of [2-(phenyl(quinolin-6-yloxy)methyl) oxazole]

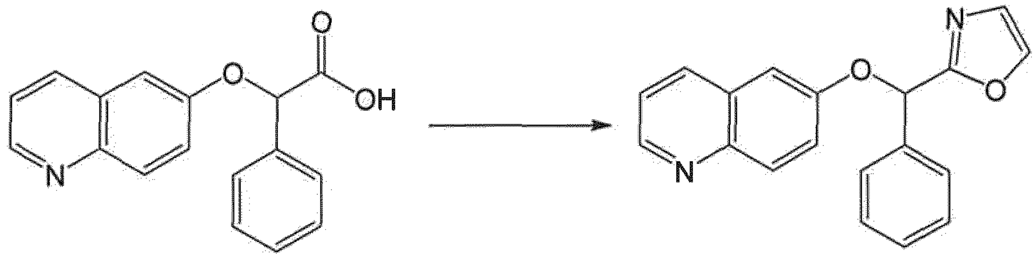

2-Phenyl-2-(quinolin-6-yloxy) acetic acid (0.60 g, 2.14 mmol) was dissolved in methylene chloride. 2,2-dimethoxymethylamine (0.25 g, 2.38 mmol), 1-ethyl-3-(3-dimethylaminopropyl) carbodiimide hydrochloride (0.58 g, 3.03 mmol), 1-hydroxybenzotriazole (0.32 g, 0.36 mmol) and triethylamine (0.44 g, 4.35 mmol) were added to the obtained solution, and the resulting mixture was stirred at room temperature overnight. The obtained liquid was diluted with water and extracted with chloroform. The organic layer was dried over anhydrous magnesium sulfate, and then inorganic substances were removed by filtration, and the resultant was concentrated under reduced pressure. The obtained concentrate was dissolved in tetrahydrofuran (20 mL), 5N hydrochloric acid (10 mL) was added thereto, and the resulting mixture was stirred at room temperature for 2 hours. The obtained liquid was extracted with ethyl acetate, the organic layer was dried over anhydrous magnesium sulfate, inorganic substances were removed by filtration, and the resultant was concentrated under reduced pressure. The obtained concentrate was added to an acetonitrile solution containing triphenylphosphine (1.13 g, 4.31 mmol) and ethane hexachloride (1.02 g, 4.31 mmol), and the resulting mixture was stirred at room temperature for 15 minutes. Pyridine (0.68 g, 8.60 mmol) was added dropwise thereto, and the resulting mixture was stirred at room temperature overnight. The obtained liquid was diluted with water and extracted with ethyl acetate. The organic layer was dried over anhydrous magnesium sulfate, inorganic substances were removed by filtration, and the resultant was concentrated under reduced pressure. The obtained concentrate was purified by silica gel chromatography to obtain 0.06 g (yield: 9%) of a desired product.

$^1$H-NMR data of the obtained desired product are shown below.

$^1$H-NMR (400 MHz, $CDCl_3$): δ6.53 (s, 1H), 7.12 (s, 1H), 7.21 (s, 1H), 7.30-7.53 (m, 5H), 7.62 (s, 1H), 7.63 (d, 2H), 8.00 (dd, 2H), 8.76 (s, 1H).

[Example 5]

5-(phenyl(quinolin-6-yloxy)methyl) oxazole

Synthesis of [5-(phenyl(quinolin-6-yloxy)methyl) oxazole] (Compound No. b-6)

(Step 1) 5-(phenyl(quinolin-6-yloxy)methyl) oxazole

Synthesis of [5-(phenyl(quinolin-6-yloxy)methyl) oxazole]

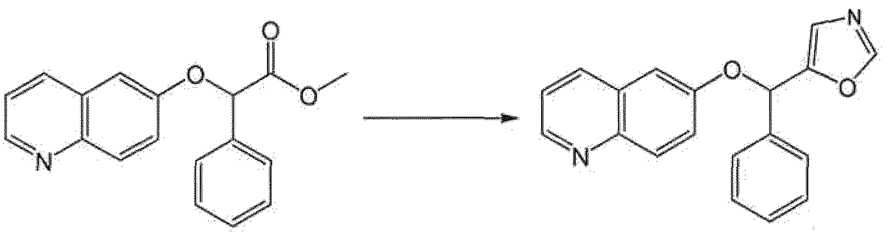

Methyl 2-phenyl-2-(quinolin-6-yloxy) acetate (0.50 g, 1.70 mmol) was dissolved in toluene. A 1.5 M toluene solution of diisobutylaluminium hydride (2.5 mL, 3.75 mmol) was added dropwise to the obtained solution at -78°C, and after completion of the dropwise addition, the resulting mixture was stirred for 1 hour. Methanol was added to the obtained liquid, and then an aqueous solution of sodium tartrate was added thereto, and the resulting mixture was stirred at room temperature for 30 minutes. Then, the resultant was extracted with ethyl acetate. The organic layer was dried over anhydrous magnesium sulfate, inorganic substances were removed by filtration, and the resultant was concentrated under reduced pressure. The obtained concentrate was dissolved in methanol. p-toluenesulfonylmethyl isocyanide (0.36

g, 1.87 mmol) and potassium carbonate (0.31 g, 2.24 mmol) were added to the obtained solution, and the resulting mixture was heated under reflux overnight. Water was added to the obtained liquid, and the resulting mixture was extracted with ethyl acetate. The organic layer was dried over anhydrous magnesium sulfate, inorganic substances were removed by filtration, and the resultant was concentrated under reduced pressure. The obtained concentrate was purified by silica gel chromatography to obtain 0.13 g (yield: 25%) of a desired product.

$^1$H-NMR data of the obtained desired product are shown below.

$^1$H-NMR (400 MHz, $CDCl_3$): δ6.43 (s, 1H), 6.90 (s, 1H), 7.10 (s, 1H), 7.29-7.55 (m, 7H), 7.88 (s, 1H), 7.93 (d, 1H), 8.00 (d, 1H), 8.76 (s, 1H).

[Example 6]

2-((4,5-dichloropyridin-2-yl)((3-(methylthio)quinolin-6-yl)oxy)methyl)-1,3,4-oxadiazole

Synthesis of [2-((4,5-dichloropyridin-2-yl)((3-(methylthio)quinolin-6-yl)oxy)methyl)-1,3,4-oxadiazole] (Compound No. a-92)

(Step 1) 3-bromo-6-(methoxymethoxy) quinoline

Synthesis of [3-bromo-6-(methoxymethoxy) quinoline]

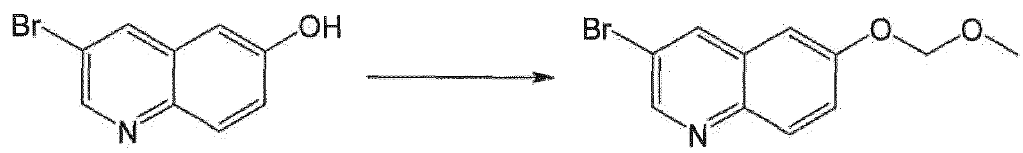

3-Bromo-6-quinolinol (15.2 g, 67.9 mmol) was dissolved in methylene chloride (200 mL). DIPEA (13.3 g, 103 mmol) and chloromethyl methyl ether (8.31 g, 103 mmol) were added to the obtained solution under ice cooling, and the resulting mixture was stirred overnight at room temperature. The obtained liquid was washed with water, and then washed with a saturated aqueous sodium bicarbonate solution. Thereafter, the resultant was dried over anhydrous magnesium sulfate, and inorganic substances were removed by filtration, followed by concentration under reduced pressure.

(Step 2) 6-(methoxymethoxy)-3-(methylthio) quinoline

Synthesis of [6-(methoxymethoxy)-3-(methylthio) quinoline]

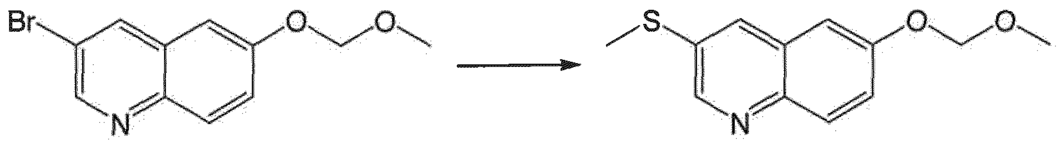

The concentrate (crude 3-bromo-6-(methoxymethoxy) quinoline) obtained in step 1 was dissolved in toluene (180 mL). Under a nitrogen atmosphere, sodium thiomethoxide (5.24 g, 74.8 mmol), xantphos (7.86 g, 13.6 mmol), and tris(dibenzylideneacetone) dipalladium(0) (6.22g, 6.79 mmol) were added to the obtained solution, and the resulting mixture was stirred at 100°C for 1.5 hours. Water was added to the obtained liquid, and the resulting mixture was extracted with ethyl acetate. The organic layer was dried over anhydrous magnesium sulfate, inorganic substances were removed by filtration, and the resultant was concentrated under reduced pressure. The obtained concentrate was purified by silica gel chromatography to obtain 13.9 g (yield: 87%, 2 steps) of a desired product.

$^1$H-NMR data of the obtained desired product are shown below.

$^1$H-NMR (400 MHz, $CDCl_3$): δ2.58 (s, 3H), 3.53 (s, 3H), 5.30 (s, 2H), 7.27 (d, 1H), 7.34 (dd, 1H), 7.79 (d, 1H), 7.95 (d, 1H), 8.66 (d, 1H).

(Step 3) 3-(methylthio)-6-quinolinol

Synthesis of [3-(methylthio)quinolin-6-ol]

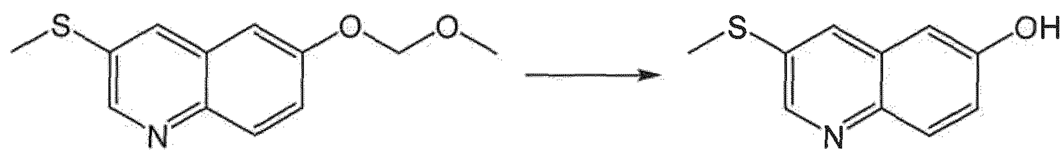

6-(Methoxymethoxy)-3-(methylthio) quinoline (13.9 g, 59.1 mmol) was dissolved in tetrahydrofuran (180 mL). 4N hydrochloric acid (180 mL) was added to the obtained solution, and the resulting mixture was stirred at room temperature overnight. Ethyl acetate was added to the obtained liquid, and a saturated aqueous sodium bicarbonate solution was added to neutralize the resulting mixture. The resulting precipitate was collected by filtration and dried to obtain 9.28 g (yield: 82%) of a desired product.
$^1$H-NMR data of the obtained desired product are shown below.
$^1$H-NMR (400 MHz, $CD_3OD$): δ2.61 (s, 3H), 7.07 (d, 1H), 7.23 (dd, 1H), 7.80 (d, 1H), 7.91 (d, 1H), 8.47 (d, 1H).

(Step 4) Ethyl 2-(4,5-dichloropyridin-2-yl)-2-((3-(methylthio)quinolin-6-yl)oxy) acetate

Synthesis of [ethyl 2-(4,5-dichloropyridin-2-yl)-2-((3-(methylthio)quinolin-6-yl)oxy) acetate]

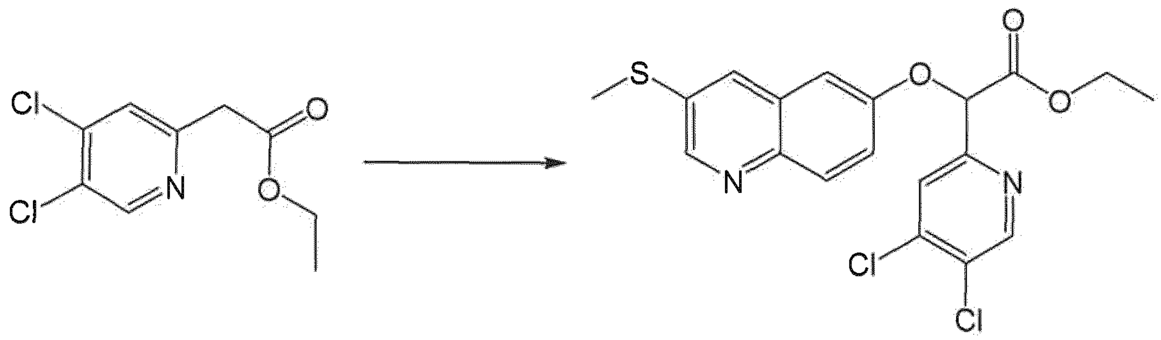

Ethyl 2-(4,5-dichloropyridin-2-yl) acetate (0.60 g, 2.56 mmol) was dissolved in carbon tetrachloride (9 mL). N-bromosuccinimide (0.45 g, 2.56 mmol) and benzoyl peroxide (0.04 g, 0.13 mmol) were added to the obtained solution, and the resulting mixture was stirred with heating under reflux for 2.5 hours and was then allowed to cool. The obtained liquid was filtered through celite, and the filtrate was concentrated under reduced pressure. The obtained concentrate was dissolved in acetone (6 mL). 3-(methylthio)-6-quinolinol (0.49 g, 2.56 mmol) and potassium carbonate (0.46 g, 3.33 mmol) were added to the obtained solution, and the resulting mixture was stirred at room temperature overnight. The obtained liquid was concentrated and extracted with ethyl acetate. The organic layer was dried over anhydrous magnesium sulfate, inorganic substances were removed by filtration, and the resultant was concentrated under reduced pressure. The obtained concentrate was purified by silica gel chromatography to obtain 0.25 g (yield: 23%) of a desired product.
$^1$H-NMR data of the obtained desired product are shown below.
$^1$H-NMR (400 MHz, $CDCl_3$): δ1.21-1.31 (m, 3H), 2.59 (s, 3H), 4.23-4.33 (m, 2H), 5.89 (s, 1H), 7.03 (d, 1H), 7.42 (dd, 1H), 7.73 (d, 1H), 7.82 (s, 1H), 7.98 (d, 1H), 8.65 (s, 1H), 8.67 (d, 1H).

(Step 5) 2-((4,5-dichloropyridin-2-yl)((3-(methylthio)quinolin-6-yl)oxy)methyl)-1,3,4-oxadiazole

Synthesis of [2-((4,5-dichloropyridin-2-yl)((3-(methylthio)quinolin-6-yl)oxy)methyl)-1,3,4-oxadiazole]

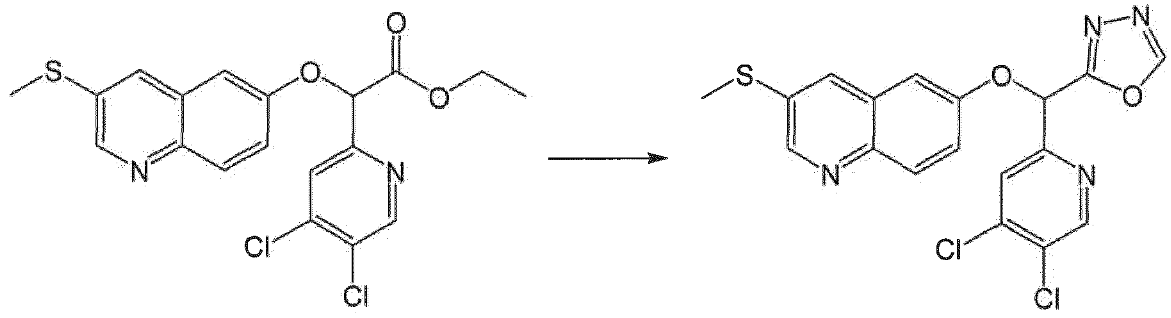

Ethyl 2-(4,5-dichloropyridin-2-yl)-2-((3-(methylthio)quinolin-6-yl)oxy) acetate (0.25 g, 0.59 mmol) was dissolved in tetrahydrofuran (3 mL). Hydrazine monohydrate (0.15 g, 3.0 mmol) and one drop of DBU were added to the obtained solution, and the resulting mixture was stirred at room temperature for 20 minutes. Chloroform was added to the obtained liquid, and the resulting mixture was washed with water. The organic layer was dried over anhydrous magnesium sulfate, inorganic substances were removed by filtration, and the resultant was concentrated under reduced pressure. The obtained concentrate was suspended in acetic acid (3 mL). Methyl orthoformate (0.31 g, 2.95 mmol) was added to the obtained suspension, and the resulting mixture was heated under reflux for 40 minutes and was then concentrated under reduced pressure. The concentrated liquid was neutralized by adding a saturated aqueous sodium bicarbonate solution, and the resulting mixture was extracted with chloroform. The organic layer was dried over anhydrous magnesium sulfate, inorganic substances were removed by filtration, and the resultant was concentrated under reduced pressure. The obtained concentrate was purified by silica gel chromatography to obtain 0.10 g (yield: 41%) of a desired product.

$^1$H-NMR data of the obtained desired product are shown below.

$^1$H-NMR (400 MHz, $CDCl_3$): δ2.59 (s, 3H), 6.81 (s, 1H), 7.21 (d, 1H), 7.42 (dd, 1H), 7.74 (d, 1H), 7.96 (s, 1H), 7.99 (d, 1H), 8.44 (s, 1H), 8.62 (s, 1H), 8.67 (d, 1H).

Some of the compounds of the present invention produced by the same method as in the above examples are shown in Tables 1 and 2. In the tables, properties, melting point (m.p.) or refractive index ($n_D$) are also shown as physical properties of each compound.

It should be noted that in the tables, Me represents a methyl group, Et represents an ethyl group, $^n$Pr represents a normal propyl group, $^i$Pr represents an isopropyl group, $^c$Pr represents a cyclopropyl group, $^c$Pen represents a cyclopentyl group, and $^t$Bu represents a tertiary butyl group, respectively.

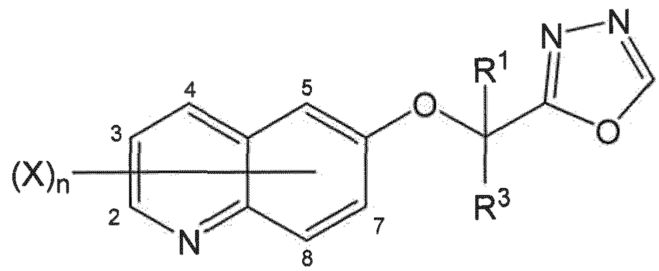

(I-1)

Table 1

| Compound No. | $R^1$ | $R^3$ | (X)n | Physical Property |
|---|---|---|---|---|
| a-1 | H | phenyl | - | m.p.: 110-112°C |
| a-2 | H | 4-F-phenyl | - | viscous oil |
| a-3 | H | 4-Cl-phenyl | - | m.p.: 112-114°C |
| a-4 | H | 4-Cl-phenyl | 3-F | m.p.: 153-160°C |
| a-5 | H | 4-Cl-phenyl | 3-Cl | m.p.: 175-179°C |
| a-6 | H | 4-F-phenyl | 3-Br | m.p.: 68-70°C |
| a-7 | H | 4-Cl-phenyl | 3-I | m.p.: 160-162°C |
| a-8 | H | 4-Cl-phenyl | 3-C≡CH | m.p.: 164-168°C |
| a-9 | H | 4-Cl-phenyl | 3-Et | m.p.: 132-136°C |
| a-10 | H | 4-Cl-phenyl | 3-Me | m.p.: 44-50°C |
| a-11 | H | 4-Cl-phenyl | 3-$^c$Pr | m.p.: 122-127°C |
| a-12 | H | 4-Cl-phenyl | 3-$^i$Pr | m.p.: 42-45°C |
| a-13 | H | 4-Cl-phenyl | 3-(C≡CSi(Me)$_3$) | m.p.: 61-65°C |
| a-14 | H | 4-Cl-phenyl | 3,5-$Cl_2$ | m.p.: 125-129°C |
| a-15 | H | 4-$CF_3$-phenyl | - | m.p.: 90-92°C |
| a-16 | H | 4-OMe-phenyl | - | m.p.: 129-131°C |
| a-17 | H | 4-SMe-phenyl | - | viscous oil |
| a-18 | H | 4-SOMe-phenyl | - | viscous oil |
| a-19 | H | 4-$SO_2$Me-phenyl | - | viscous oil |
| a-20 | H | 4-CN-phenyl | - | m.p.: 147-148°C |
| a-21 | H | 3-Cl-phenyl | - | m.p.: 105-107°C |
| a-22 | H | 3-F-phenyl | - | m.p.: 97-99°C |
| a-23 | H | 2-F-phenyl | - | viscous oil |
| a-24 | H | 3,4-$Cl_2$-phenyl | - | m.p.: 105-107°C |
| a-25 | H | thiophen-2-yl | - | viscous oil |
| a-26 | H | thiophen-3-yl | - | viscous oil |
| a-27 | H | 5-Cl-thiophen-3-yl | - | viscous oil |
| a-28 | H | 5-Br-thiophen-3-yl | - | viscous oil |
| a-29 | H | 2,5-$Cl_2$-thiophen-3-yl | - | viscous oil |
| a-30 | H | 2-Br-thiophen-3-yl | - | $n_D$(21.4°C) 1.6234 |

# Table 1 (continued)

| Compound No. | $R^1$ | $R^3$ | (X)n | Physical Property |
|---|---|---|---|---|
| a-31 | H | 4-Cl-1H-pyrazol-1-yl | - | m.p.: 108-112°C |
| a-32 | H | 4-Br-1H-pyrazol-1-yl | - | viscous oil |
| a-33 | H | 2-Cl-thiazol-4-yl | - | m.p.: 116-119°C |
| a-34 | H | thiazol-4-yl | - | $n_D$(20.6°C) 1.6104 |
| a-35 | H | pyridazin-3-yl | - | viscous oil |
| a-36 | H | 6-Cl-pyridazin-3-yl | - | m.p.: 161-165°C |
| a-37 | H | 6-Me-pyridazin-3-yl | - | m.p.: 123-126°C |
| a-38 | H | 6-SMe-pyridazin-3-yl | - | viscous oil |
| a-39 | H | 6-SOMe-pyridazin-3-yl | - | viscous oil |
| a-40 | H | 6-$CF_3$-pyridazin-3-yl | - | amorphous |
| a-41 | H | pyrazin-2-yl | - | m.p.: 156-158°C |
| a-42 | H | 5-Cl-pyrazin-2-yl | - | m.p.: 101-105°C |
| a-43 | H | 5-CN-pyrazin-2-yl | - | m.p.: 158-160°C |
| a-44 | H | pyrimidin-2-yl | - | amorphous |
| a-45 | H | 5-F-pyrimidin-2-yl | - | amorphous |
| a-46 | H | 5-Cl-pyrimidin-2-yl | - | amorphous |
| a-47 | H | pyridin-3-yl | - | viscous oil |
| a-48 | H | 6-Cl-pyridin-3-yl | - | viscous oil |
| a-49 | H | 6-CN-pyridin-3-yl | - | m.p.: 119-120°C |
| a-50 | H | pyridin-2-yl | - | amorphous |
| a-51 | H | 3,5-$F_2$-pyridin-2-yl | - | m.p.: 136-137°C |
| a-52 | H | 3-Br-5-F-pyridin-2-yl | - | m.p.: 134-137°C |
| a-53 | H | 3-Me-5-F-pyridin-2-yl | - | m.p.: 135-139°C |
| a-54 | H | 4-Cl-pyridin-2-yl | - | m.p.: 110-115°C |
| a-55 | H | 4-Br-pyridin-2-yl | - | viscous oil |
| a-56 | H | 4-SMe-pyridin-2-yl | - | viscous oil |
| a-57 | H | 4-Me-pyridin-2-yl | - | viscous oil |
| a-58 | H | 4-CN-pyridin-2-yl | - | m.p.: 132-135°C |
| a-59 | H | 5-Cl-pyridin-2-yl | - | m.p.: 129-131°C |
| a-60 | H | 5-Br-pyridin-2-yl | - | $n_D$(21.2°C) 1.6133 |

# Table 1 (continued)

| Compound No. | $R^1$ | $R^3$ | (X)n | Physical Property |
|---|---|---|---|---|
| a-61 | H | 5-Me-pyridin-2-yl | - | m.p.: 131-134°C |
| a-62 | H | 5-CN-pyridin-2-yl | - | m.p.: 152-154°C |
| a-63 | H | 5-OMe-pyridin-2-yl | - | amorphous |
| a-64 | H | 5-(C≡CH)-pyridin-2-yl | - | viscous oil |
| a-65 | H | 5-CN-pyridin-2-yl | 3-Br | m.p.: 166-167°C |
| a-66 | H | 5-$CO_2$Me-pyridin-2-yl | - | amorphous |
| a-67 | H | 5-(CH=$CH_2$)-pyridin-2-yl | - | viscous oil |
| a-68 | H | 5-$OCHF_2$-pyridin-2-yl | - | amorphous |
| a-69 | H | 5-SMe-pyridin-2-yl | - | m.p.: 45-50°C |
| a-70 | H | 5-SMe-pyridin-2-yl | 3-Br | viscous oil |
| a-71 | H | 5-SOMe-pyridin-2-yl | - | m.p.: 47-51°C |
| a-72 | H | 5-$SO_2$Me-pyridin-2-yl | - | m.p.: 50-55°C |
| a-73 | H | 5-CH=NOMe-pyridin-2-yl | - | m.p.: 129-131°C |
| a-74 | H | 5-Et-pyridin-2-yl | - | amorphous |
| a-75 | H | 5-$CF_3$-pyridin-2-yl | - | viscous oil |
| a-76 | H | 5-phenyl-pyridin-2-yl | - | m.p.: 57-62°C |
| a-77 | H | 5-(furan-2-yl)-pyridin-2-yl | - | m.p.: 150-154°C |
| a-78 | H | 5-(1,3-dioxolan-2-yl)pyridin-2-yl | - | m.p.: 119-122°C |
| a-79 | H | quinolin-2-yl | - | viscous oil |
| a-80 | H | isoquinolin-3-yl | - | viscous oil |
| a-81 | H | 5-(benzylthio)pyridin-2-yl | - | viscous oil |
| a-82 | H | 5-((4-OMe-benzyl)thio)pyridin-2-yl | - | m.p.: 113-115°C |
| a-83 | H | 5-$NO_2$-pyridin-2-yl | - | viscous oil |
| a-84 | H | 5-$SCF_3$-pyridin-2-yl | - | viscous oil |
| a-85 | H | 5-F-6-Me-pyridin-2-yl | - | amorphous |
| a-86 | H | 5-F-6-Cl-pyridin-2-yl | - | amorphous |
| a-87 | H | 4-Me-5-F-pyridin-2-yl | - | viscous oil |
| a-88 | H | 4-Me-5-F-pyridin-2-yl | 3-SMe | m.p.: 180-181°C |
| a-89 | H | 4-Me-5-Cl-pyridin-2-yl | - | $n_D$(22.2°C) 1.602 |
| a-90 | H | 4-Me-5-Cl-pyridin-2-yl | 3-SMe | m.p.: 179-181°C |

# Table 1 (continued)

| Compound No. | $R^1$ | $R^3$ | (X)n | Physical Property |
|---|---|---|---|---|
| a-91 | H | 4-Et-5-Cl-pyridin-2-yl | 3-SMe | m.p.: 122-124°C |
| a-92 | H | 4,5-$Cl_2$-pyridin-2-yl | 3-SMe | m.p.: 179-181°C |
| a-93 | H | 4-Cl-5-Br-pyridin-2-yl | 3-SMe | m.p.: 185-187°C |
| a-94 | H | 4-Cl-5-Me-pyridin-2-yl | 3-SMe | m.p.: 195-199°C |
| a-95 | H | 4-SMe-5-F-pyridin-2-yl | - | m.p.: 118-122°C |
| a-96 | H | 4-SMe-5-Br-pyridin-2-yl | - | amorphous |
| a-97 | H | 4-SMe-5-Cl-pyridin-2-yl | 3-SMe | m.p.: 68-78°C |
| a-98 | H | 4-SMe-5-Br-pyridin-2-yl | 3-SMe | m.p.: 128-130°C |
| a-99 | H | 4-OMe-5-Br-pyridin-2-yl | 3-SMe | m.p.: 138-142°C |
| a-100 | H | 4-phenyl-5-Cl-pyridin-2-yl | 3-SMe | viscous oil |
| a-101 | H | 4-Me-5-Br-pyridin-2-yl | - | m.p.: 132-136°C |
| a-102 | H | 4-Me-5-SMe-pyridin-2-yl | - | viscous oil |
| a-103 | H | 4,5-$(Me)_2$-pyridin-2-yl | - | viscous oil |
| a-104 | H | 4-Me-5-CN-pyridin-2-yl | - | m.p.: 171-174°C |
| a-105 | H | 5-F-pyridin-2-yl | 3-Me | viscous oil |
| a-106 | H | 5-F-pyridin-2-yl | 3-Et | viscous oil |
| a-107 | H | 5-F-pyridin-2-yl | 3-Cl | viscous oil |
| a-108 | H | 5-F-pyridin-2-yl | 3-Br | m.p.: 55-57°C |
| a-109 | H | 5-F-pyridin-2-yl | 3-I | m.p.: 54-56°C |
| a-110 | H | 5-F-pyridin-2-yl | 3-CN | m.p.: 92-96°C |
| a-111 | H | 5-F-pyridin-2-yl | 3,5-$Cl_2$ | m.p.: 170-174°C |
| a-112 | H | 5-F-pyridin-2-yl | 8-Me | m.p.: 128-133°C |
| a-113 | H | 5-F-pyridin-2-yl | 8-F | $n_D$(21.3°C) 1.5819 |
| a-114 | H | 5-F-pyridin-2-yl | 8-Cl | m.p.: 161-162°C |
| a-115 | H | 5-F-pyridin-2-yl | 8-Br | m.p.: 176-182°C |
| a-116 | H | 5-F-pyridin-2-yl | 8-Et | m.p.: 167-172°C |
| a-117 | H | 5-F-pyridin-2-yl | 8-$^n$Pr | m.p.: 118-121°C |
| a-118 | H | 5-F-pyridin-2-yl | 8-SMe | m.p.: 170-174°C |
| a-119 | H | 5-F-pyridin-2-yl | 8-$^c$Pr | m.p.: 191-193°C |
| a-120 | H | 5-F-pyridin-2-yl | 8-$CF_3$ | m.p.: 183-185°C |

# Table 1 (continued)

| Compound No. | $R^1$ | $R^3$ | (X)n | Physical Property |
|---|---|---|---|---|
| a-121 | H | 5-F-pyridin-2-yl | 3-SMe-8-$CF_3$ | m.p.: 158-162°C |
| a-122 | H | 5-F-pyridin-2-yl | 8-phenyl | viscous oil |
| a-123 | H | 5-F-pyridin-2-yl | 8-(2-F-phenyl) | amorphous |
| a-124 | H | 5-F-pyridin-2-yl | 8-(2-Cl-phenyl) | m.p.: 165-168°C |
| a-125 | H | 5-F-pyridin-2-yl | 8-(4-F-phenyl) | amorphous |
| a-126 | H | 5-F-pyridin-2-yl | 8-(2,4-$Cl_2$-phenyl) | amorphous |
| a-127 | H | 5-F-pyridin-2-yl | 8-(3-SMe-phenyl) | amorphous |
| a-128 | H | 5-F-pyridin-2-yl | 8-phenethyl | $n_D$(20.5°C) 1.5922 |
| a-129 | H | 5-F-pyridin-2-yl | 8-(2-Me-phenyl) | amorphous |
| a-130 | H | 5-F-pyridin-2-yl | 8-(2,6-$Cl_2$-phenyl) | amorphous |
| a-131 | H | 5-F-pyridin-2-yl | 8-(3-F-phenyl) | amorphous |
| a-132 | H | 5-F-pyridin-2-yl | 8-(3-$SO_2$Me-phenyl) | amorphous |
| a-133 | H | 5-F-pyridin-2-yl | 8-(3-SOMe-phenyl) | $n_D$(21.0°C) 1.6076 |
| a-134 | H | 5-F-pyridin-2-yl | 8-(pyridin-3-yl) | viscous oil |
| a-135 | H | 5-F-pyridin-2-yl | 8-(pyridin-4-yl) | viscous oil |
| a-136 | H | 5-F-pyridin-2-yl | 8-(pyridin-2-yl) | amorphous |
| a-137 | H | 5-F-pyridin-2-yl | 8-benzyl | viscous oil |
| a-138 | H | 5-F-pyridin-2-yl | 8-(1-Me-1H-pyrazol-5-yl) | viscous oil |
| a-139 | H | 5-F-pyridin-2-yl | 8-(1H-pyrazol-3-yl) | m.p.: 185-190°C |
| a-140 | H | 5-F-pyridin-2-yl | 8-CN | m.p.: 202-205°C |
| a-141 | H | 5-F-pyridin-2-yl | 8-(C≡CSi(Me)$_3$) | viscous oil |
| a-142 | H | 5-F-pyridin-2-yl | 3-Br-8-Me | m.p.: 207-209°C |
| a-143 | H | 5-F-pyridin-2-yl | 3-SMe-8-Me | m.p.: 123-126°C |
| a-144 | H | 5-F-pyridin-2-yl | 3-SOMe-8-Me | viscous oil |
| a-145 | H | 5-F-pyridin-2-yl | 3-$SO_2$Me-8-Me | m.p.: 177-181°C |
| a-146 | H | 5-F-pyridin-2-yl | 3-Br-7-F | m.p.: 145-148°C |
| a-147 | H | 5-F-pyridin-2-yl | 7-Me | m.p.: 115-119°C |
| a-148 | H | 5-F-pyridin-2-yl | 7-F | m.p.: 109-113°C |
| a-149 | H | 5-F-pyridin-2-yl | 7-SMe | m.p.: 142-145°C |
| a-150 | H | 5-F-pyridin-2-yl | 7-SOMe | amorphous |

## Table 1 (continued)

| Compound No. | $R^1$ | $R^3$ | (X)n | Physical Property |
|---|---|---|---|---|
| a-151 | H | 5-F-pyridin-2-yl | 7-$SO_2$Me | m.p.: 247-250°C |
| a-152 | H | 5-F-pyridin-2-yl | 3-OMe | m.p.: 43-45°C |
| a-153 | H | 5-F-pyridin-2-yl | 3-$^c$Pr | m.p.: 139-145°C |
| a-154 | H | 5-F-pyridin-2-yl | 3-$^i$Pr | viscous oil |
| a-155 | H | 5-F-pyridin-2-yl | 3-($CH_2CH_2Si(Me)_3$) | viscous oil |
| a-156 | H | 5-F-pyridin-2-yl | 3-SMe | amorphous |
| a-157 | H | 5-F-pyridin-2-yl | 3-SOMe | amorphous |
| a-158 | H | 5-F-pyridin-2-yl | 3-$SO_2$Me | m.p.: 187-190°C |
| a-159 | H | 5-F-pyridin-2-yl | 3-SEt | $n_D$(21.5°C) 1.6178 |
| a-160 | H | 5-F-pyridin-2-yl | 3-S$^i$Pr | m.p.: 135-139°C |
| a-161 | H | 5-F-pyridin-2-yl | 3-$CO_2$Et | $n_D$(22.1°C) 1.5753 |
| a-162 | H | 5-F-pyridin-2-yl | 3-$CF_2$H | amorphous |
| a-163 | H | 5-F-pyridin-2-yl | 3-$SCF_3$ | m.p.: 130-133°C |
| a-164 | H | 3-Cl-5-$CF_3$-pyridin-2-yl | - | m.p.: 160-164°C |
| a-165 | H | 5-F-pyridin-2-yl | - | m.p.: 126-128°C |
| a-166 | H | phenyl | 3-Br | m.p.: 152-154°C |
| a-167 | H | phenyl | 3-I | m.p.: 167-169°C |
| a-168 | H | phenyl | 3-CN | m.p.: 187-189°C |
| a-169 | H | phenyl | 3-Me | amorphous |
| a-170 | H | phenyl | 3-phenyl | amorphous |
| a-171 | H | phenyl | 2-Cl | m.p.: 137-140°C |
| a-172 | H | phenyl | 2-Br | m.p.: 136-138°C |
| a-173 | H | phenyl | 2-CN | m.p.: 120-124°C |
| a-174 | H | phenyl | 2-NH$^t$Bu | amorphous |
| a-175 | H | phenyl | 2-$NH_2$ | m.p.: 163-167°C |
| a-176 | H | phenyl | 2-$CF_3$ | m.p.: 141-143°C |
| a-177 | H | phenyl | 2-$CF_2CF_2CF_3$ | viscous oil |
| a-178 | H | phenyl | 4-Cl | m.p.: 149-150°C |
| a-179 | H | phenyl | 4-Me | m.p.: 115-120°C |
| a-180 | H | phenyl | 5-F | m.p.: 87-88°C |

## Table 1 (continued)

| Compound No. | $R^1$ | $R^3$ | (X)n | Physical Property |
|---|---|---|---|---|
| a-181 | H | phenyl | 5-Br | m.p.: 118-120°C |
| a-182 | H | phenyl | 7-F | m.p.: 111-112°C |
| a-183 | H | phenyl | 3-Br-7-F | m.p.: 176-178°C |
| a-184 | H | phenyl | 8-F | m.p.: 168-170°C |
| a-186 | H | 2-Cl-phenyl | - | m.p.: 83-85°C |
| a-188 | H | pyridin-4-yl | - | amorphous |
| a-189 | H | phenyl | 2-phenyl-3-$CF_3$ | m.p.: 160-162°C |
| a-190 | H | 1,3,4-oxadiazol-2-yl | - | m.p.: 140-141°C |
| a-191 | F | 4-F-phenyl | - | m.p.: 127-129°C |
| a-192 | H | phenyl | 3-$CF_3$ | m.p.: 140-141°C |
| a-193 | H | 5-Cl-pyridin-2-yl | 8-phenyl | m.p.: 64-68°C |
| a-194 | H | 5-Br-pyridin-2-yl | 8-phenyl | m.p.: 72-74°C |
| a-195 | H | 5-F-pyridin-2-yl | 7-Cl | m.p.: 163-166°C |
| a-196 | H | 5-F-pyridin-2-yl | 5-Cl | m.p.: 168-171°C |
| a-197 | H | pyridin-2-yl | 8-phenyl | m.p.: 95-97°C |
| a-198 | H | 5-CN-pyridin-2-yl | 8-phenyl | m.p.: 95-97°C |
| a-199 | H | 5-F-pyridin-2-yl | 7-Br | m.p.: 184-187°C |
| a-200 | H | 5-F-pyridin-2-yl | 7-phenyl | viscous oil |
| a-201 | H | 5-F-pyridin-2-yl | 8-COOMe | m.p.: 188-191°C |
| a-202 | H | 5-F-pyridin-2-yl | 8-(1,3,4-oxadiazol-2-yl) | m.p.: 187-192°C |
| a-203 | H | 5-F-pyridin-2-yl | 8-(2-CN-phenyl) | m.p.: 174-175°C |
| a-204 | H | 5-F-pyridin-2-yl | 8-(2,6-$F_2$-phenyl) | m.p.: 161-162°C |
| a-205 | H | 5-F-pyridin-2-yl | 8-(4-CN-phenyl) | m.p.: 81-83°C |
| a-206 | H | 5-F-pyridin-2-yl | 7-$^n$Pr | viscous oil |
| a-207 | H | 5-F-pyridin-2-yl | 7-Et | m.p.: 126-129°C |
| a-208 | H | 5-F-pyridin-2-yl | 3-phenyl | viscous oil |
| a-209 | H | 5-F-pyridin-2-yl | 3-(2-F-phenyl) | viscous oil |
| a-210 | H | 5-F-pyridin-2-yl | 3-(5-F-pyridin-3-yl) | m.p.: 170-174°C |

## Table 1 (continued)

| Compound No. | $R^1$ | $R^3$ | (X)n | Physical Property |
|---|---|---|---|---|
| a-211 | H | 5-F-pyridin-2-yl | 8-$CH_2$SMe | m.p.: 145-147°C |
| a-212 | H | 5-F-pyridin-2-yl | 8-(2-OMe-phenyl) | amorphous |
| a-213 | H | 5-F-pyridin-2-yl | 8-(2-$CF_3$-phenyl) | amorphous |
| a-214 | H | 5-F-pyridin-2-yl | 8-(4-OMe-phenyl) | amorphous |
| a-215 | H | 5-F-pyridin-2-yl | 8-(3-OMe-phenyl) | amorphous |
| a-216 | H | 5-F-pyridin-2-yl | 8-(thiophen-3-yl) | amorphous |
| a-217 | H | 5-F-pyridin-2-yl | 8-(1H-1,2,4-triazol-1-yl) | m.p.: 172-176°C |
| a-218 | H | 5-F-pyridin-2-yl | 8-(3-Cl-1H-1,2,4-triazol-1-yl) | m.p.: 186-189°C |
| a-219 | H | 5-F-pyridin-2-yl | 8-(1H-imidazol-1-yl) | viscous oil |
| a-220 | H | 5-F-pyridin-2-yl | 8-(1H-pyrazol-1-yl) | m.p.: 146-149°C |
| a-221 | H | 5-F-pyridin-2-yl | 3-Br-8-Cl | m.p.: 216-218°C |
| a-222 | H | 5-F-pyridin-2-yl | 8-(3,5-$Me_2$-isoxazol-4-yl) | amorphous |
| a-223 | H | 5-F-pyridin-2-yl | 3-SMe-8-Cl | m.p.: 167-170°C |
| a-224 | H | 5-F-pyridin-2-yl | 3-SMe-8-phenyl | viscous oil |
| a-225 | H | 5-F-pyridin-2-yl | 3-(1,3,4-oxadiazol-2-yl) | viscous oil |
| a-226 | H | 5-F-pyridin-2-yl | 8-(4-Cl-1H-pyrazol-1-yl) | m.p.: 167-170°C |
| a-227 | H | 5-F-pyridin-2-yl | 5-Cl-8-(4-Cl-1H-pyrazol-1-yl) | m.p.: 230-233°C |
| a-228 | H | 5-F-pyridin-2-yl | 3-$SO_2$Me-8-phenyl | viscous oil |
| a-229 | H | 5-F-pyridin-2-yl | 3-SOMe-8-phenyl | viscous oil |
| a-230 | H | 5-F-pyridin-2-yl | 3-$SO_2$Me-8-Cl | amorphous |
| a-231 | H | 5-F-pyridin-2-yl | 3-SMe-8-(pyridin-2-yl) | viscous oil |
| a-232 | H | 5-F-pyrimidin-2-yl | 8-phenyl | amorphous |
| a-233 | H | 5-F-pyridin-2-yl | 8-(1,2,4-oxadiazol-3-yl) | m.p.: 150-154°C |
| a-234 | H | 5-F-pyridin-2-yl | 3-(oxazol-5-yl) | m.p.: 144-148°C |
| a-235 | H | 5-F-pyridin-2-yl | 3-CH$(OEt)_2$ | viscous oil |
| a-236 | H | 5-F-pyridin-2-yl | 8-(1,3-dioxolan-2-yl) | m.p.: 186-187°C |
| a-237 | H | 5-F-pyridin-2-yl | 2-$^n$Bu | m.p.: 109-113°C |
| a-238 | H | 5-F-pyridin-2-yl | 3-(1,3-dioxolan-2-yl) | viscous oil |
| a-239 | H | 5-F-pyridin-2-yl | 3-formyl | viscous oil |
| a-240 | H | pyridin-2-yl | 8-(2-F-pyridin-4-yl) | m.p.: 140-142°C |

## Table 1 (continued)

| Compound No. | $R^1$ | $R^3$ | (X)n | Physical Property |
|---|---|---|---|---|
| a-241 | H | 5-F-pyridin-2-yl | 2-Cl | m.p.: 120-122°C |
| a-242 | H | 5-F-pyridin-2-yl | 2-Me | m.p.: 140-141°C |
| a-243 | H | 5-F-pyridin-2-yl | 8-$CH_2SO_2Me$ | m.p.: 217-219°C |
| a-244 | H | 5-F-pyridin-2-yl | 8-$CH_2SOMe$ | m.p.: 183-186°C |
| a-245 | H | 5-F-pyridin-2-yl | 8-$CH=CBr_2$ | m.p.: 154-156°C |
| a-246 | H | 5-F-pyridin-2-yl | 3-$^nPr$ | viscous oil |
| a-247 | H | 5-F-pyridin-2-yl | 8-Ac | m.p.: 172-174°C |
| a-248 | H | 5-F-pyridin-2-yl | 3-Br-8-phenyl | amorphous |
| a-249 | H | 5-F-pyridin-2-yl | 3,5-$Br_2$-8-phenyl | m.p.: 163-165°C |
| a-250 | H | 5-F-pyridin-2-yl | 8-$CH_2OMe$ | m.p.: 156-157°C |
| a-251 | H | 5-F-pyridin-2-yl | 8-propionyl | m.p.: 109-112°C |
| a-252 | H | 5-F-pyridin-2-yl | 8-$CH=CCl_2$ | m.p.: 155-158°C |
| a-253 | H | 5-F-pyridin-2-yl | 3-CN-8-phenyl | amorphous |
| a-254 | H | 5-F-pyridin-2-yl | 8-$CH=CF_2$ | m.p.: 159-161°C |
| a-255 | H | 5-F-pyridin-2-yl | 8-$CH_2CH_2CF_3$ | viscous oil |
| a-256 | H | 5-F-pyridin-2-yl | 3-(4-Me-phenyl) | viscous oil |
| a-257 | H | 5-F-pyridin-2-yl | 3-(4-CN-phenyl) | m.p.: 182-184°C |
| a-258 | H | 5-F-pyridin-2-yl | 3-(pyrimidin-5-yl) | m.p.: 218-220°C |
| a-259 | H | 5-F-pyridin-2-yl | 3-(2-Me-phenyl) | viscous oil |
| a-260 | H | 5-F-pyridin-2-yl | 3-(1-Me-1H-pyrazol-4-yl) | m.p.: 159-162°C |
| a-261 | H | 5-F-pyridin-2-yl | 3-(3-Cl-phenyl) | viscous oil |
| a-262 | H | 5-F-pyridin-2-yl | 3-(3-OMe-phenyl) | m.p.: 120-122°C |
| a-263 | H | 5-F-pyridin-2-yl | 3-(isoxazol-3-yl) | m.p.: 226-228°C |
| a-264 | H | 5-F-pyridin-2-yl | 3-(pyridin-4-yl) | viscous oil |
| a-265 | H | 5-F-pyridin-2-yl | 8-(thiophen-2-yl) | m.p.: 135-137°C |
| a-266 | H | 5-F-pyridin-2-yl | 2,3-$Me_2$ | m.p.: 178-180°C |
| a-267 | H | 5-F-pyridin-2-yl | 3-(3,5-$Me_2$-isoxazol-4-yl) | viscous oil |
| a-268 | H | 5-F-pyridin-2-yl | 3-(5-$CF_3$-isoxazol-3-yl) | m.p.: 140-143°C |
| a-269 | H | 5-F-pyridin-2-yl | 2-$NH_2$ | m.p.: 168-172°C |
| a-270 | H | 5-F-pyridin-2-yl | 2-OH | m.p.: 220°C up |

## Table 1 (continued)

| Compound No. | $R^1$ | $R^3$ | (X)n | Physical Property |
|---|---|---|---|---|
| a-271 | H | 5-F-pyridin-2-yl | 2-OMe | m.p.: 106-108°C |
| a-272 | H | 5-F-pyridin-2-yl | 3-(2,6-$Cl_2$-phenyl) | viscous oil |
| a-273 | H | 5-F-pyridin-2-yl | 3-(5-Me-isoxazol-3-yl) | m.p.: 144-147°C |
| a-274 | H | 5-F-pyridin-2-yl | 3-(5,5-$Me_2$-4,5-dihydroisoxazol-3-yl) | m.p.: 122-125°C |
| a-275 | H | 5-F-pyridin-2-yl | 3-(prop-1-yn-1-yl) | m.p.: 196-197°C |
| a-276 | H | 5-F-pyridin-2-yl | 3-($^c$Pr-ethynyl) | m.p.: 168-170°C |
| a-277 | H | 5-F-pyridin-2-yl | 3-(4,5-dihydroisoxazol-3-yl) | m.p.: 210-213°C |
| a-278 | H | 5-F-pyridin-2-yl | 8-vinyl | m.p.: 158-160°C |
| a-279 | H | 5-F-pyridin-2-yl | 3-$CH_2$OMe | m.p.: 129-130°C |
| a-280 | H | 5-F-pyridin-2-yl | 8-(2-F-pyridin-4-yl) | m.p.: 174-175°C |
| a-281 | H | 5-F-pyridin-2-yl | 3-CH=$CCl_2$ | m.p.: 142-144°C |
| a-282 | H | 5-F-pyridin-2-yl | 3-(prop-1-en-2-yl) | m.p.: 101-103°C |
| a-283 | H | 5-F-pyridin-2-yl | 3-CH=$CMe_2$ | m.p.: 122-127°C |
| a-284 | H | 5-F-pyridin-2-yl | 3-$CH_2O^iPr$ | viscous oil |
| a-285 | H | 5-F-pyridin-2-yl | 8-(pyrimidin-5-yl) | m.p.: 81-83°C |
| a-286 | H | 5-F-pyridin-2-yl | 3-$CH_2$SMe | viscous oil |
| a-287 | H | 5-F-pyridin-2-yl | 8-$NH_2$ | viscous oil |
| a-288 | H | 5-F-pyridin-2-yl | 8-$^c$Pen | m.p.: 140-141°C |
| a-289 | H | 4-(prop-1-yn-1-yl)pyridin-2-yl | - | m.p.: 143-147°C |
| a-290 | H | 5-F-pyridin-2-yl | 8-$^t$Bu | m.p.: 54-57°C |
| a-291 | H | 5-F-pyridin-2-yl | 8-I | m.p.: 208-210°C |
| a-292 | H | 5-F-pyridin-2-yl | 8-$^i$Pr | m.p.: 121-123°C |
| a-293 | H | 5-F-pyridin-2-yl | 8-(thiazol-5-yl) | m.p.: 139-140°C |
| a-294 | H | 4-(prop-1-yn-1-yl)pyridin-2-yl | 3-SMe | m.p.: 175-177°C |
| a-295 | H | 5-F-pyridin-2-yl | 8-$CHF_2$ | m.p.: 193-195°C |
| a-296 | H | 5-F-pyridin-2-yl | 8-$CH_2$F | m.p.: 165-166°C |
| a-297 | H | 2-Cl-thiazol-4-yl | 3-Br | m.p.: 143-145°C |
| a-298 | H | 2-Cl-thiazol-4-yl | 3-SMe | m.p.: 146-149°C |
| a-299 | H | 5-F-pyridin-2-yl | 4-Me | m.p.: 123-125°C |
| a-300 | H | 5-F-pyridin-2-yl | 3-Br-8-Br | m.p.: 228-231°C |

# Table 1 (continued)

| Compound No. | $R^1$ | $R^3$ | (X)n | Physical Property |
|---|---|---|---|---|
| a-300 | H | 5-F-pyridin-2-yl | 3-Br-8-Br | m.p.: 228-231°C |
| a-301 | H | 4-(prop-1-yn-1-yl)pyridin-2-yl | 8-Br | m.p.: 213-215°C |
| a-302 | H | 4-(prop-1-yn-1-yl)pyridin-2-yl | 8-F | viscous oil |
| a-303 | H | 5-Cl-4-(prop-1-yn-1-yl)pyridin-2-yl | 8-F | m.p.: 164-166°C |
| a-304 | H | 4-ethynylpyridin-2-yl | - | viscous oil |
| a-305 | H | 5-Cl-4-(prop-1-yn-1-yl)pyridin-2-yl | 8-Br | m.p.: 150-152°C |
| a-306 | H | 5-Cl-4-(3-methoxyprop-1-yn-1-yl)pyridin-2-yl | 8-F | m.p.: 136-138°C |
| a-307 | H | 4-(3-methoxyprop-1-yn-1-yl)pyridin-2-yl | 8-F | m.p.: 150-152°C |
| a-308 | H | 4-Br-pyridin-2-yl | 8-F | amorphous |
| a-309 | H | 5-F-pyridin-2-yl | 3-$CH(OMe)_2$ | viscous oil |
| a-310 | H | 4-$^n$Pr-pyridin-2-yl | 8-F | viscous oil |
| a-311 | H | 5-F-pyridin-2-yl | 2-vinyl | amorphous |
| a-312 | H | 5-F-pyridin-2-yl | 3-I-8-Br | m.p.: 165-167°C |
| a-313 | H | 4-OMe-pyridin-2-yl | 8-F | viscous oil |
| a-314 | H | 4-Me-pyridin-2-yl | 8-F | m.p.: 107-109°C |
| a-315 | H | 5-F-pyridin-2-yl | 2-($C\equiv CSi(Me)_3$) | amorphous |
| a-316 | H | 4-$CH_2OMe$-pyridin-2-yl | 8-F | $n_D$(22.1°C) 1.5644 |
| a-317 | H | 4-$CH_2OEt$-pyridin-2-yl | 8-F | $n_D$(17.1°C) 1.5748 |
| a-318 | H | 5-F-pyridin-2-yl | 2-$C\equiv CH$ | m.p.: 123-125°C |
| a-319 | H | 4-$CH_2SMe$-pyridin-2-yl | 8-F | viscous oil |
| a-320 | H | 5-F-pyridin-2-yl | 2-$CH_2OAc$ | m.p.: 133-135°C |
| a-321 | H | 4-($CH(OMe)_2$)-pyridin-2-yl | 8-F | viscous oil |
| a-322 | H | 5-F-pyridin-2-yl | 2-$CH_2OH$ | m.p.: 93-95°C |

Table 2

| Compound No. | Structure | Physical Property |
|---|---|---|
| a-185 | 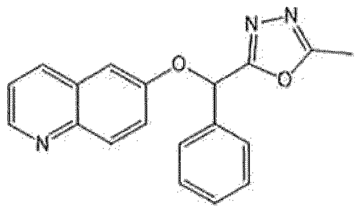 | m.p.: 114-116°C |
| a-187 | 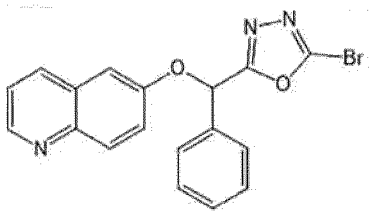 | amorphous |
| a-323 | 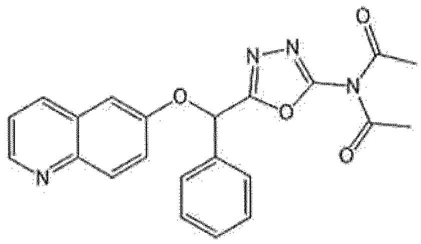 | $n_D$(22.1 °C) 1.5802 |
| b-1 | 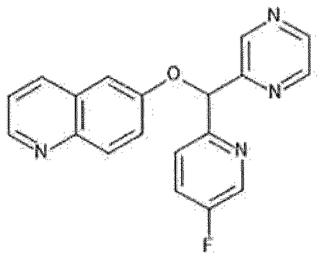 | viscous oil |
| b-2 | 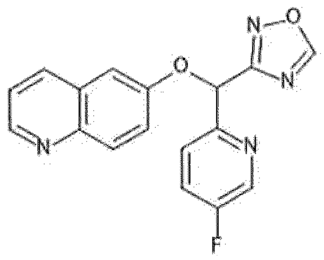 | m.p.: 115-119°C |
| b-3 | 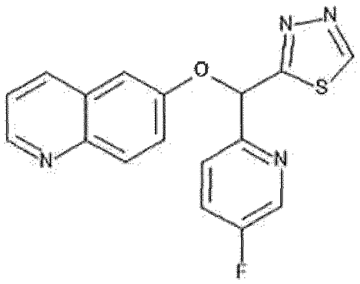 | m.p.: 92-94°C |

(continued)

| Compound No. | Structure | Physical Property |
|---|---|---|
| b-4 | 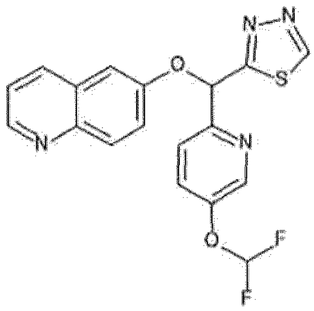 | m.p.: 60-62°C |
| b-5 | 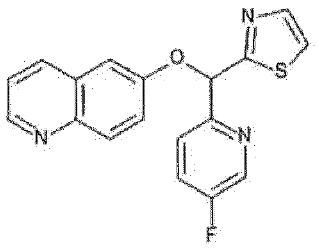 | viscous oil |
| b-6 | 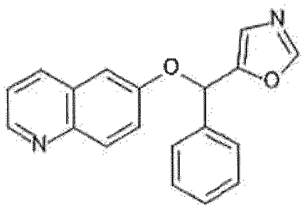 | m.p,: 110-113°C |
| b-7 | 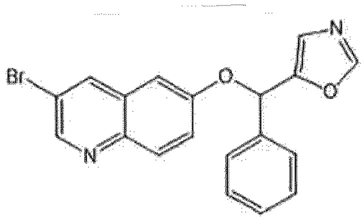 | viscous oil |
| b-8 | 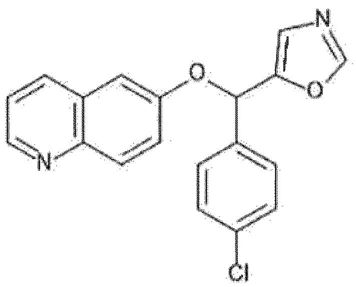 | amorphous |
| b-9 | 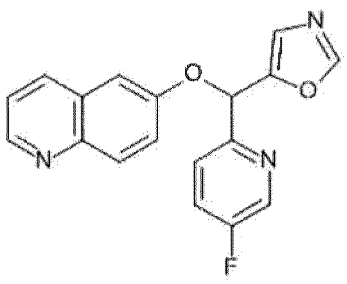 | viscous oil |

(continued)

| Compound No. | Structure | Physical Property |
|---|---|---|
| b-10 | 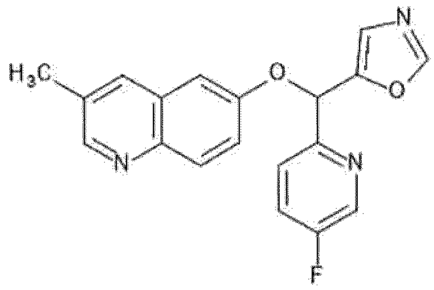 | viscous oil |
| b-11 | 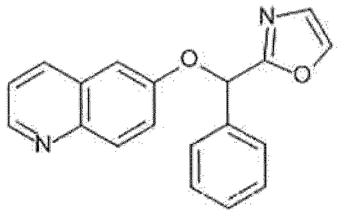 | m.p. : 122-124°C |
| b-12 | 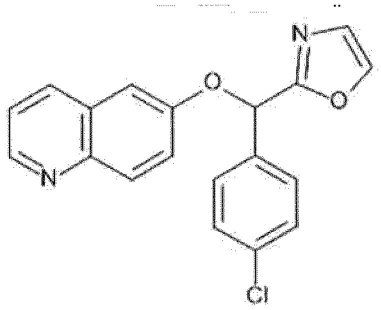 | amorphous |
| b-13 | 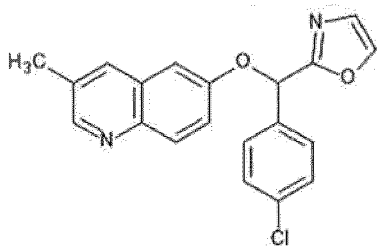 | viscous oil |
| b-14 | 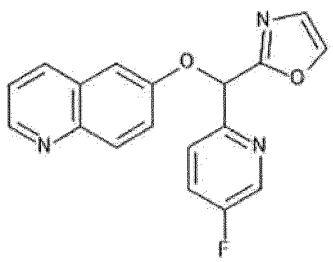 | m.p.: 104-110°C |

(continued)

| Compound No. | Structure | Physical Property |
|---|---|---|
| b-15 | 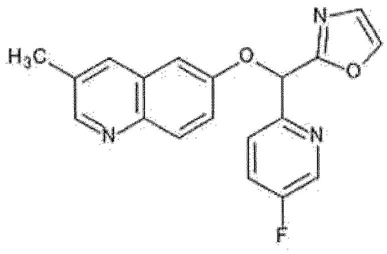 | viscous oil |
| b-16 | 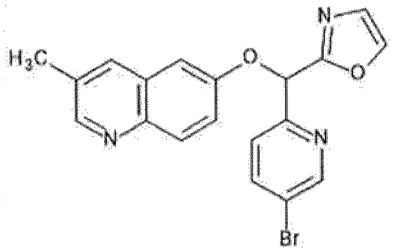 | m.p.: 136-139°C |
| b-17 | 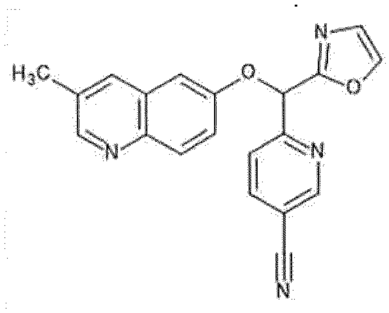 | m.p.: 168-172°C |
| b-18 | 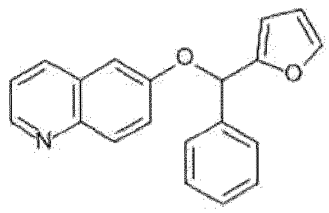 | m.p.: 215°C decomp. |
| b-19 | 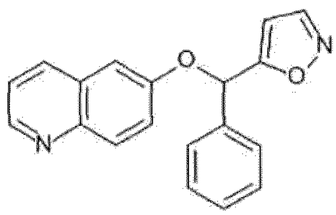 | viscous oil |
| b-20 | 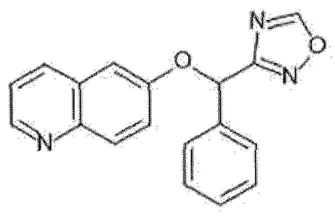 | m.p.:138-140°C |

(continued)

| Compound No. | Structure | Physical Property |
|---|---|---|
| b-21 | 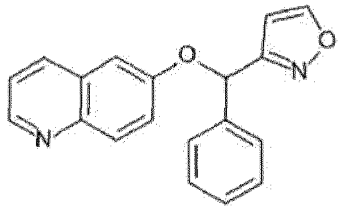 | m.p.:126-128°C |
| b-22 | 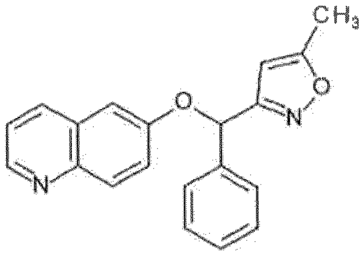 | m.p.:1 08-11 0°C |
| b-23 | 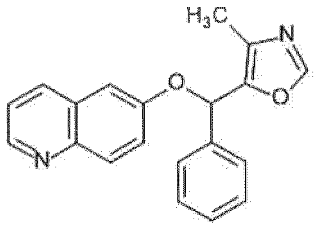 | viscous oil |
| b-24 | 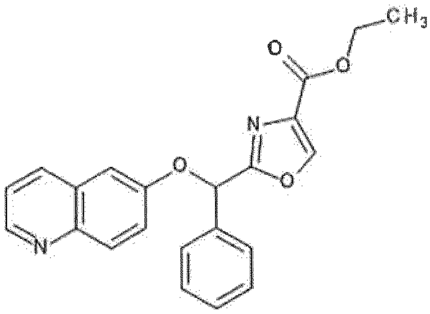 | m.p.: 103-107°C |
| b-25 | 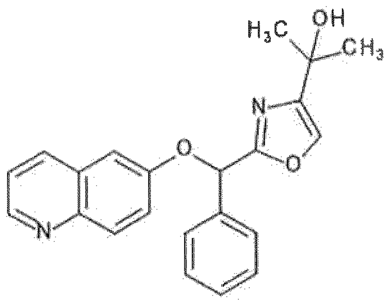 | m.p.: 130-134°C |

(continued)

| Compound No. | Structure | Physical Property |
|---|---|---|
| b-26 | 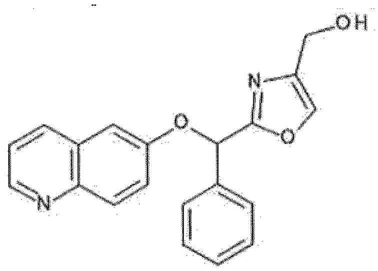 | viscous oil |
| b-27 | 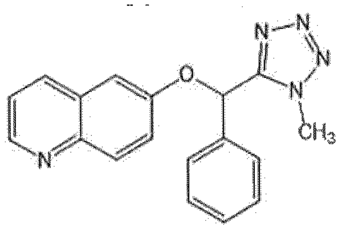 | m.p.: 111-113°C |
| b-28 | 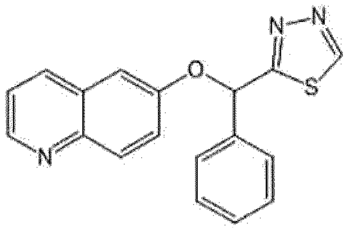 | m.p.:149-151°C |
| b-29 | 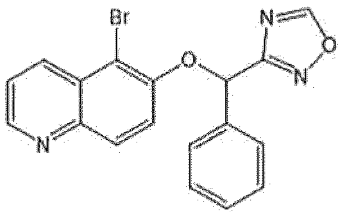 | m.p.:53-55°C |
| b-30 | 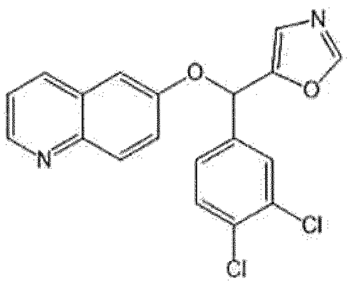 | viscous oil |
| b-31 | 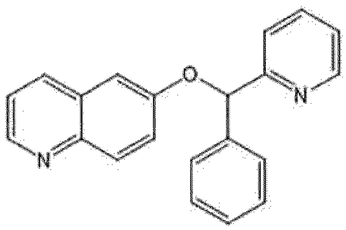 | m.p.:71-73°C |

(continued)

| Compound No. | Structure | Physical Property |
|---|---|---|
| b-32 | 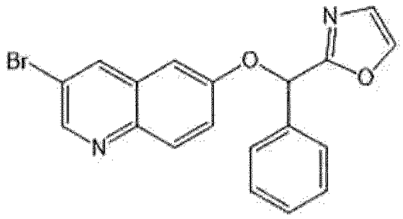 | m.p.: 136-137°C |
| b-33 | 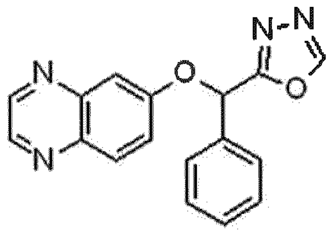 | m.p.: 155-157°C |
| b-34 | 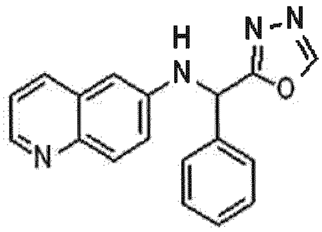 | m.p.: 161-163°C |
| b-35 | 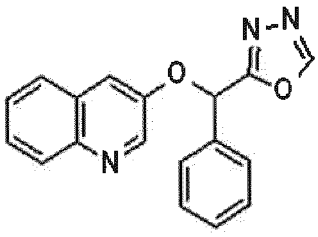 | m.p.: 108-110°C |
| b-36 | 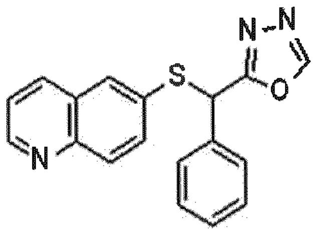 | m.p.: 126-130°C |
| b-37 | 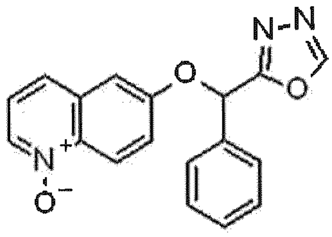 | amorphous |

(continued)

| Compound No. | Structure | Physical Property |
|---|---|---|
| b-38 | 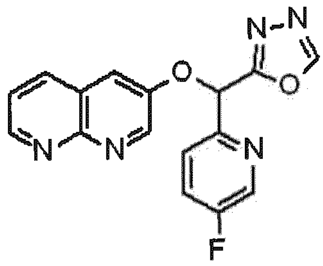 | m.p.: 154-158°C |
| b-39 | 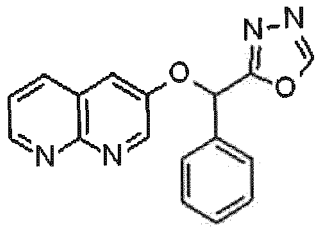 | viscous oil |
| b-40 | 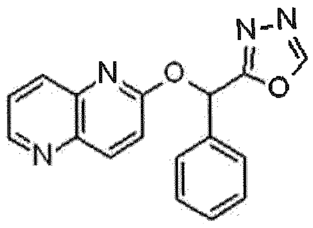 | viscous oil |
| b-41 | 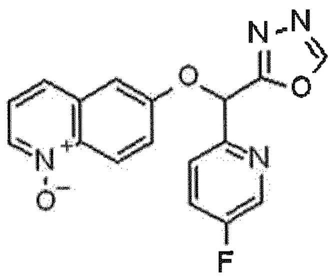 | m.p.: 176-177°C |
| b-42 | 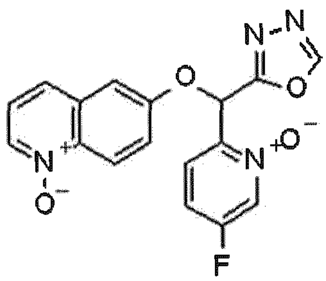 | m.p.: 224-226°C |
| b-43 | 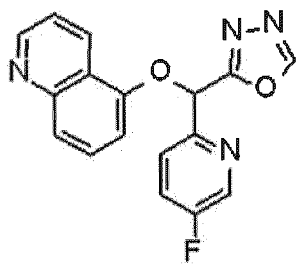 | m.p.: 198-200°C |

(continued)

| Compound No. | Structure | Physical Property |
|---|---|---|
| b-44 | 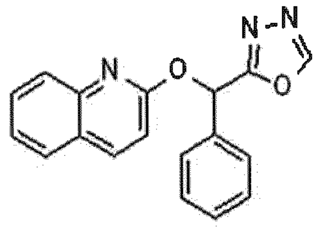 | viscous oil |
| b-45 | 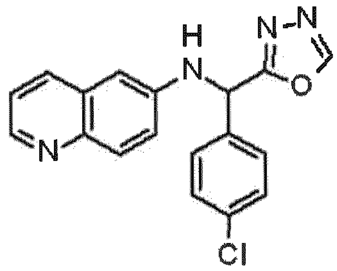 | m.p.: 151-152°C |
| b-46 | 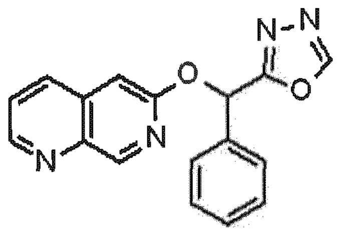 | m.p.: 136-139°C |
| b-47 | 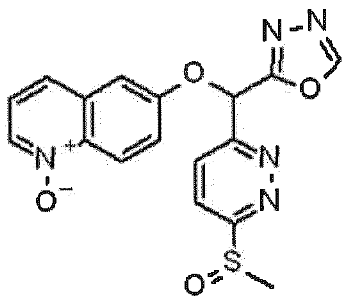 | amorphous |
| b-48 | 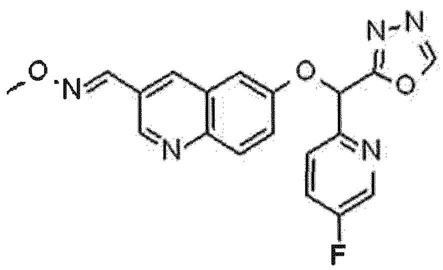 | m.p.: 148-152 °C |

(continued)

| Compound No. | Structure | Physical Property |
|---|---|---|
| b-49 | 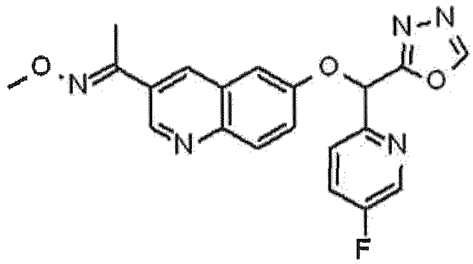 | viscous oil |
| b-50 | 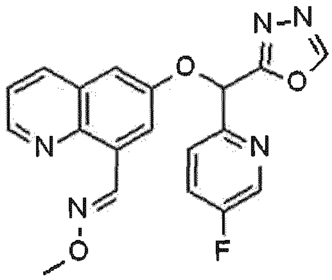 | m.p.: 164-166°C |
| b-51 | 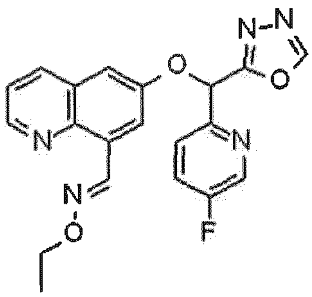 | m.p. :182-184°C |
| b-52 | 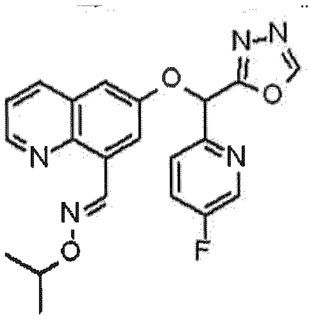 | m.p.: 158-159°C |
| b-53 | 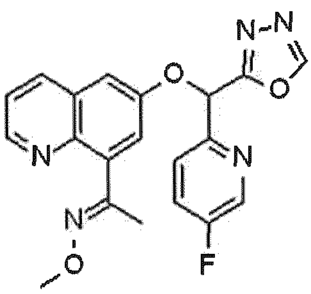 | viscous oil |

(continued)

| Compound No. | Structure | Physical Property |
|---|---|---|
| b-54 | 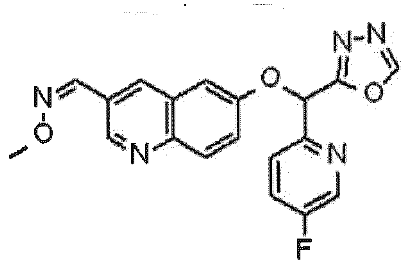 | viscous oil |
| b-55 | 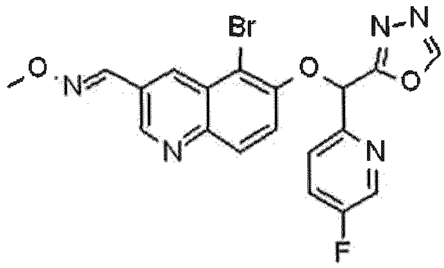 | m.p.:172-173°C |
| b-56 | 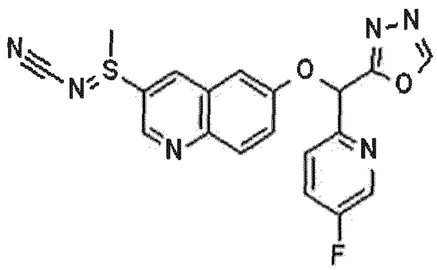 | viscous oil |
| b-57 | 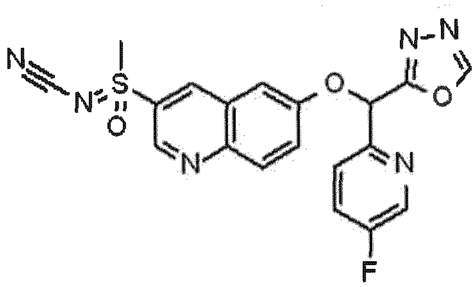 | viscous oil |
| b-58 | 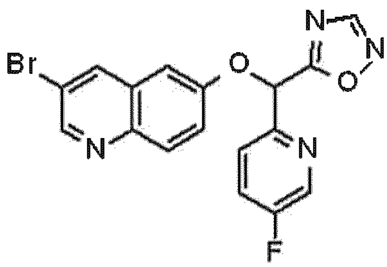 | viscous oil |

(continued)

| Compound No. | Structure | Physical Property |
|---|---|---|
| b-59 | 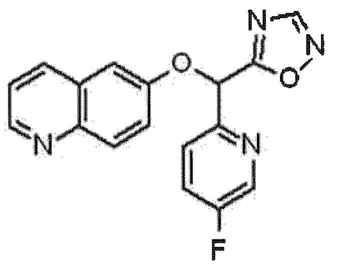 | m.p.: 100-104°C |
| b-60 | 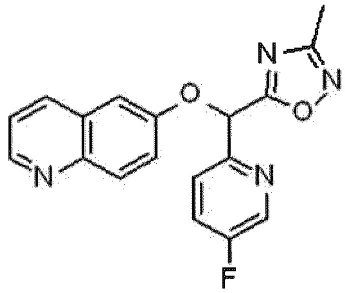 | m.p.: 79-81°C |
| b-61 | 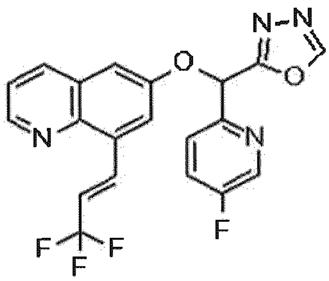 | m.p.: 147-150°C |
| b-62 | 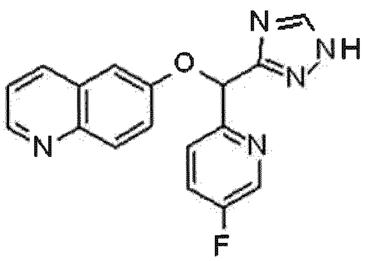 | viscous oil |
| b-63 | 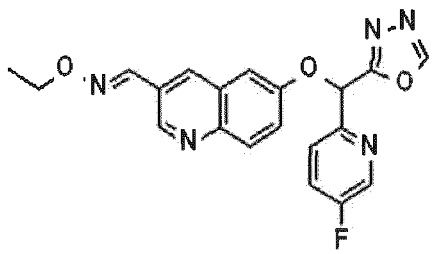 | m.p.: 130-133 °C |

(continued)

| Compound No. | Structure | Physical Property |
|---|---|---|
| b-64 | 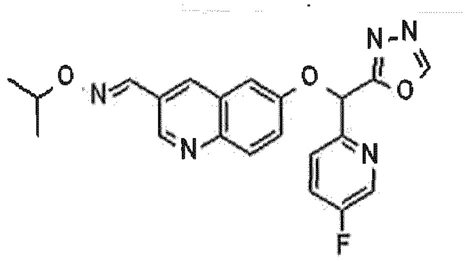 | m.p.: 112-115°C |
| b-65 | 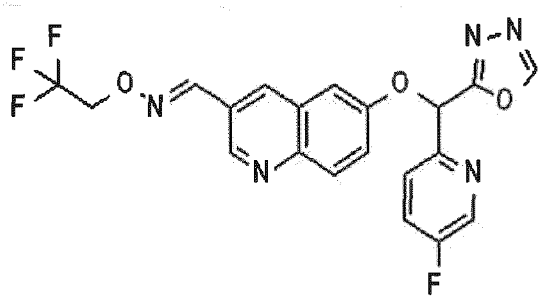 | m.p.:118-120°C |
| b-66 | 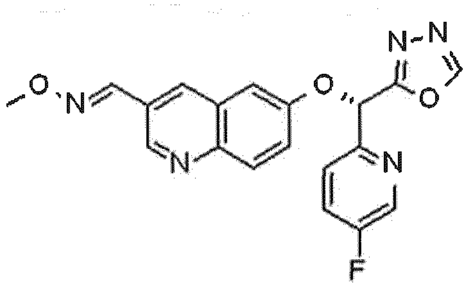 | m.p : 136-137°C |
| b-67 | 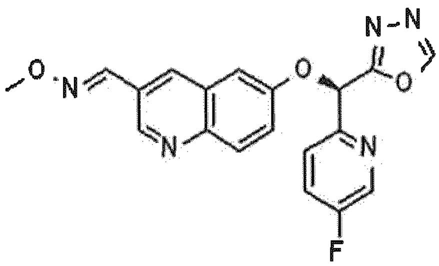 | m.p.: 136-138°C |
| b-68 | 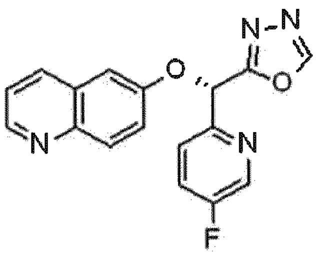 | viscous oil |

(continued)

| Compound No. | Structure | Physical Property |
|---|---|---|
| b-69 | 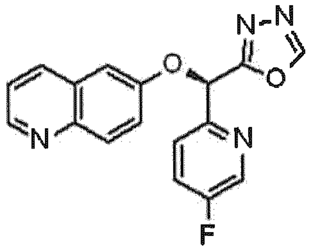 | viscous oil |
| b-70 | 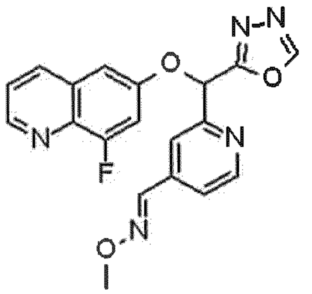 | m.p.: 174-177°C |
| b-71 | 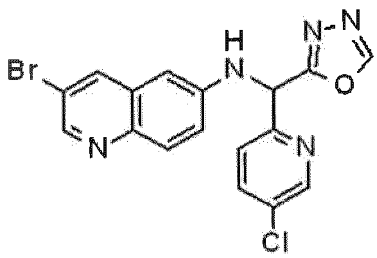 | m.p.: 214-217°C |
| b-72 | 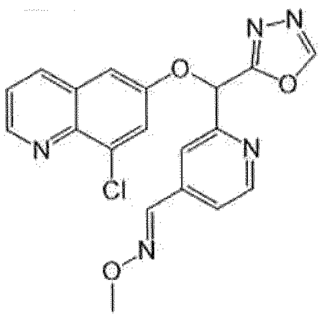 | m.p.: 166-168°C |
| b-73 | 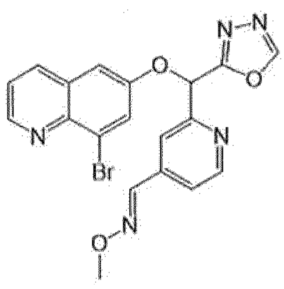 | m.p.: 165-167°C |

(continued)

| Compound No. | Structure | Physical Property |
|---|---|---|
| b-74 | 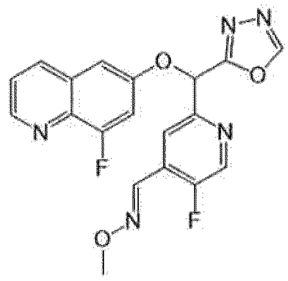 | amorphous |
| b-75 | 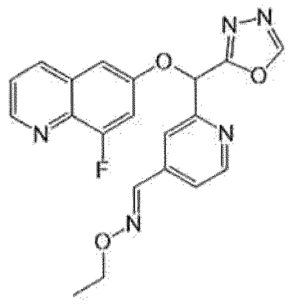 | m.p.: 168-171°C |
| b-76 | 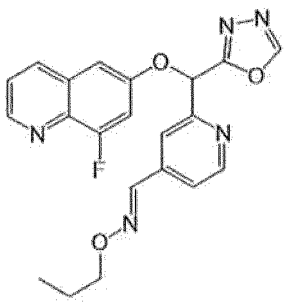 | viscous oil |
| b-77 | 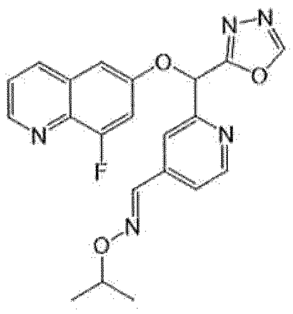 | m.p.: 126-129°C |

(continued)

| Compound No. | Structure | Physical Property |
|---|---|---|
| b-78 | 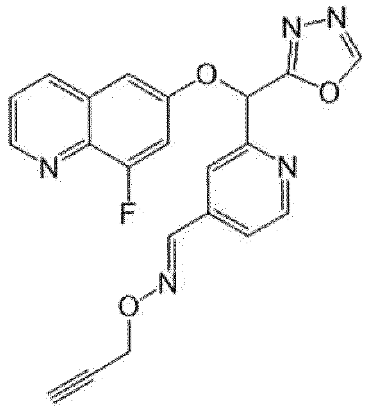 | m.p.: 128-130°C |

Among the compounds shown in Tables 1 and 2, $^1$H-NMR ($CDCl_3$) data were measured for compounds having physical properties of viscous oil or amorphous. The measured values are shown in Table 3.

Table 3

| Compound No. | $^1$H-NMR ($CDCl_3$-$d_6$, δ ppm) |
|---|---|
| a-2 | 6.73 (s, 1 H), 7.13 (dd, 2H), 7.26 (s, 1 H), 7.32-7.37 (m, 1 H), 7.46-7.50 (m, 1 H), 7.60-7.66 (m, 2H), 7.97-8.04 (m, 2H), 8.39 (s, 1 H), 8.79 (s, 1H) |
| a-17 | 2.47 (s, 3H), 6.69 (s, 1H), 7.21 (d, 1H), 7.27-7.30 (m 2H), 7.33-7.36 (m, 1H), 7.47-7.51 (m, 1H), 7.53 (d, 2H), 7.99-8.03 (m, 2H), 8.38 (s, 1H), 8.78 (d, 1H). |
| a-18 | 2.73-3.06 (m, 3H), 6.81 -6.84 (m, 1 H), 7.23-7.24 (m, 1 H), 7.35-7.39 (m, 1 H), 7.49 (dd, 1 H), 7.72-7.74 (m, 2H), 7.81-7.88 (m, 2H), 8.00-8.07 (m, 2H), 8.40 (d, 1 H), 8.80 (dd, 1 H). |
| a-19 | 3.06 (s, 3H), 6.84 (s, 1H), 7.23 (d, 1H), 7.36 (dd, 1H), 7.50 (dd, 1H), 7.86 (d, 2H), 8.01-8.07 (m, 4H), 8.41 (s, 1H), 8.80 (dd, 1H). |
| a-23 | 7.04 (s, 1H), 7.10-7.19 (m, 1H), 7.20-7.28 (m, 2H), 7.30-7.43 (m, 2H), 7.49 (d, 1H), 7.74-7.83 (m, 1H), 7.98-8.07 (m, 2H), 8.42 (s, 1H), 8.78 (s, 1H) |
| a-25 | 7.02-7.06 (m, 2H), 7.30-7.52 (m, 5H), 8.03 (d, 2H), 8.45 (s, 1H), 8.80 (s, 1H) |
| a-26 | 6.84 (s, 1 H), 7.12-7.58 (m, 6H), 7.97-8.10 (m, 2H), 8.40 (s, 1 H), 8.77 (s, 1 H) |
| a-27 | 6.70 (s, 1 H), 7.12 (s, 1 H), 7.25 (s, 1 H), 7.27-7.37 (m, 2H), 7.43 (d, 1 H), 7.98-8.07 (m, 2H), 8.42 (s, 1 H), 8..79 (s, 1 H) |
| a-28 | 6.74 (s, 1 H), 6.81 (s, 1 H), 7.25-7.49 (m, 6H), 8.01 -8.07 (m, 2H), 8.41 (s, 1H) 8.79 (s, 1H) |
| a-29 | 6.78 (s, 1 H), 7.16 (s, 1 H), 7.25 (s, 1 H), 7.36-7.43 (m, 2H), 8.02-8.08 (m, 2H), 8.46 (s, 1 H), 8.82 (s, 1H) |
| a-32 | 7.37 (d, 1H), 7.38 (dd, 1H), 7.44 (dd, 1H), 7.55 (s, 1H), 7.60 (s, 1H), 7.96 (s, 1H), 8.05 (s, 1H), 8.08 (s, 1H), 8,55 (s, 1H), 8.84 (dd, 1H). |
| a-35 | 7.13 (s, 1 H), 7.30-7.37 (m, 2H), 7.49 (d, 1 H), 7.57-7.63 (m, 1 H), 7.98-8.07 (m, 3H), 8.48 (s, 1 H), 8.78 (s, 1H), 9.22 (s, 1 H) |
| a-38 | 2.71 (s, 3H), 7.03 (s, 1H), 7.32-7.39 (m, 2H), 7.40-7.49 (m, 2H), 7.74 (d, 1H), 8.01 (s, 1H), 8.04 (s, 1H), 8.47 (s, 1H), 8.78 (s, 1H) |
| a-39 | 3.01 (s, 3H), 7.20 (s, 1 H), 7.32-7.41 (m, 2H), 7.46-7.53 (m, 1 H), 8,02-8.09 (m, 2H), 8.32 (s, 1 H), 8.34 (s, 1 H), 8.50 (s, 1 H), 8.80 (s, 1 H) |
| a-40 | 7.24 (s, 1 H), 7.32-7.41 (m, 2H), 7.48 (d, 1 H), 7.97 (d, 1 H), 8.00-8.09 (m, 2H), 8.27 (d, 1 H), 8.50 (s, 1 H), 8.80 (s, 1 H) |

(continued)

| Compound No. | $^1$H-NMR ($CDCl_3$-$d_6$, δ ppm) |
|---|---|
| a-44 | 6.99 (s, 1 H), 7.32 (s, 1 H), 7.33-7.43 (m, 2H), 7.59 (d, 1 H), 8.00-8.06 (m, 2H), 8.50 (s, 1 H), 8.80 (s, 1 H), 8.85 (s, 1 H), 8.86 (s, 1 H) |
| a-45 | 6.98 (s, 1H), 7.27 (d, 1H), 7.34 (dd, 1H), 7.52 (dd, 1H), 800 (d, 1H), 8.03 (d, 1H), 8.48 (s, 1H), 8.69 (s, 2H), 8.79 (dd, 1H). |
| a-46 | 6.95 (s, 1 H), 7.27 (d, 1 H), 7.34 (dd, 1 H), 7.52 (dd, 1 H), 8.01 (d, 1 H), 8.03 (d, 1 H), 8.48 (s, 1 H), 8.79-8.81 (m, 3H). |
| a-47 | 6.82 (s, 1H), 7.28 (s, 1H), 7.33-7.46 (m, 2H), 7.51 (d, 1H), 7 98-8.10 (m, 3H), 8.45 (s, 1H), 8.68 (s, 1H), 8.81 (s, 1H), 8.90 (s, 1H) |
| a-48 | 6.78 (s, 1H), 7.25 (s, 1H), 7.36 (dd, 1H), 7.42 (d, 1H), 7.47-7.52 (m, 1H), 7.96 (dd, 1H), 8.01 (d, 1H), 8.07 (d, 1H), 8.42 (s, 1H), 8.66 (d, 1H), 8.81 (dd, 1H). |
| a-50 | 6.87 (s, 1 H), 7.28-7.38 (m, 3H), 7.50-7.55 (m, 1 H), 7.78-7.84 (m, 2H), 8.00-8.08 (m, 2H), 8.45 (s, 1 H), 8.63 (s, 1 H), 8.80 (s, 1H) |
| a-55 | 684 (s, 1 H), 7.32 (d, 1H), 7.37 (dd, 1H), 7.51-7.56 (m, 2H), 8.04-8.09 (m, 3H), 8.43-8.45 (m, 2H), 8.81 (dd, 1H). |
| a-56 | 2.51 (s, 3H), 6.80 (s, 1H), 7.10 (dd, 1H), 7.30 (d, 1H), 7.35 (dd, 1H), 7.55 (dd, 1H), 7.61 (d, 1H), 8.04-8.07 (m, 2H), 8.40 (d, 1H), 8.43 (s, 1H), 8.80 (dd, 1H). |
| a-57 | 2.41 (s, 3H), 6.82 (s, 1H), 7.14 (d, 1H), 7.29 (d, 1H), 7.35 (dd, 1H), 7.53 (dd, 1H), 7.63 (s, 1H), 8.03-8.07 (m, 2H), 8.43 (s, 1H), 8.48 (d, 1H), 8.80 (dd, 1H). |
| a-63 | 3.88 (s, 3H), 6.82 (s, 1 H), 7.28-7.30 (m, 2H), 7.36 (dd, 1 H), 7.52 (dd, 1 H), 7.73 (d, 1 H), 8.03 (dt, 1 H), 8.32 (d, 1 H), 8.43 (s, 1 H), 8.80 (dd, 1H) |
| a-64 | 3.28 (s, 1H), 6.86 (s, 1H), 7.29 (d, 1H), 7.37 (dd, 1H), 7.53 (dd, 1H), 7.81 (d, 1H), 7.90 (dd, 1H), 8.05 (dd, 2H), 8.46 (s, 1H), 8.71 (t, 1H), 8.81 (dd, 1H) |
| a-66 | 3.97 (s, 3H), 6.93 (s, 1 H), 7.31 (d, 1 H), 7.37 (dd, 1 H), 7.54 (dd, 1 H), 7.96 (d, 1 H), 8.05 (dd, 2H), 8.43 (dd, 1 H), 8.47 (s, 1 H), 8.81 (s, 1 H), 9.21 (d, 1H) |
| a-67 | 5.44 (d, 1H), 5.87 (d, 1H), 6.72 (dd, 1H), 6.85 (s, 1H), 7.29 (d, 1H), 7.36 (dd, 1H), 7.53 (dd, 1H), 7.78 (d, 1H), 7.85 (dd, 1H), 8.04 (dd, 2H), 8.44 (s, 1H), 8.62 (d, 1H), 8.80 (dd, 1H) |
| a-68 | 6.58 (t, 1H), 6.87 (s, 1H), 7.30 (d, 1H), 7.38 (dd, 1H), 7.53 (dd, 1H), 7.62 (dd, 1H), 7.87 (d, 1H), 8.05 (t, 2H), 8.44 (s, 1H), 8.49 (d, 1H), 8.82 (dd, 1H) |
| a-70 | 2.51 (s, 3H), 6.78 (s, 1H), 7.18 (d, 1H), 7.49 (dd, 1H), 7.61-7.69 (m, 2H), 7.99 (d, 1H), 8.17 (d, 1H), 8.42 (s, 1H), 8.47 (d, 1H), 8.76 (d, 1H). |
| a-74 | 1.26 (t, 3H), 2.68 (q, 2H), 6.85 (s, 1H), 7.30 (d, 1H), 7.32-7.37 (m, 1H), 7.53 (dd, 1H), 7.63 (dd, 1H), 7.73 (d, 1H), 8.03 (dd, 2H), 8.45 (s, 1H), 8.48 (d, 1H), 8.79 (dd, 1H) |
| a-75 | 6.91 (s, 1 H), 7.30 (s, 1 H), 7.33-7.40 (m, 1 H), 7.51 (d, 1 H), 7.99-8.09 (m, 4H), 8.44 (s, 1 H), 8.80 (s, 1 H), 8.88 (s, 1 H) |
| a-79 | 7.02 (s, 1 H), 7.32-7.35 (m, 2H), 7.56-7.62 (m, 2H), 7.75-7.79 (m, 1 H), 7.86 (d, 1 H), 7.93 (d, 1 H), 8.01 (d, 1 H), 8.06 (d, 1 H), 8.11 (d, 1 H), 8.29 (d, 1 H), 8.45 (s, 1 H), 8.79-8.80 (m, 1 H) |
| a-80 | 7.04 (s, 1 H), 7.34-7.37 (m, 2H), 7.59-7.62 (m, 1 H), 7.65-7.69 (m, 1 H), 7.73-7.77 (m, 1 H), 7.90 (d, 1 H), 8.02-8.08 (m, 3H), 8.17 (s, 1 H), 8.43 (s, 1 H), 8.79-8.80 (m, 1 H) |
| a-81 | 4.10 (s, 2H), 6.78 (s, 1H), 7.20-7.30 (m, 7H), 7.31-7.38 (m, 1H), 7.49 (d, 1H), 7.61-7.70 (m, 2H), 7.99-8.06 (m, 2H), 8.41 (s, 1H), 8.49 (s, 1H), 8.79 (s, 1H) |
| a-83 | 6.95 (s, 1H), 7.30 (s, 1 H), 7.31 -7.40 (m, 1 H), 7.53 (d, 1 H), 8.01 - 8.12 (m, 3H), 8.45 (s, 1H), 8.59-8.63 (m, 1H), 8.81 (s, 1H), 9.41 (s, 1H) |
| a-84 | 6.89 (s, 1 H), 7.30 (s, 1 H), 7.33-7.40 (m, 1 H), 7.52 (d, 1 H), 7.95 (d, 1 H), 8.02-8.13 (m, 3H), 8.44 (s, 1 H), 8.80 (s, 2H) |

(continued)

| Compound No. | $^1$H-NMR ($CDCl_3$-$d_6$, δ ppm) |
|---|---|
| a-85 | 2.53-2.54 (m, 3H), 6.80 (s, 1 H), 7.27-7.28 (m, 1 H), 7.35-7.38 (m, 1 H), 7.42 (t, 1 H), 7.50-7.53 (m, 1 H), 7.65-7.68 (m, 1 H), 8.02-8.06 (m, 2H), 8.44 (s, 1 H), 8.80-8.81 (m, 1 H) |
| a-86 | 6.80 (s, 1 H), 7.26-7.27 (m, 1 H), 7.37-7.40 (m, 1H), 7.49-7.52 (m, 1 H), 7.61 (t, 1 H), 7.81 -7.83 (m, 1 H), 8.04-8.08 (m, 2H), 8.46 (s, 1 H), 8.82-8.83 (m, 1 H) |
| a-87 | 2.36 (s, 3H), 6.80 (s, 1 H), 7.28-7.29 (m, 1 H), 7.35-7.39 (m, 1 H), 7.51 -7.54 (m, 1 H), 7.68-7.69 (d, 1 H), 8.03-8.07 (m, 2H), 8.36 (s, 1 H), 8.44 (s, 1 H), 8.81-8.82 (m, 1 H) |
| a-96 | 2.52 (s, 3H), 6.77 (s, 1H), 7.29 (d, 1H), 7.37 (dd, 1H), 7.51 (s, 1H), 7.52 (dd, 1H), 8.04-8.08 (m, 2H), 8.44 (s, 1H), 8.51 (s, 1H), 8.82 (dd, 1H). |
| a-100 | 2.58 (s, 3H), 6.85 (s, 1H), 7.23 (s, 1H), 7.40 (d, 1H), 7.48 (s, 5H), 7.74 (s, 1H), 7.81 (s, 1H), 7.96 (d, 1H), 8.43 (s, 1H), 8.66 (s, 2H) |
| a-102 | 2.36 (s, 3H), 2.54 (s, 3H), 6.79 (s, 1H), 7.28 (d, 1H), 7.35 (dd, 1H), 7.52 (dd, 1H), 7.55 (s, 1H), 8.02-8.06 (m, 2H), 8.34 (s, 1H), 8.43 (s, 1H), 8.80 (d, 1H) |
| a-103 | 2,27 (s, 3H), 2.31 (s, 3H), 6.78 (s, 1 H), 7.28 (d, 1 H), 7.34 (dd, 1 H), 7.52 (dd, 1 H), 7.55 (s, 1 H), 8.02-8.05 (m, 2 H), 8.35 (s, 1 H), 8.43 (s, 1 H), 8.79 (d, 1H). |
| a-105 | 2.47 (s, 3H), 6.83 (s, 1 H), 7.19 (d, 1 H), 7.40 (dd, 1 H), 7.49-7.54 (m, 1 H), 7.77 (d, 1 H), 7.83 (dd, 1 H), 8.00 (d, 1 H), 8.42 (s, 1 H), 8.46 (d, 1 H), 8.64 (d, 1H). |
| a-106 | 1.33 (t, 3H), 2.80 (q, 2H), 6.86 (s, 1 H), 7.25 (d, 1 H), 7.45 (dd, 1 H), 7.53 (dt, 1 H), 7.80 (d, 1 H), 7.87 (dd, 1 H), 8.01 (d, 1 H), 8.47 (s, 1 H), 8.48 (d, 1 H), 8.68 (d, 1 H) |
| a-107 | 6.85 (s, 1 H), 7.22 (d, 1 H), 7.49-7.57 (m, 2H), 7.85 (dd, 1 H), 8.01 -8.04 (m, 2H), 8.46 (s, 1 H), 8.49 (d, 1 H), 8.70 (d, 1 H) |
| a-122 | 6.89 (s, 1 H), 7.25 (s, 1 H), 7.33-7.60 (m, 6H), 7.67 (d, 2H), 7.83-7.91 (m, 1 H), 7.84 (d, 1 H), 8.43 (s, 1 H), 8.49 (s, 1 H), 8.82 (s,1 H) |
| a-123 | 6.88 (s, 1H), 7.17-7.56 (m, 8H), 7.83-7.90 (m, 1H), 8.05 (d, 1H), 8.43 (s, 1H), 8.47 (s, 1H), 8.80 (s, 1H) |
| a-125 | 6.89 (s, 1H), 7.13-7.19 (m, 2H), 7.28 (s, 1H), 7.33-7.39 (m, 1H), 7.50-7.57 (m, 2H), 7.61-7.67 (m, 2H), 7.85-7.89 (m, 1H), 8.06 (d, 1H), 8.43 (s, 1H), 8.49 (s, 1H), 8.82 (s, 1H) |
| a-126 | 6.87 (s, 1 H), 7.30-7.55 (m, 7H), 7.87-7.90 (m, 1 H), 8.06 (d, 1 H), 8.43 (s, 1H). 8.47 (s, 1 H), 8.76 (s, 1 H) |
| a-127 | 2.51 (s, 3H), 6.89 (s, 1H), 7.25-756 (m, 8H), 7.85-7.89 (m, 1H), 8.06 (d, 1H), 8.43 (s, 1H), 8.48 (s, 1H), 8.81 (s, 1H) |
| a-129 | 6.87 (s, 1H), 7.22-7.35 (m, 6H), 7.40 (s, 1H), 7.50-7.54 (m, 1H), 7.83-7.89 (m, 1H), 8.05 (d, 1H), 8.43 (s, 1H), 8.48 (s, 1H), 8.77 (s, 1H) |
| a-130 | 6.88 (s, 1 H), 7.24-7.45 (m, 6H), 7.50-7.54 (m, 1 H), 7.85-7.90 (m, 1 H), 8.06 (d, 1 H), 8.43 (s, 1 H), 8.46 (s, 1 H), 8.75 (s, 1 H) |
| a-131 | 6.89 (s, 1H), 7.08-7.13 (m, 1H), 7.29 (s, 1H), 7.37-7.57 (m, 6H), 7.84-7.90 (m, 1H), 8.07 (d, 1H), 8.43 (s, 1H), 8.49 (s, 1H), 8.82 (s, 1 H) |
| a-132 | 3.10 (s, 3H), 6.90 (s, 1H), 7.36 (s, 1H), 7.39-7.42 (m, 1H), 7.51 - 7.60 (m, 2H), 7.68 (dd, 1H), 7.88-7.81 (m, 1H), 7.97-8,12 (m, 3H), 8.23 (s, 1H). 8.44 (s, 1H), 8.47 (s, 1H), 8.79 (s, 1H) |
| a-134 | 6.89 (s, 1H), 7.32 (s, 1H), 7.39-7.57 (m, 4H), 7.86-7.90 (m, 1H), 8.03-8.11 (m, 2H), 8.43 (s, 1H), 8.48 (s, 1H), 8.64 (s, 1H), 8.81 (s, 1H), 8.89 (s, 1H) |
| a-135 | 6.88 (s, 1 H), 7.30-7.43 (m, 2H), 7.46-7.60 (m, 4H), 7.81-7.88 (m, 1 H), 8.02-8.10 (m, 1 H), 8.40-8.50 (m, 2H), 8.68-8.73 (m, 2 H), 8.80 (s, 1H) |
| a-136 | 6.91 (s, 1H), 7.30-7.42 (m, 3H), 7.50-7.54 (m, 1H), 7.81 (dd, 1H), 7.86-7.92 (m, 1H), 7.99 (s, 1H), 8.07-8.13 (m, 2H), 8.41 (s, 1H), 8.46 (s, 1H), 8.76 (s, 1H), 8.84 (s, 1H) |

(continued)

| Compound No. | $^{1}$H-NMR ($CDCl_3$-$d_6$, δ ppm) |
|---|---|
| a-137 | 4.61 (s, 2H), 6.77 (s, 1H), 7.10-7.51 (m, 9H), 7.76-7.80 (m, 1H), 8.00 (d, 1H), 8.39 (s, 1H), 8.44 (s, 1H), 8.83 (s, 1H) |
| a-138 | 3.70 (s, 3H), 6.39 (s, 1H), 6.88 (s, 1H), 7.37-7.81 (m, 2H), 7.50-7.59 (m, 2H), 7.60 (s, 1H), 7.83-7.90 (m, 1H), 8.09 (d, 1H), 8.44 (s, 1H), 8.48 (s, 1H), 8.88 (s, 1H) |
| a-141 | 0.31 (s, 9H), 6.82 (s, 1H), 7.25 (s, 1H), 7.36-7.40 (m, 1H), 7.48-7.55 (m, 1H), 7.75 (s, 1H), 7.81-7.86 (m, 1H), 8.00 (d, 1H), 8.43 (s, 1H), 8.45 (s, 1H), 8.88 (s, 1H) |
| a-144 | 2.80 (s, 3H), 2.85 (s, 3H), 6.84 (s, 1 H), 7.19 (s, 1 H), 7.50-7.56 (m, 2H) 7.82-7.86 (m, 1 H), 8.39 (dd, 1 H), 8.45 (d, 1 H), 8.50 (d, 1 H), 8.83 (dd, 1 H). |
| a-150 | 3.46-3.48 (m, 3H), 7.56 (s, 1H), 7.67 (dd, 1H), 7.93 (s, 1H), 7.95-7.98 (m, 1H), 8.03-8.06 (m, 1H), 8.34 (d, 1H), 8.51 (s, 1H), 8.65 (d, 1H), 8.95 (dd, 1H), 9.34 (s, 1H). |
| a-154 | 1.34 (s, 3H), 1.36 (s, 3H), 3.06-3.13 (m, 1H), 6.86 (s, 1H), 7.26 (d, 1H), 7.45 (dd, 1H), 7.54 (dt, 1H), 7.81 (d, 1H), 7.87 (dd, 1H), 8.01 (d, 1H), 8.46 (s, 1H), 8.49 (d, 1H), 8.72 (d, 1H) |
| a-155 | 0.00 (s, 9H), 0.85-0.90 (m, 2H), 2.69-2.73 (m, 2H), 6.80 (s, 1H), 7.18 (d, 1H), 7.38 (dd, 1H), 7.47 (dt, 1H), 7.75 (s, 1H), 7.81 (dd, 1H), 7.95 (d, 1H), 8.41 (t, 2H), 8.62 (d, 1H) |
| a-156 | 2.57 (s, 3H), 6.83 (s, 1H), 7.19 (d, 1H), 7.39 (dd, 1H), 7.50-7.55 (m, 1H), 7.71 (d, 1H), 7.83 (dd, 1H), 7.96 (d, 1H), 8.42 (d, 1H), 8.47 (d, 1H), 8.65 (d, 1H), |
| a-157 | 2.85 (d, 3H), 6.84 (s, 1H), 7.36 (s, 1H), 7.51 -7.55 (m, 1H), 7.61 - 7.64 (m, 1H), 7.82-7.86 (m, 1H), 8.11 (d, 1H), 8.41 (d, 1H), 8.44 (s, 1H), 8.49 (d, 1H), 8.83 (dd, 1H). |
| a-162 | 6.84 (t, 1 H), 6.85 (s, 1 H), 7.35 (s, 1 H), 7.51-7.62 (m, 2H), 7.80-7.87 (m, 1 H), 8.10 (d, 1 H), 8.17 (s, 1 H), 8.43 (s, 1 H), 8.48 (s, 1 H), 8.90 (s, 1 H) |
| a-169 | 2.47 (s, 3H), 6.73 (s, 1 H), 7.16 (s, 1 H), 7.37-7.51 (m, 3H), 7.62-7.64 (m, 2H), 7.97 (s, 1 H), 7.99 (d, 1 H), 8.37 (s, 1 H), 8.63 (s, 1 H) |
| a-170 | 6.78 (s, 1 H), 7.40 (s, 1 H), 7.48-7.53 (m, 10H), 7.63-7.70 (m, 3H), 8.39 (s, 1 H), 9.04 (s, 1H) |
| a-174 | 1.49 (s, 9H), 4.49 (bs, 1H), 6.53 (d, 1H), 6.63 (s, 1H), 7.07 (d, 1H), 7.27-7.30 (m, 1H), 7.35-7.44 (m, 3H), 7.57-7.62 (m, 4H), 8.35 (s, 1H). |
| a-177 | 6.78 (s, 1 H), 7.30 (s, 1 H), 7.37-7.47 (m, 3H), 7.58-7.69 (m, 4H), 8.11-8.20 (m, 2H), 8.40 (s, 1H) |
| a-187 | 6.67 (s, 1 H), 7.22 (s, 1 H), 7.32-7.40 (m, 1 H), 7.40-7.54 (m, 4H), 7.63 (d, 2H), 8.01 -8.06 (m, 2H), 8.81 (s, 1 H) |
| a-188 | 6.78 (s, 1 H), 7.24 (s, 1 H), 7.37-7.41 (m, 1 H), 7.51-7.59 (m, 3H), 8.01-8.10 (m, 2H), 8.43 (s, 1 H), 8.72 (s, 1 H), 8.73 (s, 1 H), 8.82 (s, 1H) |
| a-200 | 8.85 (dd, 1 H), 8.41 (d, 1 H), 8.36 (s, 1 H), 8.10 (s, 1 H), 8.07 (s, 1 H), 7.67-7.64 (m, 2H), 7.54-7.37 (m, 7H), 6.81 (s, 1 H). |
| a-206 | 8.78 (dd, 1 H), 8.50 (d, 1 H), 8.43 (s, 1 H), 8.01 (d, 1 H), 7.90 (s, 1 H), 7.86 (dd, 1 H), 7.58-7.53 (m, 1 H), 7.33-7.30 (m, 1 H), 7.21 (s, 1 H), 6.89 (s, 1 H), 2.92-2.88 (m, 2H), 1.82-1.75 (m, 1 H), 1.05 (t, 3H). |
| a-208 | 9.07 (d, 1H), 8.50 (d, 1H), 8.45 (s, 1H), 8.18 (d, 1H), 8.09 (d, 1H), 7.90-7.86 (m, 1H), 7.71-7.68 (m, 2H), 7.57-7.50 (m, 4H), 7.46-7.42 (m, 1H) 7.35 (d, 1H), 6.88 (s, 1H). |
| a-209 | 9.00 (dd, 1 H), 8.49 (d, 1 H), 8.45 (s, 1 H), 8.20 (s, 1 H), 8.10 (d, 1 H), 7.89-7.86 (m, 1 H), 7.57-7.51 (m, 3H), 7.45-7.41 (m, 1 H), 7.34-7.21 (m, 3H), 6.88 (s, 1H). |
| a-212 | 3.69 (s, 3H), 6.88 (s, 1 H), 7.03-7.09 (m, 2H), 7.30-7.33 (m, 3H), 7.38-7.42 (m, 1 H), 7.50-7.55 (m, 2H), 7.86-7.90 (m, 1 H), 8.03 (d, 1 H), 8.45 (s, 1 H), 8.47 (d, 1 H), 8.76 (d, 1 H) |
| a-213 | 6.88 (d, 1 H), 7.26-7.38 (m, 3H), 7.43-7.45 (m, 1 H), 7.50-7.64 (m, 3H), 7.80-7.89 (m, 2H), 8.07 (d, 1 H), 8.44 (d, 1 H), 8.48 (d, 1 H), 8.75 (d, 1H) |

(continued)

| Compound No. | $^{1}$H-NMR ($CDCl_3$-$d_6$, δ ppm) |
|---|---|
| a-214 | 3.88 (s, 3H), 6.90 (s, 1 H), 7.03 (d, 2H), 7.24 (d, 1 H), 7.35-7.38 (m, 1 H), 7.51 -7.57 (m, 2H), 7.64 (d, 2H), 7.87-7.90 (m, 1 H), 8.07 (d, 1 H), 8.44 (s, 1 H), 8.49 (d, 1 H), 8.83 (d, 1H) |
| a-215 | 3.86 (s, 3H), 6.90 (s, 1 H), 6.97 (d, 1 H), 7.22-7.29 (m, 3H), 7.36-7.42 (m, 2H), 7.51 -7.57 (m, 2H), 7.87-7.90 (m, 1 H), 8.08 (d, 1 H), 8.45 (s, 1 H), 8.49 (d, 1 H), 8.84 (d, 1 H) |
| a-216 | 6.90 (s, 1 H), 7.24 (d, 1 H), 7.37-7.45 (m, 2H), 7.51-7.57 (m,1 H), 7.63 (d, 1H), 7.68 (d, 1 H), 7.87-7.90 (m, 1 H), 7.96 (d, 1 H), 8.05 (d, 1 H), 8.44 (s, 1 H), 8.49 (d, 1 H), 8.86 (d, 1 H) |
| a-219 | 8.87 (dd, 1 H), 8.51 (d, 1 H), 8.46 (s, 1 H), 8.17 (s, 1 H), 8.13 (dd, 1 H), 7.89-7.86 (m, 1 H), 7.62-7.43 (m, 4H), 7.34 (d, 1 H), 7.27 (d, 1 H), 6.90 (s, 1 H). |
| a-222 | 2.16 (s, 3H), 2.34 (s, 3H), 6.88 (s, 1 H), 7.36-7.43 (m, 3H), 7.52-7.59 (m, 1 H), 7.87-7.90 (m, 1 H), 8.09 (d, 1 H), 8.46 (s, 1 H), 8.50 (d, 1 H), 8.81 (d, 1H) |
| a-224 | 8.67 (d, 1 H), 8.49 (d, 1 H), 8.44 (s, 1 H), 7.90-7.87 (m, 1 H), 7.77 (d, 1 H), 7.66-7.64 (m, 1 H), 7.57-7.40 (m, 4H), 7.20 (d, 1 H), 6.88 (s, 1 H), 2.59 (s, 3H). |
| a-225 | 6.87 (s, 1H), 7.40 (s, 1H), 7.50-7.57 (m, 1H), 7.59-7.63 (m, 1H), 7.82-7.87 (m, 1H), 8.11 (d, 1H), 8.44 (s, 1H), 8.49 (s, 1H), 8.57 (s, 1H), 8.71 (s, 1H), 9.45 (s, 1H) |
| a-228 | 9.22 (d, 1 H), 8,67 (d, 1 H), 8.53 (d, 1 H), 8.47 (s, 1 H), 7.89-7.86 (m, 1 H), 7.75 (d, 1 H), 7.65-7.63 (m, 2H), 7.58-7.42 (m, 5H), 6.91 (s, 1 H), 3.16 (s, 3H). |
| a-229 | 8.84 (dd, 1 H), 8.51 (d, 1 H), 8.49 (d, 1 H), 8.46 (s, 1 H), 7.89-7.85 (m, 1 H), 7.67-7.64 (m, 3H), 7.57-7.43 (m, 4H), 7.37 (d, 1 H), 6.90 (s, 1 H), 2.85 (s, 3H). |
| a-230 | 9.30 (d, 1H), 8.69 (d, 1H), 8.52 (d, 1H), 8.47 (s, 1H), 7.89 (d 1H), 7.86-7.82 (m 1H), 7.59-7.52 (m, 1H), 7.40 (d, 1H), 6.86 (s, 1H), 3.18 (s, 3H). |
| a-231 | 8.88 (d, 1H), 8.67-8.65 (m, 2H), 8.50 (d, 1H), 8.46 (s, 1H), 8,06-8.03 (m, 1H), 7.91 -7.88 (m, 1H), 7.78 (d, 1H), 7.59-7.55 (m, 1H), 7.48 (d, 1H), 7.44-7.41 (m, 1H), 7.26 (s, 1H), 6.90 (s, 1H), 260 (s, 3H). |
| a-232 | 7.05 (s, 1 H), 7.29 (d, 1 H), 7.35-7.49 (m, 4H), 7.59 (d 1 H), 7.66 (s, 1 H), 7.67 (s, 1 H), 8.06 (d, 1 H), 8.52 (s, 1 H), 8.71 (s, 2 H), 8.83 (d, 1H), |
| a-235 | 1,20-1.30 (m, 6H), 3.54-3.69 (m, 4H), 5.69 (s, 1 H), 6.83 (s, 1 H), 7.29 (s, 1 H), 7.47-7.54 (m, 2H), 7.81 -7.87 (m, 1 H), 8.03 (d, 1 H), 8.11 (s, 1 H), 8.43 (s, 1 H), 8.47 (s, 1 H), 8.86 (s, 1H) |
| a-238 | 4.04-4.20 (m, 4H), 5.97 (s, 1 H), 6.83 (s, 1 H), 7.29 (s, 1 H), 7.49-7.53 (m, 2H), 7.80-7.85 (m, 1 H), 8.04 (d, 1 H), 8.10 (s, 1 H), 8.43 (s, 1 H), 8.47 (s, 1 H), 8.87 (s, 1 H) |
| a-239 | 6.87 (s, 1H), 7.44 (s, 1H), 7.50-7.56 (m, 1H), 7.62-7.69 (m, 1 H), 7.82-7.88 (m, 1H), 8.11 (d, 1H), 8.44 (s, 1H), 8.49 (s, 1H), 8.50 (s, 1H), 9.23 (s, 1H), 10.21 (s, 1H) |
| a-246 | 8.66 (d, 1H), 8.49 (d, 1H), 8.43 (s, 1H), 8.02 (d, 1H), 7.88-7.85 (m, 1H), 7.79 (s, 1H), 7.56-7.51 (m, 1H), 7.46 (dd, 1H), 7.24 (d, 1H), 6.85 (s, 1H), 2.76 (t, 2H), 1.78 (m, 2H), 1.00 (t, 3H). |
| a-248 | 6.86 (s, 1H), 7.19 (s, 1H), 7.39-7.65 (m, 7H), 7.80-7.89 (m, 1H), 8.21 (s, 1H), 8.43 (s, 1H), 8.49 (s, 1H), 8.78 (s, 1H) |
| a-253 | 6.89 (s, 1 H), 7.33 (s, 1 H), 7.42-7.63 (m, 6H), 7.70 (s, 1 H), 7.82-7.88 (m, 1 H), 8.44 (s, 1 H), 8.45 (s, 1 H), 8.49 (s, 1 H), 8.91 (s, 1 H) |
| a-255 | 8.82 (dd, 1 H), 8.49 (d, 1 H), 8.43 (s, 1 H), 8.03 (dd, 1 H), 7.87-7.84 (m, 1 H), 7.57-7.49 (m, 1 H), 7.40-7.36 (m, 2H), 7.18 (d, 1 H), 6.85 (s, 1 H), 3.48 (t, 2H), 2.69-2.57 (m, 2H). |
| a-256 | 9.06 (d, 1 H), 8.50 (d, 1 H), 8.45 (s, 1 H), 8.16 (d, 1 H), 8.08 (d, 1 H), 7.89-7.86 (m, 1 H), 7.60-7.48 (m, 4H), 7.34-7.32 (m, 3H), 6.88 (s, 1 H), 2.44 (s, 3 H). |
| a-259 | 8.81 (d, 1 H), 8.49 (d, 1 H), 8.44 (s, 1 H), 8.11 (d, 1 H), 7.96 (d, 1 H), 7.89-7.86 (m, 1 H), 7.57-7,52 (m, 2H), 7.36-7.28 (m, 5H), 6.87 (s, 1 H), 2.31 (s,3H). |
| a-261 | 9.03 (d, 1H), 8.50 (d, 1H), 8.45 (s, 1H), 8.17 (d, 1H), 8.10 (d, 1H), 7.89-7.86 (m, 1H), 7.68 (d, 1H), 7.58-7.52 (m, 3H), 7.48-7.40 (m, 2H), 7.35 (d, 1H), 6.88 (s, 1H) |

(continued)

| Compound No. | $^1$H-NMR ($CDCl_3$-$d_6$, δ ppm) |
|---|---|
| a-264 | 9.08 (d, 1 H), 8.77 (d, 1 H), 8.50 (d, 1 H), 8.45 (s, 1 H), 8.27 (d, 1 H), 8.12 (d, 1 H), 7.90-7.87 (m, 1 H), 7.67-7.52 (m, 4H) 7.40 (d, 1 H), 6.89 (s, 1H). |
| a-267 | 8.72 (d, 1 H), 8.49 (d, 1 H), 8.45 (s, 1 H), 8.10 (d, 1 H), 7.91 (s, 1 H), 7.90-7.86 (m, 1 H), 7.57-7.53 (m, 2H), 7.34 (d, 1 H), 6.88 (s, 1 H), 2.47 (s, 3 H), 2.32 (s, 3H). |
| a-272 | 8.71 (d, 1H), 8.49 (d, 1H), 8.45 (s, 1H), 8.13 (d, 1H), 7.99 (d, 1H), 7.89-7.86 (m, 1H), 7.59-7.52 (m, 2H), 7.48-7.45 (m, 2H), 7.34-7.28 (m, 2H), 6.87 (s, 1H). |
| a-284 | 8.77 (d, 1 H), 8.49 (d, 1 H), 8.44 (s, 1 H), 8.05 (d, 1 H), 8.00 (d, 1 H), 7.87-7.85 (m, 1 H), 7.56-7.47 (m, 2H), 7.28 (d, 1 H), 6.85 (s, 1 H), 4.66 (s, 2H), 3.79-3.73 (m, 1 H), 1.29-1.24 (m, 6H). |
| a-286 | 2.01 (s, 3H), 3.80 (S, 2H), 6.86 (s, 1H), 7.27 (s, 1H), 7.40-7.56 (m, 2H), 7.84-7.88 (m, 1H), 7.92 (s, 1H), 8.04 (d, 1H), 8.45 (s, 1H), 8.49 (s, 1H), 8.77 (s, 1H) |
| a-287 | 5.06 (br-s, 2H), 6.58 (s, 1H), 6.68 (s, 1H), 6.80 (s, 1H), 7.28-7.33 (m, 1H), 7.47-7.53 (m, 1H), 7.80-7.91 (m, 2H), 8.41 (s, 1H), 8.45 (s, 1H), 8.60 (s, 1H) |
| a-302 | 8.86 (dd, 1H), 8.54 (d, 1H), 8.44 (s, 1H), 8.07 (d, 1H), 7.75 (s, 1H), 7.45-7.42 (m, 1H), 7.30-7.26 (m, 2H), 7.12 (s, 1H), 6.81 (s, 1H), 2.09 (s, 3H). |
| a-304 | 8.82 (dd, 1 H), 8.61 (d, 1 H), 8.45 (s, 1 H), 8.08-8.03 (m, 2H), 7.90 (s, 1 H), 7.56 (dd, 1 H), 7.39-7.31 (m, 3H), 6.86 (s, 1 H), 3.38 (s, 1H). |
| a-308 | 8.87 (dd, 1H), 8.45 (s, 1H), 8.44 (s, 1H), 8.09 (d, 1H), 8.01 (d, 1H), 7.54 (dd, 1H), 7.47-7.44 (m, 1H), 7.31 (dd, 1H), 7.15 (d, 1H), 6.83 (s, 1H). |
| a-309 | 3.35 (s, 6H), 5.58 (s, 1H), 6.83 (s, 1H), 7.28 (s, 1H), 7.48-7.53 (m, 2H), 7.80-7.87 (m, 1H), 803 (d, 1H), 8.09 (s, 1H), 8.42 (s, 1H), 8.48 (s, 1H), 8.83 (s, 1H) |
| a-31 0 | 8.85 (dd, 1 H), 8.51 (d, 1 H), 8.44 (s, 1 H), 8.06 (d, 1 H), 7.60 (s, 1 H), 7.45 (dd, 1 H), 7.31 (dd, 1 H), 7.16-7.13 (m, 2H), 6.82 (s, 1 H), 2.67 (t, 2H), 1.72-1.61 (m, 2H), 0.96 (t, 3H). |
| a-311 | 5.63(d, 1H), 6.34(d, 1H), 6.94(dd, 1H), 7.27(s, 1H), 7.56-7.61 (m, 2H), 7.77(d, 1H), 7.90-7.95 (m, 3H), 8.20(d, 1H), 863(d, 1H), 9.33(s, 1H). |
| a-313 | 3.89 (s, 3H), 6.78 (s, 1H), 6.83 (dd, 1H), 7.12 (s, 1H), 7.27-7.31 (m, 2H), 7.42 (dd, 1H), 8.04-8.06 (m, 1H), 8.44 (s, 1H), 8.44 (d, 2H), 8.84 (dd, 1H). |
| a-315 | 0.30(s, 9H), 6.84(s, 1 H), 7.25-7.27(m, 1 H), 7.48-7.57(m, 3H), 7.85(dd, 1 H), 7.98(d, 1 H), 8.05(d, 1 H), 8.44(s, 1 H), 8.48(d, 1 H). |
| a-319 | 1.93 (s, 3H), 3.66 (s, 2H), 6.82 (s, 1H), 7.11 (s, 1H), 7.24-7.29 (m, 2H), 7.40-7.43 (m, 1H), 7.72 (s, 1H), 8.03 (d, 1H), 8.42 (s, 1H), 8.54 (d, 1H), 8.82 (d, 1H). |
| a-321 | 3.32 (s, 3H), 3.33 (s, 3H), 5.44 (s, 1H), 6.87 (s, 1H), 7.14 (d, 1H), 7.28-7.32 (m, 1H), 7.41-7.45 (m, 2H), 7.88 (s, 1H), 8.04-8.07 (m, 1H), 8.44 (s, 1H), 8.63 (d, 1H), 8.84 (dd, 1H). |
| b-1 | 6.65 (s, 1 H), 7.14 (s, 1 H), 7.22-7.38 (m, 1 H), 7.40-7.47 (m, 1 H), 7.50-7.58 (m, 1 H), 7.66-7.74 (m, 1 H), 7.77 (d, 1 H), 8.01 (d, 1 H), 8.46-8.57 (m, 3 H), 8.87 (s, 1 H), 8.96 (s, 1 H) |
| b-5 | 6.82 (s, 1 H), 7.22 (s, 1 H), 7.30-7.47 (m, 3H), 7.54 (d, 1 H), 7.63-7.70 (m, 1 H), 7.79 (s, 1 H), 7.97-8.04 (m, 2H), 8.51 (s, 1 H), 8.76 (s, 1 H) |
| b-7 | 6.42 (s, 1 H), 6.90 (s, 1 H), 7.01 (s, 1 H), 7.33-7.58 (m, 6H), 7.90 (s, 1 H), 7.97 (d, 1 H), 8.11 (s, 1 H), 8.74 (s, 1 H ) |
| b-8 | 6.42 (s, 1 H), 6.94 (s, 1 H), 7.09 (d, 1 H), 7.34 (dd, 1 H), 7.40 (dt, 2H), 7.48 (tt, 3H), 7.90 (s, 1 H), 7.98 (dd, 1 H), 8.03 (d, 1 H), 8.78 (dd, 1H) |
| b-9 | 6.58 (s, 1H), 7.03 (s, 1H), 7.11 (d, 1H), 7.31 (dd, 1H), 7.44-7.49 (m, 2H), 7.70 (dd, 1H), 7.89 (s, 1H), 7.89 (d, 1H), 8.01 (d, 1H), 8.48 (d, 1H), 8.76 (dd, 1H). |
| b-10 | 2.46 (s, 3H), 6.56 (s, 1H), 7.02-7.04 (m, 2H), 7.37 (dd, 1H), 7.43-7.48 (m, 1H), 7.66-7.73 (m, 2H), 7.88 (s, 1H), 7.96 (d, 1H), 8.48 (d, 1H), 8.62 (d, 1H). |

(continued)

| Compound No. | $^1$H-NMR ($CDCl_3$-$d_6$, δ ppm) |
|---|---|
| b-12 | 6.51 (s, 1 H), 7.14 (s, 1 H), 7.19 (d, 1 H), 7.34 (dd, 1 H), 7.40 (dt, 2H), 7.51 (dd, 1 H), 7.57 (dt, 2H), 7.65 (s, 1 H), 8.01 (dt, 2H), 8.78 (dd, 1H) |
| b-13 | 2.38 (s, 3H), 6.72 (s, 1H), 7.22 (d, 1H), 7.23 (d, 1H), 7.39 (dt, 2H), 7.45 (dd, 1H), 7.60 (dt, 2H), 7.80 (d, 1H), 7.87 (d, 1H), 7.93 (d, 1H), 8.48 (d, 1H) |
| b-15 | 2.46 (s, 3H), 6.64 (s, 1 H), 7.14-7.15 (m, 2H), 7.42-7.52 (m, 2H), 7.66 (s, 1 H), 7.76 (s, 1 H), 7.80 (dd, 1 H), 7.95 (dd, 1 H), 8.46 (d, 1 H), 8.61 (d, 1H). |
| b-19 | 6.23 (s, 1H), 6.52 (s, 1H), 7.10 (s, 1H), 7.29-7.56 (m, 7H), 7.93-8.03 (m, 2H), 8.19 (s, 1H), 8.77 (s, 1H) |
| b-23 | 2.39 (s, 3H), 6.00 (s, 1H), 7,15 (d, 1H), 7.29 (d, 1H), 7.43 (dd, 1H), 7.60 (s, 1H), 7.72 (t, 2H), 8.02 (dd, 2H), 8.14 (dd, 2H), 8.76 (dd, 1H), 8.87 (dd, 1H) |
| b-26 | 2.16 (bs, 1H), 4.59 (d, 2H), 6.48 (s, 1H), 7.18 (d, 1H), 7.29-7.43 (m, 4H), 7.49 (dd, 1H), 7.57 (d, 1H), 7.60 (dd, 2H), 7.97 (d, 1H), 7.99 (d, 1H), 8.75 (dd, 1H). |
| b-30 | 6.39 (s, 1 H), 6.98 (s, 1 H), 7.07 (s, 1 H), 7.32-7.51 (m, 4H), 7.65 (s, 1 H), 7.89 (s, 1 H), 7.98 (d, 1 H), 8.03 (d, 1 H), 8.79 (s, 1 H) |
| b-37 | 8.71 (d, 1H), 8.42 (dd, 1H), 7.65-7.40 (m, 7H), 7.31 (d, 1H), 7.27-7.24 (m, 2H), 6.75 (s, 1H). |
| b-39 | 6.73 (s, 1 H), 7.33-7.49 (m, 4H), 7.52-7.63 (m, 3H), 8.02 (d, 1 ), 8.42 (s, 1 H), 8.94-8.97 (m, 2H) |
| b-40 | 7.30 (d, 1 H), 7.32-7.44 (m, 3H), 7.49-7.53 (m, 1 H), 7.60-7.68 (m, 3H), 8.07 (d, 1 H), 8.28 (d, 1 H), 8.36 (s, 1 H), 8.79 (s, 1 H) |
| b-44 | 7.08 (d, 1 H), 7.32-7.45 (m, 4H), 7.56-7.74 (m, 5H), 7.79 (d, 1 H), 8.03 (d, 1 H), 8.34 (s, 1 H) |
| b-47 | 3.03 (s, 3H), 7.21 (s, 1H), 7.24-7.31 (m, 1H), 7.43-7.53 (m, 2H), 7.50-7.53 (m, 1H), 8.30-8.51 (m, 4H), 8.72 (d, 1H) |
| b-49 | 2.28 (s, 3H), 4.04 (s, 3H), 6.84 (s, 1H), 7.29 (s, 1H), 7.46-7.53 (m, 2H), 7.81-7.87 (m, 1H), 8.01 (d, 1H), 8.11 (s, 1H), 8.43 (s, 1H), 8.47 (s, 1H), 9.17 (s, 1H) |
| b-53 | 8.82 (dd, 1H), 8.48 (d, 1H), 8.43 (s, 1H), 8.05 (dd, 1H), 7.89-7.86 (m, 1H), 7.57-7.52 (m, 2H), 7.39 (dd, 1H), 7.30 (dd, 1H), 6.86 (s, 1H), 4.10 (s, 3H), 2.43 (s, 3H). |
| b-54 | 9.05 (d, 1H), 8.77 (d, 1H), 8.50 (d, 1H), 8.45 (d, 1H), 8.06 (d, 1H), 7.88-7.85 (m, 1H), 7.58-7.52 (m, 2H), 7.45 (s, 1H), 7.34 (s, 1H), 6.87 (s, 1H), 4.14 (s, 3H). |
| b-56 | 8.99 (dd, 1 H), 8.63 (dd, 1 H), 8.52 (d, 1 H), 8.48 (s, 1 H), 8.17 (d, 1 H), 7.87-7.84 (m, 1 H), 7.73 (dd, 1 H), 7.58-7.54 (m, 1 H), 7.45 (d, 1 H), 6.89 (s, 1 H), 3.17 (d, 3H). |
| b-57 | 9.20 (dd, 1H), 8.75 (d, 1H), 8.52 (d, 1H), 8.48 (d, 1H), 8.23 (d, 1H), 7.88-7.78 (m, 2H), 7.59-7.48 (m, 2H), 6.90 (s, 1H), 3.47 (d, 3H). |
| b-58 | 8.79 (d, 1H), 8.50 (d, 1H), 8.4 7 (s, 1H), 8.18 (d, 1H), 8.04 (d, 1H), 7.86-7.83 (m, 1H), 7.56-7.51 (m, 2H), 7.12 (d, 1H), 6.82 (s, 1H). |
| b-62 | 8.78 (dd, 1 H), 8.50 (d, 1 H), 8.17 (s, 1 H), 7.99-7.94 (m, 2H), 7.78 (s, 1 H), 7.49-7.43 (m, 2H), 7.35-7.32 (m, 1 H), 7.23 (d, 1 H), 6.76 (s, 1 H). |
| b-68 | 6.84 (s, 1 H), 7.25 (s, 1 H), 7.30-7.37 (m, 1 H), 7.47-7.44 (m, 2H), 7.80-7.88 (m, 1 H), 8.00-8.08 (m, 2H), 8.42 (s, 1 H), 8.45 (s, 1 H), 8.79 (s, 1 H) |
| b-69 | 6.84 (s, 1H), 7.25 (s, 1H), 7.30-7.37 (m, 1H), 7.47-7.44 (m, 2H), 7.80-7,88 (m, 1H), 8.00-8.08 (m, 2H), 8.42 (s, 1H), 8.45 (s, 1H), 8.79 (s, 1H) |
| b-7 4 | 4.05 (s, 3H), 6.82 (s, 1 H), 7.11 (s, 1 H), 7.25-7.30 (m, 1 H), 7.40-7.44 (m, 1 H), 8.04 (d, 1 H), 8.19 (d, 1 H), 8.23 (s, 1 H), 8.44 (s, 1 H), 8.48 (s, 1 H), 8.33 (d, 1H) |

(continued)

| Compound No. | $^1$H-NMR ($CDCl_3$-$d_6$, δ ppm) |
|---|---|
| b-76 | 0.96-1.00 (m, 3H), 1.60-1.77 (m, 2H), 4.17 (t, 2H), 6.85 (s, 1H), 7.14 (s, 1H), 7.29-7.32 (m, 1H), 7.42-7.45 (m, 1H), 7.50-7.51 (m, 1H), 7.95 (s, 1H), 8.05-8.07 (m, 2H), 8.44 (s, 1H), 8.62 (d, 1H), 8.84 (dd, 1H). |
| *Note: Measurement solvent of Compound No. a-150: DMSO-d6<br>*Note: Measurement solvent of Compound No. a-311 DMSO-d6 | |

[Biological test]

The following test examples show that the compounds of the present invention are useful as active ingredients in agricultural and horticultural fungicides.

(Preparation of test emulsion)

5 parts by weight of the compound of the present invention, 93.5 parts by weight of dimethylformamide, and 1.5 parts by weight of polyoxyethylene alkylaryl ether were mixed and dissolved to prepare an emulsion (I) containing 5% of an active ingredient.

The control value was calculated by the following formula.

$$\text{Control value (\%)} = 100 - \{(\text{lesion area ratio in treated group}) / (\text{lesion area ratio in untreated group})\} \times 100$$

(Test Example 1) Tomato late blight control test

Water was added to the emulsion (I) so that the quinoline compound concentration became 125 ppm, followed by dissolution to obtain a drug solution. Subsequently, the above drug solution was sprayed onto a tomato seedling (variety "Regina", 4-5 leaf stage) cultivated in a seedling raising pot. After air drying, a suspension of zoosporangia of tomato late blight pathogen (Phytophthora infestans) was spray-inoculated on the tomato seedling sprayed with the drug solution (treated group). As a control, a tomato seedling not sprayed with the drug solution was inoculated in the same manner as described above (untreated group). They were left to stand in a moist chamber at 20°C. Four days after the inoculation, the leaves of the wheat seedling were visually observed, the lesion area ratio was determined, and the control value was calculated.

The compounds in Table 4 were tested for tomato late blight control. All compounds showed a control value of 75% or more.

Table 4

| | | | | | | | | | | |
|---|---|---|---|---|---|---|---|---|---|---|
| a-1 | a-34 | a-66 | a-101 | a-139 | a-171 | a-216 | a-253 | a-294 | b-5 | b-45 |
| a-2 | a-35 | a-67 | a-102 | a-140 | a-172 | a-217 | a-254 | a-295 | b-6 | b-46 |
| a-3 | a-36 | a-68 | a-103 | a-141 | a-175 | a-218 | a-255 | a-296 | b-8 | b-48 |
| a-4 | a-38 | a-69 | a-104 | a-142 | a-182 | a-219 | a-258 | a-297 | b-9 | b-49 |
| a-5 | a-41 | a-70 | a-105 | a-143 | a-184 | a-220 | a-260 | a-299 | b-10 | b-53 |
| a-6 | a-42 | a-71 | a-106 | a-144 | a-186 | a-221 | a-263 | a-301 | b-11 | b-54 |
| a-8 | a-43 | a-72 | a-107 | a-145 | a-188 | a-222 | a-264 | a-302 | b-12 | b-55 |
| a-9 | a-44 | a-73 | a-108 | a-146 | a-190 | a-223 | a-265 | a-303 | b-13 | b-56 |
| a-10 | a-45 | a-74 | a-109 | a-147 | a-193 | a-224 | a-266 | a-304 | b-14 | b-58 |
| a-11 | a-46 | a-75 | a-110 | a-148 | a-194 | a-225 | a-269 | a-305 | b-15 | b-59 |
| a-15 | a-47 | a-76 | a-112 | a-149 | a-195 | a-230 | a-272 | a-306 | b-16 | b-63 |
| a-16 | a-48 | a-77 | a-113 | a-150 | a-196 | a-232 | a-273 | a-307 | b-17 | b-64 |
| a-17 | a-49 | a-78 | a-114 | a-151 | a-197 | a-233 | a-274 | a-308 | b-18 | b-65 |

(continued)

| | | | | | | | | | | |
|---|---|---|---|---|---|---|---|---|---|---|
| a-18 | a-50 | a-80 | a-115 | a-152 | a-198 | a-234 | a-275 | a-309 | b-20 | b-66 |
| a-19 | a-51 | a-81 | a-116 | a-153 | a-199 | a-235 | a-276 | a-310 | b-21 | b-67 |
| a-20 | a-52 | a-83 | a-117 | a-154 | a-200 | a-236 | a-277 | a-311 | b-23 | b-68 |
| a-21 | a-53 | a-85 | a-118 | a-155 | a-201 | a-237 | a-278 | a-313 | b-26 | b-69 |
| a-22 | a-54 | a-87 | a-119 | a-156 | a-202 | a-238 | a-279 | a-314 | b-28 | b-70 |
| a-23 | a-55 | a-88 | a-120 | a-157 | a-203 | a-239 | a-280 | a-315 | b-30 | b-72 |
| a-24 | a-56 | a-89 | a-122 | a-158 | a-204 | a-240 | a-281 | a-316 | b-32 | b-73 |
| a-25 | a-57 | a-90 | a-123 | a-159 | a-205 | a-241 | a-282 | a-317 | b-33 | b-74 |
| a-26 | a-58 | a-91 | a-124 | a-160 | a-206 | a-242 | a-283 | a-318 | b-34 | b-75 |
| a-27 | a-59 | a-92 | a-125 | a-161 | a-207 | a-243 | a-284 | a-319 | b-36 | b-76 |
| a-28 | a-60 | a-95 | a-129 | a-162 | a-208 | a-244 | a-285 | a-320 | b-37 | |
| a-29 | a-61 | a-96 | a-131 | a-163 | a-209 | a-246 | a-286 | a-321 | b-38 | |
| a-30 | a-62 | a-97 | a-134 | a-165 | a-210 | a-247 | a-287 | b-1 | b-39 | |
| a-31 | a-63 | a-98 | a-135 | a-166 | a-211 | a-250 | a-289 | b-2 | b-40 | |
| a-32 | a-64 | a-99 | a-136 | a-167 | a-212 | a-251 | a-291 | b-3 | b-41 | |
| a-33 | a-65 | a-100 | a-138 | a-169 | a-213 | a-252 | a-292 | b-4 | b-43 | |

(Test Example 2) Grape downy mildew control test

Water was added to the emulsion (I) so that the quinoline compound concentration became 100 ppm, followed by dissolution to obtain a drug solution. Subsequently, the above drug solution was sprayed on a leaf disc of grape (variety "Chardonnay") placed on agar in a 6-well plate. After air drying, a suspension of zoosporangia of grape downy mildew pathogen (Plasmopara viticola) was spray-inoculated on the grape leaf disc sprayed with the drug solution (treated group). As a control, a grape leaf disc not sprayed with the drug solution was inoculated in the same manner as described above (untreated group). They were left to stand in a moist chamber at 20°C. Seven days after the inoculation, the grape leaf disc was visually observed, the lesion area ratio was determined, and the control value was calculated.

The compounds in Table 5 were tested for grape downy mildew control. All compounds showed a control value of 75% or more.

**Table 5**

| | | | | | | | | |
|---|---|---|---|---|---|---|---|---|
| a-1 | a-56 | a-104 | a-136 | a-194 | a-224 | a-280 | b-3 | b-66 |
| a-2 | a-57 | a-105 | a-140 | a-195 | a-225 | a-286 | b-5 | b-67 |
| a-3 | a-59 | a-106 | a-142 | a-196 | a-231 | a-287 | b-6 | b-68 |
| a-4 | a-63 | a-107 | a-143 | a-197 | a-232 | a-289 | b-7 | b-69 |
| a-6 | a-64 | a-108 | a-144 | a-198 | a-238 | a-291 | b-8 | b-70 |
| a-10 | a-65 | a-109 | a-145 | a-199 | a-239 | a-292 | b-10 | b-72 |
| a-14 | a-69 | a-110 | a-146 | a-200 | a-241 | a-295 | b-11 | b-73 |
| a-15 | a-74 | a-112 | a-147 | a-204 | a-242 | a-296 | b-13 | b-74 |
| a-22 | a-75 | a-113 | a-148 | a-205 | a-243 | a-302 | b-14 | b-75 |
| a-23 | a-76 | a-114 | a-149 | a-206 | a-245 | a-303 | b-15 | b-76 |
| a-26 | a-80 | a-115 | a-152 | a-207 | a-246 | a-304 | b-16 | |
| a-27 | a-82 | a-116 | a-155 | a-208 | a-247 | a-305 | b-18 | |
| a-28 | a-87 | a-117 | a-156 | a-209 | a-250 | a-306 | b-45 | |
| a-29 | a-88 | a-119 | a-159 | a-210 | a-252 | a-307 | b-48 | |
| a-30 | a-89 | a-120 | a-161 | a-211 | a-254 | a-308 | b-49 | |
| a-33 | a-91 | a-121 | a-162 | a-212 | a-265 | a-310 | b-54 | |
| a-42 | a-95 | a-122 | a-165 | a-216 | a-266 | a-314 | b-55 | |
| a-49 | a-96 | a-123 | a-166 | a-220 | a-268 | a-316 | b-58 | |
| a-53 | a-97 | a-125 | a-169 | a-221 | a-272 | a-317 | b-59 | |
| a-54 | a-98 | a-129 | a-182 | a-222 | a-275 | a-318 | b-63 | |

(continued)

| | | | | | | | | |
|---|---|---|---|---|---|---|---|---|
| a-55 | a-101 | a-134 | a-184 | a-223 | a-278 | b-2 | b-65 | |

Since those randomly selected from among the compounds of the present invention exhibit the above-mentioned effects, it can be understood that the compounds of the present invention including the compounds that are not exemplified are compounds having fungicidal effects, which causes no phytotoxicity to plant bodies, with little toxicity to humans, animals and fish and little impact on the environment.

[Industrial Applicability]

According to the present invention, it is possible to provide a quinoline compound excellent in fungicidal activity, excellent in safety and can be synthesized in an industrially favorable manner, and an agricultural and horticultural fungicide containing this compound as an active ingredient.

## Claims

1. A compound represented by a formula (II), an N-oxide compound, or a salt thereof:

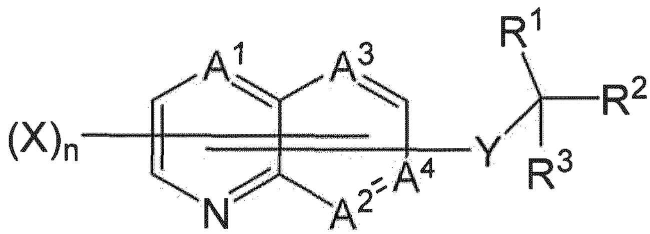

(II)

wherein

$R^1$ is a hydrogen atom, a substituted or unsubstituted $C_{1-6}$ alkyl group, or a halogeno group;
$R^2$ is a substituted or unsubstituted 5- to 6-membered heteroaryl group;
$R^3$ is a substituted or unsubstituted $C_{6-10}$ aryl group or a substituted or unsubstituted 5- to 10-membered heteroaryl group;
Y is an oxygen atom, a nitrogen atom, or a sulfur atom;
$A^1$, $A^2$, $A^3$ and $A^4$ are each independently a carbon atom or a nitrogen atom,
provided that two or more of $A^1$ to $A^4$ do not represent nitrogen atoms at the same time;
X is a substituent;
n represents the number of chemically acceptable substituents represented by X, and is any one of integers of 0 to 5; and
when n is 2 or more, the substituents X may be the same or different from each other.

2. The compound according to Claim 1, wherein the formula (II) is a formula (I), an N-oxide compound, or a salt thereof:

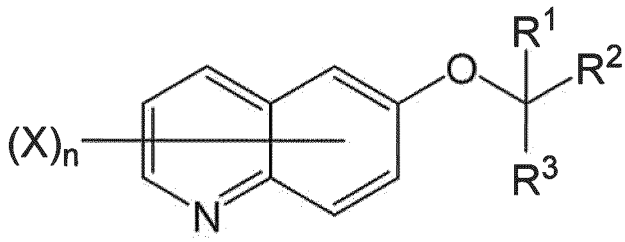

(I)

wherein $R^1$, $R^2$, $R^3$, X and n are the same as those defined in the formula (II).

3. An agricultural and horticultural fungicide comprising, as an active ingredient, at least one selected from the group consisting of the compound according to Claim 1 or 2 and a salt thereof.

**INTERNATIONAL SEARCH REPORT**

International application No. PCT/JP2018/035113

A. CLASSIFICATION OF SUBJECT MATTER
Int.Cl. [see extra sheet]

According to International Patent Classification (IPC) or to both national classification and IPC

B. FIELDS SEARCHED

Minimum documentation searched (classification system followed by classification symbols)
Int.Cl. C07D403/00, A01N43/00, A01N47/00, A01N55/00, C07D405/00, C07D413/00, C07D417/00, C07D487/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | WO 2017/007694 A1 (GILEAD SCIENCES, INC.) 12 January 2017, claim 1, page 181, compound 7, page 205, compound 109, page 260, compound 344, page 351, compound 718, etc. & JP 2018-520160 A & US 2017/0008873 A1 & EP 3319955 A1 & KR 10-2018-0022981 A & CN 108026067 A | 1 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 05.11.2018 | 20.11.2018 |
| Name and mailing address of the ISA/ Japan Patent Office 3-4-3, Kasumigaseki, Chiyoda-ku, Tokyo 100-8915, Japan | Authorized officer<br>Telephone No. |

Form PCT/ISA/210 (second sheet) (January 2015)

INTERNATIONAL SEARCH REPORT

International application No. PCT/JP2018/035113

C (Continuation). DOCUMENTS CONSIDERED TO BE RELEVANT

| Category* | Citation of document, with indication, where appropriate, of the relevant passages | Relevant to claim No. |
|---|---|---|
| X | WO 2017/007689 A1 (GILEAD SCIENCES, INC.) 12 January 2017, claim 1, pages 169-171, compounds 1, 7, 9, etc. & JP 2018-520161 A & US 2017/0008905 A1 & EP 3191470 A1 & KR 10-2018-0022982 A & CN 107922390 A | 1 |
| X | WO 2016/180536 A1 (SELVITA, S. A.) 17 November 2016, claim 1, page 467, intermediate 254 & EP 3295444 A1 & DE 102015005968 A1 | 1 |
| X | WO 2011/142316 A1 (KYOWA HAKKO KIRIN CO., LTD.) 17 November 2011, claim 1, example 23 & US 2013/0065905 A1, claim 1, example 23 & EP 2570411 A1 & CN 102892759 A & KR 10-2013-0062951 A | 1 |
| X | JP 2010-536829 A (ABBOTT GMBH & CO. KG) 02 December 2010, claim 1, examples 70, 73 & US 2010/0222346 A1, claim 1, examples 70, 73 & WO 2009/024611 A2 & EP 2178842 A2 & CN 101784533 A | 1 |
| X | MAO, J. L. et al., From serendipity to rational antituberculosis drug discovery of mefloquine-isoxazole carboxylic acid esters, Journal of Medicinal Chemistry, 2009, 52(22), pp. 6966-6978, p. 6969, fig. 4, D) | 1 |
| A | WO 2013/058256 A1 (NIPPON SODA CO., LTD.) 25 April 2013, claims 1-7 (Family: none) | 1-3 |
| A | JP 2014-166991 A (NIPPON SODA CO., LTD.) 11 September 2014, claims 1-4 (Family: none) | 1-3 |

Form PCT/ISA/210 (continuation of second sheet) (January 2015)

**INTERNATIONAL SEARCH REPORT**

International application No.
PCT/JP2018/035113

(Continuation of Box No. A)

CLASSIFICATION OF SUBJECT MATTER
C07D403/14(2006.01)i, A01N43/42(2006.01)i, A01N43/60(2006.01)i, A01N43/653(2006.01)i, A01N43/713(2006.01)i, A01N43/76(2006.01)i, A01N43/78(2006.01)i, A01N43/80(2006.01)i, A01N43/824(2006.01)i, A01N43/836(2006.01)i, A01N43/90(2006.01)i, A01N47/02(2006.01)i, A01N47/40(2006.01)i, A01N55/10(2006.01)i, A01P3/00(2006.01)i, C07D405/12(2006.01)i, C07D413/12(2006.01)i, C07D413/14(2006.01)i, C07D417/14(2006.01)i, C07D487/04(2006.01)i

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2017184719 A
- WO 2005105100 A